# EUROPEAN PATENT SPECIFICATION

(11) **EP 2 914 720 B1**
(45) Date of publication and mention of the grant of the patent: **31.08.2022**
(21) Application number: 13792210.0
(22) Date of filing: 05.11.2013
(51) Int. Cl.: C12N 9/54

(54) **COMPOSITIONS AND METHODS COMPRISING THERMOLYSIN PROTEASE VARIANTS**
ZUSAMMENSETZUNGEN UND VERFAHREN MIT THERMOLYSINPROTEASEVARIANTEN
COMPOSITIONS ET PROCÉDÉS COMPORTANT DES VARIANTS DE THERMOLYSINE PROTÉASE

(30) Priority: 05.11.2012 US 201261722660 P
(43) Date of publication of application: 09.09.2015
(73) Proprietor: Danisco US Inc., Palo Alto, California 94304 (US)
(72) Inventor: ALEKSEYEV, Viktor Yuryevich, Palo Alto California 94304 (US); BABE, Lilia Maria, Palo Alto California 94304 (US); ESTELL, David A., Palo Alto California 94304 (US); GOEDEGEBUUR, Frits, Palo Alto California 94304 (US); MULDER, Harm, Palo Alto California 94304 (US); TORRES PAZMINO, Daniel Esteban, Palo Alto California 94304 (US); YAO, Jian, Palo Alto California 94304 (US); BOTT, Richard, R., Palo Alto California 94304 (US)
(74) Representative: Mewburn Ellis LLP
(86) International application number: PCT/US2013/068590
(87) International publication number: WO 2014/071410

(56) References cited:
- WO-A1-2012/110563
- WO-A1-2012/110563
- WO-A2-2004/033668
- WO-A2-2004/033668
- WO-A2-2007/044993
- WO-A2-2009/058303
- WO-A2-2009/058303
- KUSANO M ET AL: "Effects of the mutational combinations on the activity and stability of thermolysin", JOURNAL OF BIOTECHNOLOGY, ELSEVIER SCIENCE PUBLISHERS, AMSTERDAM, NL, vol. 147, no. 1, 3 May 2010 (2010-05-03), pages 7-16, XP027013981, ISSN: 0168-1656 [retrieved on 2010-03-07]
- YASUKAWA ET AL: "Improving the activity and stability of thermolysin by site-directed mutagenesis", BIOCHIMICA ET BIOPHYSICA ACTA (BBA) - PROTEINS & PROTEOMICS, ELSEVIER, NETHERLANDS, vol. 1774, no. 10, 28 September 2007 (2007-09-28), pages 1281-1288, XP022277478, ISSN: 1570-9639, DOI: 10.1016/J.BBAPAP.2007.08.002

## Description

### BACKGROUND OF THE INVENTION

Bacilli are gram-positive bacteria that secrete a number of industrially useful enzymes, which can be produced cheaply in high volume by fermentation. Examples of secreted *Bacillus* enzymes are the subtilisin serine proteases, zinc containing neutral proteases, alpha-amylases, and cellulases. *Bacillus* proteases are widely used in the textile, laundry and household industries (Galante, Current Organic Chemistry, 7:1399-1422, 2003; and Showell, Handbook of Detergents, Part D: Formulation, Hubbard (ed.), NY: Taylor and Francis Group, 2006). The classification of proteases found in microorganisms is based on their catalytic mechanism which results in four groups: the serine proteases; metallo-proteases; cysteine proteases; and aspartic proteases. The serine proteases have alkaline pH optima, the metalloproteases are optimally active around neutrality, and the cysteine and aspartic enzymes have acidic pH optima (Biotechnology Handbooks, Bacillus. vol. 2, edited by Harwood, 1989 Plenum Press, New York). WO2004/033668 describes protease enzymes, polynucleotides encoding the enzymes, and methods of making and using these polynucleotides and polypetides. WO2012/110563 describes thermolysin-like metalloproteases for use in cleaning, as well as detergent compositions comprising said thermolysin-like metalloproteases. WO2009/058303 describes methods and compositions comprising at least one thermolysin-like neutral protease enzyme with improved storage stability and/or catalytic activity. Kusano et al., Journal of Biotechnology, 2010, Vol. 147, No. 1, pp. 7-16, describes effects of the mutantional combinations on the activity and stability of thermolysin. Although serine proteases have long been known in the art of industrial enzymes, there remains a need for engineered proteases that are suitable for particular conditions and uses.

### SUMMARY OF THE INVENTION

The present disclosure provides, *inter alia,* thermolysin enzyme variants as defined in the claims, and compositions and methods related to the production and use thereof In a first aspect the present invention provides a thermolysin enzyme variant of the thermolysin of SEQ ID NO:3 as defined in claim 1.

In a second aspect the present invention provides a cleaning composition comprising at least one thermolysin enzyme variant of the first aspect of the invention.

In some embodiments the cleaning composition is a detergent composition.

In some embodiments the cleaning composition is a laundry detergent composition, a dish detergent composition, or a hard surface cleaning composition.

In a third aspect the present invention provides a method of cleaning, comprising contacting a surface or an item with a cleaning composition comprising at least one thermolysin enzyme variant of the first aspect of the invention.

In a related aspect the present invention provides a method of cleaning comprising contacting a surface or an item with a cleaning composition of the second aspect of the invention.

### BRIEF DESCRIPTION OF THE DRAWINGS

Figure 1 shows the plasmid map of pHPLT-proteinaseT.
Figures 2A-2C provide a phylogenetic tree of 424 members of the MEROPS family M4. The position of the X-axis is correct for Figure 2A, while the X-axis for Figures 2B and 2C have moved in manipulation.

### DESCRIPTION OF THE INVENTION

The present invention provides improved metalloprotease enzymes, especially enzymes useful for detergent compositions, as defined in the claims. Specifically, the present invention provides metalloprotease enzyme variants having one modification, as defined in the claims. This can be achieved by making improvements to the enzyme by improving wash performance, stability of the enzyme in detergent compositions, and/or thermostability of the enzyme that improve effectiveness of the enzyme in a wash cycle. The present invention provides variant thermolysin metalloprotease enzymes, that are particularly well suited to and useful in a variety of cleaning applications. The invention includes compositions comprising at least one of the variant thermolysins as defined in the claims. Some such compositions comprise detergent compositions. The invention provides various species, including *Bacillus and Geobacillus species* variant metalloprotease enzymes and compositions comprising one or more such variant thermolysins, provided that such enzymes are as defined in the claims. The thermolysin enzyme variant of the present invention can be combined with other enzymes useful in detergent compositions. The invention also provides methods of cleaning using the thermolysin enzyme variant of the present invention.

In addition, the present invention provides compositions comprising the thermolysin enzyme variant, as defined in claim 1. In some embodiments, the present invention provides cleaning compositions comprising the thermolysin enzyme variant.

It is to be appreciated those certain feature of the invention, which are, for clarity, described above and below in the context of separate embodiments, may also be provided in combination in a single embodiment. Conversely, various feature of the invention that are, for brevity, described in the context of a single embodiment, may also be provided separately or in any sub-combination.

### DEFINITIONS

Unless otherwise indicated, the practice of the present invention involves conventional techniques commonly used in molecular biology, protein engineering, microbiology, and recombinant DNA, which are within the skill of the art. Such techniques are known to those of skill in the art and are described in numerous texts and reference works well known to those of skill in the art.

Unless defined otherwise herein, all technical and scientific terms used herein have the same meaning as commonly understood by one of ordinary skill in the art to which this invention pertains. Many technical dictionaries are known to those of skill in the art. Although any methods and materials similar or equivalent to those described herein find use in the practice of the present invention, some suitable methods and materials are described herein. Accordingly, the terms defined immediately below are more fully described by reference to the Specification as a whole. Also, as used herein, the singular "a", "an" and "the" includes the plural reference unless the context clearly indicates otherwise. Numeric ranges are inclusive of the numbers defining the range. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

The practice of the present invention employs, unless otherwise indicated, conventional techniques of protein purification, molecular biology, microbiology, recombinant DNA techniques and protein sequencing, all of which are within the skill of those in the art.

Furthermore, the headings provided herein are not limitations of the various aspects or embodiments of the invention which can be had by reference to the specification as a whole. Accordingly, the terms defined immediately below are more fully defined by reference to the specification as a whole. Nonetheless, in order to facilitate understanding of the invention, a number of terms are defined below.

It is intended that every maximum numerical limitation given throughout this specification include every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

As used herein, the terms "protease" and "proteinase" refer to an enzyme protein that has the ability to break down other proteins. A protease has the ability to conduct "proteolysis," which begins protein catabolism by hydrolysis of peptide bonds that link amino acids together in a peptide or polypeptide chain forming the protein. This activity of a protease as a protein-digesting enzyme is referred to as "proteolytic activity." Many well known procedures exist for measuring proteolytic activity *(See e.g.,* Kalisz, "Microbial Proteinases," In: Fiechter (ed.), Advances in Biochemical Engineering/Biotechnology, (1988)). For example, proteolytic activity may be ascertained by comparative assays which analyze the respective protease's ability to hydrolyze a commercial substrate. Exemplary substrates useful in the analysis of protease or proteolytic activity, include, but are not limited to, di-methyl casein (Sigma C-9801), bovine collagen (Sigma C-9879), bovine elastin (Sigma E-1625), and bovine keratin (ICN Biomedical 902111). Colorimetric assays utilizing these substrates are well known in the art *(See e.g.,* WO 99/34011 and U.S. Pat. No. 6,376,450). The pNA assay *(See e.g.,* Del Mar et al., Anal. Biochem. 99:316-320 [1979]) also finds use in determining the active enzyme concentration for fractions collected during gradient elution. This assay measures the rate at which p-nitroaniline is released as the enzyme hydrolyzes the soluble synthetic substrate, succinyl-alanine-alanine-proline-phenylalanine-p-nitroanilide (suc-AAPF-pNA). The rate of production of yellow color from the hydrolysis reaction is measured at 410 nm on a spectrophotometer and is proportional to the active enzyme concentration. In addition, absorbance measurements at 280 nanometers (nm) can be used to determine the total protein concentration. The active enzyme/total protein ratio gives the enzyme purity.

As used herein, the term "thermolysin" refers any member of the M4 protease family as described in MEROPS - The Peptidase Data base *(See,* Rawlings et al., MEROPS: the peptidase database, Nucl Acids Res, 34 Database issue, D270-272 [2006]), of which thermolysin (TLN; EC 3.4.24.27) is the prototype. The amino acid sequence of thermolysin, (EC 3.4.24.27) the neutral metallo endo-peptidase secreted from *Bacillus thermoproteolyticus* was first reported by Titani et al (Titani et al, (1972), Amino-acid sequence of thermolysin. Nature New Biol. 238:35-37). Subsequently, the gene for this enzyme was cloned by O'Donohue et al (O'Donohue,M.J (1994) Cloning and expression in Bacillus subtilis of the npr gene from Bacillus thermoproteolyticus Rokko coding for the thermostable metalloprotease thermolysin. Biochem. J. 300:599-603) and the sequence set forth as UniProtKB/Swiss-Prot Accession No. P00800 (SEQ ID NO:4). The only differences between the protein sequences reported by Titani et al and O'Donohue et al are the confirmation of Asn at position 37 (instead of Asp) and Gln at position 119 (instead of Glu). As such the terms "thermolysin," "stearolysin", "bacillolysin," "proteinase-T", "PrT", "Thermolysin-like protease", and "TLPs", are used interchangeably herein to refer to the neutral metalloprotease enzyme of *Bacillus thermoproteolyticus.*

As used herein, the term "variant polypeptide" refers to a polypeptide comprising an amino acid sequence that differs in at least one amino acid residue from the amino acid sequence of a parent or reference polypeptide (including but not limited to wild-type polypeptides).

As used herein, "the genus *Bacillus"* includes all species within the genus *"Bacillus,"* as known to those of skill in the art, including but not limited to *B. subtilis, B. licheniformis, B. lentus, B. brevis, B. stearothermophilus, B.* alkalophilus, *B. amyloliquefaciens, B. clausii, B. halodurans, B. megaterium, B. coagulans, B. circulans, B. lautus,* and *B. thuringiensis.* It is recognized that the genus *Bacillus* continues to undergo taxonomical reorganization. Thus, it is intended that the genus include species that have been reclassified, including but not limited to such organisms as *B. stearothermophilus,* which is now named *"Geobacillus stearothermophilus."* The production of resistant endospores in the presence of oxygen is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus.*

The terms "polynucleotide" and "nucleic acid," which are used interchangeably herein, refer to a polymer of any length of nucleotide monomers covalently bonded in a chain. DNA (deoxyribonucleic acid), a polynucleotide comprising deoxyribonucleotides, and RNA (ribonucleic acid), a polymer of ribonucleotides, are examples of polynucleotides or nucleic acids having distinct biological function. Polynucleotides or nucleic acids include, but are not limited to, a single-, double- or triple-stranded DNA, genomic DNA, cDNA, RNA, DNA-RNA hybrid, or a polymer comprising purine and pyrimidine bases, or other natural, chemically, biochemically modified, non-natural or derivatized nucleotide bases. The following are non-limiting examples of polynucleotides: genes, gene fragments, chromosomal fragments, expressed sequence tag(s) (EST(s)), exons, introns, messenger RNA (mRNA), transfer RNA (tRNA), ribosomal RNA (rRNA), ribozymes, complementary DNA (cDNA), recombinant polynucleotides, branched polynucleotides, plasmids, vectors, isolated DNA of any sequence, isolated RNA of any sequence, nucleic acid probes, and primers.

As used herein, the term "mutation" refers to changes made to a reference amino acid or nucleic acid sequence. It is intended that the term encompass substitutions, insertions and deletions.

As used herein, the term "vector" refers to a nucleic acid construct used to introduce or transfer nucleic acid(s) into a target cell or tissue. A vector is typically used to introduce foreign DNA into a cell or tissue. Vectors include plasmids, cloning vectors, bacteriophages, viruses *(e.g.,* viral vector), cosmids, expression vectors, shuttle vectors, and the like. A vector typically includes an origin of replication, a multicloning site, and a selectable marker. The process of inserting a vector into a target cell is typically referred to as transformation..

As used herein, the term "expression cassette," "expression plasmid" or "expression vector" refers to a nucleic acid construct or vector generated recombinantly or synthetically for the expression of a nucleic acid of interest in a target cell. An expression vector or expression cassette typically comprises a promoter nucleotide sequence that drives expression of the foreign nucleic acid. The expression vector or cassette also typically includes any other specified nucleic acid elements that permit transcription of a particular nucleic acid in a target cell. A recombinant expression cassette can be incorporated into a plasmid, chromosome, mitochondrial DNA, plastid DNA, virus, or nucleic acid fragment. Many prokaryotic and eukaryotic expression vectors are commercially available.

As used herein, a "plasmid" refers to an extrachromosomal DNA molecule which is capable of replicating independently from the chromosomal DNA. A plasmid is double stranded (ds) and may be circular and is typically used as a cloning vector.

As used herein in the context of introducing a nucleic acid sequence into a cell, the term "introduced" refers to any method suitable for transferring the nucleic acid sequence into the cell. Such methods for introduction include but are not limited to protoplast fusion, transfection, transformation, electroporation, conjugation, and transduction *(See e.g.,* Ferrari et al., "Genetics," in Hardwood et al. (eds.), Bacillus, Plenum Publishing Corp., pp. 57-72 [1989]).

Transformation refers to the genetic alteration of a cell which results from the uptake, optional genomic incorporation, and expression of genetic material (*e.g*., DNA).

As used herein, a nucleic acid is "operably linked" with another nucleic acid sequence when it is placed into a functional relationship with another nucleic acid sequence. For example, a promoter or enhancer is operably linked to a nucleotide coding sequence if the promoter affects the transcription of the coding sequence. A ribosome binding site may be operably linked to a coding sequence if it is positioned so as to facilitate translation of the coding sequence. Typically, "operably linked" DNA sequences are contiguous. However, enhancers do not have to be contiguous. Linking is accomplished by ligation at convenient restriction sites. If such sites do not exist, synthetic oligonucleotide adaptors or linkers may be used in accordance with conventional practice.

As used herein the term "gene" refers to a polynucleotide (e.g., a DNA segment), that encodes a polypeptide and includes regions preceding and following the coding regions as well as intervening sequences (introns) between individual coding segments (exons).

As used herein, "recombinant" when used with reference to a cell typically indicates that the cell has been modified by the introduction of a foreign nucleic acid sequence or that the cell is derived from a cell so modified. For example, a recombinant cell may comprise a gene not found in identical form within the native (non-recombinant) form of the cell, or a recombinant cell may comprise a native gene (found in the native form of the cell) but which has been modified and re-introduced into the cell. A recombinant cell may comprise a nucleic acid endogenous to the cell that has been modified without removing the nucleic acid from the cell; such modifications include those obtained by gene replacement, site-specific mutation, and related techniques known to those of ordinary skill in the art. Recombinant DNA technology includes techniques for the production of recombinant DNA *in vitro* and transfer of the recombinant DNA into cells where it may be expressed or propagated, thereby producing a recombinant polypeptide. "Recombination," "recombining," and "recombined" of polynucleotides or nucleic acids refer generally to the assembly or combining of two or more nucleic acid or polynucleotide strands or fragments to generate a new polynucleotide or nucleic acid. The recombinant polynucleotide or nucleic acid is sometimes referred to as a chimera. A nucleic acid or polypeptide is "recombinant" when it is artificial or engineered.

As used herein, the term nucleic acid or gene "amplification" refers to a process by which specific DNA sequences are disproportionately replicated such that the amplified nucleic acid or gene becomes present in a higher copy number than was initially present in the genome.

"Amplification" is a special case of nucleic acid replication involving template specificity. It is to be contrasted with non-specific template replication (*i.e.,* replication that is template-dependent but not dependent on a specific template). Template specificity is here distinguished from fidelity of replication (*i.e.,* synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

As used herein, the term "primer" refers to an oligonucleotide (a polymer of nucleotide residues), whether occurring naturally as in a purified restriction digest or produced synthetically, which is capable of acting as a point of initiation of synthesis when placed under conditions in which synthesis of a primer extension product which is complementary to a nucleic acid strand is induced (*i.e.,* in the presence of nucleotides and an inducing agent such as DNA polymerase and at a suitable temperature and pH). A primer is preferably single stranded for maximum efficiency in amplification, but may alternatively be double stranded. If double stranded, the primer is first treated to separate its strands before being used to prepare extension products. The primer must be sufficiently long to prime the synthesis of extension products in the presence of the inducing agent. The exact length of a primer depends on a variety of factors, including temperature, source of primer, and the use of the method.

As used herein, the term "probe" refers to an oligonucleotide, whether occurring naturally as in a purified restriction digest or produced synthetically, recombinantly or by PCR amplification, which is typically capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule," so that it is detectable in any detection system, including, but not limited to enzyme (e.g., ELISA, as well as enzyme-based histochemical assays), fluorescent, radioactive, and luminescent systems. I t is not intended that the present invention be limited to any particular detection system or label.

As used herein, the term "target," when used in reference to the polymerase chain reaction, refers to the region of nucleic acid bounded by the primers used for polymerase chain reaction. Thus, the "target" is sought to be sorted out from other nucleic acid sequences. A nucleotide "segment" is a region of a nucleic acid within the target nucleic acid sequence.

As used herein, the term "polymerase chain reaction" (PCR) refers to the methods of U.S. Patent Nos. 4,683,195 4,683,202, and 4,965,188, which include methods for increasing the concentration of a segment of a target sequence in a mixture of genomic DNA without cloning or purification. This process for amplifying the target sequence is well known in the art.

As used herein, the term "amplification reagents" refers to those reagents (*e.g*., deoxyribonucleotide triphosphates, buffer, etc.) needed for amplification except for primers, nucleic acid template, and the amplification enzyme. Typically, amplification reagents along with other reaction components are placed and contained in a reaction vessel (test tube, microwell, etc.).

As used herein, the term "restriction endonuclease" or "restriction enzyme" refers to an enzyme (*e.g*., bacterial enzyme) that is capable of cutting double-stranded or single-stranded DNA at or near a specific sequence of nucleotides known as a restriction site. The nucleotide sequence comprising the restriction site is recognized and cleaved by a given restriction endonuclease or restriction enzyme and is frequently the site for insertion of DNA fragments. A restriction site can be engineered into an expression vector or DNA construct.

"Homologous recombination" refers to the exchange of DNA fragments between two DNA molecules or paired chromosomes at the site of identical or nearly identical nucleotide sequences.

A nucleic acid or polynucleotide is said to "encode" a polypeptide if, in its native state or when manipulated by methods known to those of skill in the art, it can be transcribed and/or translated to produce the polypeptide or a fragment thereof. The anti-sense strand of such a nucleic acid is also said to encode the sequence.

"Host strain" or "host cell" refers to a suitable host for an expression vector comprising a DNA sequence of interest.

A "protein" or "polypeptide" comprises a polymeric sequence of amino acid residues. The terms "protein" and "polypeptide" are used interchangeably herein. The single and 3-letter code for amino acids as defined in conformity with the IUPAC-IUB Joint Commission on Biochemical Nomenclature (JCBN) is used through out this disclosure. The single letter X refers to any of the twenty amino acids. It is also understood that a polypeptide may be coded for by more than one nucleotide sequence due to the degeneracy of the genetic code. Mutations can be named by the one letter code for the parent amino acid, followed by a position number and then the one letter code for the variant amino acid. For example, mutating glycine (G) at position 87 to serine (S) is represented as "G087S" or "G87S". Mutations can also be named by using the three letter code for an amino acid followed by its position in the polypeptide chain as counted from the N-terminus; for example, Ala10 for alanine at position 10. Multiple mutations are indicated by inserting a "-" between the mutations. Mutations at positions 87 and 90 are represented as either "G087S-A090Y" or "G87S-A90Y" or "G87S + A90Y" or "G087S + A090Y". For deletions, the one letter code "Z" is used. For an insertion relative to the parent sequence, the one letter code "Z" is on the left side of the position number. For a deletion, the one letter code "Z" is on the right side of the position number. For insertions, the position number is the position number before the inserted amino acid(s), plus 0.01 for each amino acid. For example, an insertion of three amino acids alanine (A), serine (S) and tyrosine (Y) between position 87 and 88 is shown as "Z087.01A-Z087.02S-Z087.03Y." Thus, combining all the mutations above plus a deletion at position 100 is: "G087S-Z087.01A-Z087.02S-Z087.03Y-A090Y-A100Z." When describing modifications, a position followed by amino acids listed in parentheses indicates a list of substitutions at that position by any of the listed amino acids. For example, 6(L,I) means position 6 can be substituted with a leucine or isoleucine.

A "prosequence" or "propeptide sequence" refers to an amino acid sequence between the signal peptide sequence and mature protease sequence that is necessary for the proper folding and secretion of the protease; they are sometimes referred to as intramolecular chaperones. Cleavage of the prosequence or propeptide sequence results in a mature active protease. Bacterial metalloproteases are often expressed as pro-enzymes.

The term "signal sequence" or "signal peptide" refers to a sequence of amino acid residues that may participate in the secretion or direct transport of the mature or precursor form of a protein. The signal sequence is typically located N-terminal to the precursor or mature protein sequence. The signal sequence may be endogenous or exogenous. A signal sequence is normally absent from the mature protein. A signal sequence is typically cleaved from the protein by a signal peptidase after the protein is transported.

The term "mature" form of a protein, polypeptide, or peptide refers to the functional form of the protein, polypeptide, or peptide without the signal peptide sequence and propeptide sequence.

The term "precursor" form of a protein or peptide refers to a mature form of the protein having a prosequence operably linked to the amino or carbonyl terminus of the protein. The precursor may also have a "signal" sequence operably linked to the amino terminus of the prosequence. The precursor may also have additional polypeptides that are involved in post-translational activity (e.g., polypeptides cleaved therefrom to leave the mature form of a protein or peptide).

The term "wild-type" in reference to an amino acid sequence or nucleic acid sequence indicates that the amino acid sequence or nucleic acid sequence is native or naturally occurring sequence. As used herein, the term "naturally-occurring" refers to anything (e.g., proteins, amino acids, or nucleic acid sequences) that are found in nature.

As used herein, the term "non-naturally occurring" refers to anything that is not found in nature (e.g., recombinant nucleic acids and protein sequences produced in the laboratory), as modification of the wild-type sequence.

As used herein with regard to amino acid residue positions, "corresponding to" or "corresponds to" or "corresponds" refers to an amino acid residue at the enumerated position in a protein or peptide, or an amino acid residue that is analogous, homologous, or equivalent to an enumerated residue in a protein or peptide. As used herein, "corresponding region" generally refers to an analogous position in a related proteins or a reference protein.

The terms "derived from" and "obtained from" refer to not only a protein produced or producible by a strain of the organism in question, but also a protein encoded by a DNA sequence isolated from such strain and produced in a host organism containing such DNA sequence. Additionally, the term refers to a protein which is encoded by a DNA sequence of synthetic and/or cDNA origin and which has the identifying characteristics of the protein in question. To exemplify, "proteases derived from *Bacillus"* refers to those enzymes having proteolytic activity which are naturally produced by *Bacillus,* as well as to serine proteases like those produced by *Bacillus* sources but which through the use of genetic engineering techniques are produced by non*-Bacillus* organisms transformed with a nucleic acid encoding the serine proteases.

The term "identical" in the context of two nucleic acids or polypeptide sequences refers to the residues in the two sequences that are the same when aligned for maximum correspondence, as measured using one of the following sequence comparison or analysis algorithms.

As used herein, "homologous genes" refers to a pair of genes from different, but usually related species, which correspond to each other and which are identical or very similar to each other. The term encompasses genes that are separated by speciation (i.e., the development of new species) (*e.g*., orthologous genes), as well as genes that have been separated by genetic duplication (*e.g*., paralogous genes).

As used herein, "% identity or percent identity" refers to sequence similarity. Percent identity may be determined using standard techniques known in the art *(See e.g.,* Smith and Waterman, Adv. Appl. Math. 2:482 [1981]; Needleman and Wunsch, J. Mol. Biol. 48:443 [1970]; Pearson and Lipman, Proc. Natl. Acad. Sci. USA 85:2444 [1988]; software programs such as GAP, BESTFIT, FASTA, and TFASTA in the Wisconsin Genetics Software Package (Genetics Computer Group, Madison, WI); and Devereux et al., Nucl. Acid Res. 12:387-395 [1984]). One example of a useful algorithm is PILEUP. PILEUP creates a multiple sequence alignment from a group of related sequences using progressive, pair-wise alignments. It can also plot a tree showing the clustering relationships used to create the alignment. PILEUP uses a simplification of the progressive alignment method of Feng and Doolittle *(See,* Feng and Doolittle, J. Mol. Evol. 35:351-360 [1987]). The method is similar to that described by Higgins and Sharp *(See,* Higgins and Sharp, CABIOS 5:151-153 [1989]). Useful PILEUP parameters include a default gap weight of 3.00, a default gap length weight of 0.10, and weighted end gaps. Other useful algorithm is the BLAST algorithms described by Altschul *et al.,* (*See,* Altschul et al., J. Mol. Biol. 215:403-410 [1990]; and Karlin and Altschul, Proc. Natl. Acad. Sci. USA 90:5873-5787 [1993]). The BLAST program uses several search parameters, most of which are set to the default values.

The NCBI BLAST algorithm finds the most relevant sequences in terms of biological similarity but is not recommended for query sequences of less than 20 residues (Altschul, SF et al. (1997) Nucleic Acids Res. 25:3389-3402 and Schaffer, AA et al. (2001) Nucleic Acids Res. 29:2994-3005). Example default BLAST parameters for a nucleic acid sequence searches are:
- Neighboring words threshold : 11
- E-value cutoff: 10
- Scoring Matrix : NUC.3.1 (match = 1, mismatch = -3)
- Gap Opening : 5
- Gap Extension : 2
- and the following parameters for amino acid sequence searches:
- Word size : 3
- E-value cutoff: 10
- Scoring Matrix : BLOSUM62
- Gap Opening : 11
- Gap extension : 1

A percent (%) amino acid sequence identity value is determined by the number of matching identical residues divided by the total number of residues of the "reference" sequence including any gaps created by the program for optimal/maximum alignment. If a sequence is 90% identical to SEQ ID NO: A, SEQ ID NO: A is the "reference" sequence. BLAST algorithms refer the "reference" sequence as "query" sequence.

The CLUSTAL W algorithm is another example of a sequence alignment algorithm. *See* Thompson et al. (1994) Nucleic Acids Res. 22:4673-4680. Default parameters for the CLUSTAL W algorithm are:

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |
| Toggle Residue specific penalties: | ON |
| Toggle hydrophilic penalties: | ON |
| Toggle end gap separation penalty | OFF. |

In CLUSTAL algorithms, deletions occurring at either terminus are included. For example, a variant with five amino acid deletion at either terminus (or within the polypeptide) of a polypeptide of 500 amino acids would have a percent sequence identity of 99% (495/500 identical residues × 100) relative to the "reference" polypeptide. Such a variant would be encompassed by a variant having "at least 99% sequence identity" to the polypeptide.

A polypeptide of interest may be said to be "substantially identical" to a reference polypeptide if the polypeptide of interest comprises an amino acid sequence having at least about 60%, least about 65%, least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 99.5% sequence identity to the amino acid sequence of the reference polypeptide. The percent identity between two such polypeptides can be determined manually by inspection of the two optimally aligned polypeptide sequences or by using software programs or algorithms (e.g., BLAST, ALIGN, CLUSTAL) using standard parameters. One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative amino acid substitution or one or more conservative amino acid substitutions.

A nucleic acid of interest may be said to be "substantially identical" to a reference nucleic acid if the nucleic acid of interest comprises a nucleotide sequence having least about 60%, least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 99.5% sequence identity to the nucleotide sequence of the reference nucleic acid. The percent identity between two such nucleic acids can be determined manually by inspection of the two optimally aligned nucleic acid sequences or by using software programs or algorithms (e.g., BLAST, ALIGN, CLUSTAL) using standard parameters. One indication that two nucleic acid sequences are substantially identical is that the two nucleic acid molecules hybridize to each other under stringent conditions (e.g., within a range of medium to high stringency).

A nucleic acid or polynucleotide is "isolated" when it is at least partially or completely separated from other components, including but not limited to for example, other proteins, nucleic acids, cells, etc. Similarly, a polypeptide, protein or peptide is "isolated" when it is at least partially or completely separated from other components, including but not limited to for example, other proteins, nucleic acids, cells, etc. On a molar basis, an isolated species is more abundant than are other species in a composition. For example, an isolated species may comprise at least about 60%, about 65%, about 70%, about 75%, about 80%, about 85%, about 90%, about 91%, about 92%, about 93%, about 94%, about 95%, about 96%, about 97%, about 98%, about 99%, or about 100% (on a molar basis) of all macromolecular species present. Preferably, the species of interest is purified to essential homogeneity (*i.e.,* contaminant species cannot be detected in the composition by conventional detection methods). Purity and homogeneity can be determined using a number of techniques well known in the art, such as agarose or polyacrylamide gel electrophoresis of a nucleic acid or a protein sample, respectively, followed by visualization upon staining. If desired, a high-resolution technique, such as high performance liquid chromatography (HPLC) or a similar means can be utilized for purification of the material.

"Hybridization" refers to the process by which one strand of nucleic acid forms a duplex with, *i.e.,* base pairs with, a complementary strand. A nucleic acid sequence is considered to be "selectively hybridizable" to a reference nucleic acid sequence if the two sequences specifically hybridize to one another under moderate to high stringency hybridization and wash conditions. Hybridization conditions are based on the melting temperature (Tm) of the nucleic acid binding complex or probe. For example, "maximum stringency" typically occurs at about Tm-5°C (5° below the Tm of the probe); "high stringency" at about 5-10°C below the Tm; "intermediate stringency" at about 10-20°C below the Tm of the probe; and "low stringency" at about 20-25°C below the Tm. Functionally, maximum stringency conditions can be used to identify sequences having strict identity or near-strict identity with the hybridization probe; while intermediate or low stringency hybridization can be used to identify or detect polynucleotide sequence homologs.

Moderate and high stringency hybridization conditions are well known in the art. Stringent hybridization conditions are exemplified by hybridization under the following conditions: 65°C and 0.1X SSC (where 1X SSC = 0.15 M NaCl, 0.015 M Na₃ citrate, pH 7.0). Hybridized, duplex nucleic acids are characterized by a melting temperature (Tₘ), where one half of the hybridized nucleic acids are unpaired with the complementary strand. Mismatched nucleic acids within the duplex lower the Tₘ. Very stringent hybridization conditions involve 68°C and 0.1X SSC. A nucleic acid encoding a variant metalloprotease can have a Tₘ reduced by 1°C - 3°C or more compared to a duplex formed between the nucleic acid of SEQ ID NO: 4 and its identical complement.

Another example of high stringency conditions includes hybridization at about 42°C in 50% formamide, 5X SSC, 5X Denhardt's solution, 0.5% SDS and 100 µg/ml denatured carrier DNA followed by washing two times in 2X SSC and 0.5% SDS at room temperature and two additional times in 0.1X SSC and 0.5% SDS at 42°C. An example of moderate stringent conditions include an overnight incubation at 37°C in a solution comprising 20% formamide, 5 × SSC (150mM NaCl, 15 mM trisodium citrate), 50 mM sodium phosphate (pH 7.6), 5 × Denhardt's solution, 10% dextran sulfate and 20 mg/ml denatured sheared salmon sperm DNA, followed by washing the filters in 1× SSC at about 37 - 50°C. Those of skill in the art know how to adjust the temperature, ionic strength, etc. to accommodate factors such as probe length and the like.

The term "purified" as applied to nucleic acids or polypeptides generally denotes a nucleic acid or polypeptide that is essentially free from other components as determined by analytical techniques well known in the art *(e.g.,* a purified polypeptide or polynucleotide forms a discrete band in an electrophoretic gel, chromatographic eluate, and/or a media subjected to density gradient centrifugation). For example, a nucleic acid or polypeptide that gives rise to essentially one band in an electrophoretic gel is "purified." A purified nucleic acid or polypeptide is at least about 50% pure, usually at least about 60%, about 65%, about 70%, about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8% or more pure (e.g., percent by weight on a molar basis). In a related sense, the present disclosure describes methods of enriching compositions for one or more molecules of the invention, such as one or more polypeptides of the invention or polynucleotides disclosed herein. A composition is enriched for a molecule when there is a substantial increase in the concentration of the molecule after application of a purification or enrichment technique. A substantially pure polypeptide of the invention or polynucleotide disclosed herein (*e.g*., substantially pure metalloprotease polypeptide or polynucleotide encoding a metalloprotease polypeptide of the invention, respectively) will typically comprise at least about 55%, about 60%, about 65%, about 70%, about 75%, about 80%, about 85%, about 90%, about 91%, about 92%, about 93%, about 94%, about 95%, about 96%, about 97%, about 98, about 99%, about 99.5% or more by weight (on a molar basis) of all macromolecular species in a particular composition.

The term "enriched" refers to a compound, polypeptide, cell, nucleic acid, amino acid, or other specified material or component that is present in a composition at a relative or absolute concentration that is higher than a starting composition.

In a related sense, the present disclosure describes methods of enriching compositions for one or more molecules of the invention, such as one or more polypeptides of the invention (*e.g*., one or more metalloprotease polypeptides of the invention) or one or more nucleic acids disclosed herein *(e.g.,* one or more nucleic acids encoding one or more metalloprotease polypeptides of the invention). A composition is enriched for a molecule when there is a substantial increase in the concentration of the molecule after application of a purification or enrichment technique. A substantially pure polypeptide or polynucleotide will typically comprise at least about 55%, about 60%, about 65%, about 70%, about 75%, about 80%, about 85%, about 90%, about 91%, about 92%, about 93%, about 94%, about 95%, about 96%, about 97%, about 98, about 99%, about 99.5% or more by weight (on a molar basis) of all macromolecular species in a particular composition.

As used herein, the term "combinatorial mutagenesis" or "combinatorial" refers to methods in which libraries of nucleic acid variants of a reference nucleic acid sequence are generated. In these libraries, the variants contain one or several mutations chosen from a predefined set of mutations. The methods also provide means to introduce random mutations which were not members of the predefined set of mutations. Some such methods include those set forth in U.S. Patent No. 6,582,914. Some such combinatorial mutagenesis methods include and/or encompass methods embodied in commercially available kits (*e.g*., QUIKCHANGE^{®} Multi Site-Directed Mutagenesis Kit (Stratagene), PCR fusion/extension PCR).

As used herein, "having improved properties" used in connection with a variant protease refers to a variant protease with improved or enhanced wash or cleaning performance, and/or improved or enhanced stability optionally with retained wash or cleaning performance, relative to the corresponding reference protease (*e.g*., wild-type or naturally-occurring protease). The improved properties of a variant protease may comprise improved wash or cleaning performance and/or improved stability. In some embodiments not covered by the invention, the invention provides variant proteases of the invention that exhibit one of more of the following properties: improved hand wash performance, improved hand or manual dishwashing performance, improved automatic dishwashing performance, improved laundry performance, and/or improved stability relative to a reference protease (*e.g*., wild-type protease, such as a wild-type thermolysin).

As used herein, the term "functional assay" refers to an assay that provides an indication of a protein's activity. For example, in the case of enzymes, a functional assay involves determining the effectiveness of the enzyme in catalyzing a reaction.

As used herein, the term "target property" refers to the property of the starting gene that is to be altered. It is not intended that the present invention be limited to any particular target property.

The term "property" or grammatical equivalents thereof in the context of a nucleic acid, as used herein, refer to any characteristic or attribute of a nucleic acid that can be selected or detected. These properties include, but are not limited to, a property affecting binding to a polypeptide, a property conferred on a cell comprising a particular nucleic acid, a property affecting gene transcription (e.g., promoter strength, promoter recognition, promoter regulation, enhancer function), a property affecting RNA processing (*e.g*., RNA splicing, RNA stability, RNA conformation, and post-transcriptional modification), a property affecting translation (*e.g*., level, regulation, binding of mRNA to ribosomal proteins, post-translational modification). For example, a binding site for a transcription factor, polymerase, regulatory factor, etc., of a nucleic acid may be altered to produce desired characteristics or to identify undesirable characteristics.

The term "property" or grammatical equivalents thereof in the context of a polypeptide (including proteins), as used herein, refer to any characteristic or attribute of a polypeptide that can be selected or detected. These properties include, but are not limited to oxidative stability, substrate specificity, catalytic activity, enzymatic activity, thermal stability, alkaline stability, pH activity profile, resistance to proteolytic degradation, K_{M}, k_{cat}, k_{cat}/k_{M} ratio, protein folding, inducing an immune response, ability to bind to a ligand, ability to bind to a receptor, ability to be secreted, ability to be displayed on the surface of a cell, ability to oligomerize, ability to signal, ability to stimulate cell proliferation, ability to inhibit cell proliferation, ability to induce apoptosis, ability to be modified by phosphorylation or glycosylation, and/or ability to treat disease, etc.

As used herein, the term "screening" has its usual meaning in the art. In one exemplary screening process, a mutant nucleic acid or variant polypeptide encoded therefrom is provided and a property of the mutant nucleic acid or variant polypeptide, respectively, is assessed or determined. The determined property of the mutant nucleic acid or variant polypeptide may be compared to a property of the corresponding precursor (parent) nucleic acid or to the property of the corresponding parent polypeptide, respectively.

It will be apparent to the skilled artisan that the screening procedure for obtaining a nucleic acid or protein with an altered property depends upon the property of the starting material the modification of which the generation of the mutant nucleic acid is intended to facilitate. The skilled artisan will therefore appreciate that the invention is not limited to any specific property to be screened for and that the following description of properties lists illustrative examples only. Methods for screening for any particular property are generally described in the art. For example, one can measure binding, pH, specificity, etc., before and after mutation, wherein a change indicates an alteration. Preferably, the screens are performed in a high-throughput manner, including multiple samples being screened simultaneously, including, but not limited to assays utilizing chips, phage display, and multiple substrates and/or indicators.

The terms "modified nucleic acid sequence" and "modified gene" are used interchangeably herein to refer to a nucleic acid sequence that includes a deletion, insertion or interruption of naturally occurring (*i.e.,* wild-type) nucleic acid sequence. Thus, an insertion may result in either a truncated protein or an elongated protein as an expression product.

A "mutant" nucleic acid sequence typically refers to a nucleic acid sequence that has an alteration in at least one codon occurring in a host cell's wild-type sequence such that the expression product of the mutant nucleic acid sequence is a protein with an altered amino acid sequence relative to the wild-type protein. The expression product may have an altered functional capacity (*e.g*., enhanced enzymatic activity).

As used herein, the phrase "alteration in substrate specificity" refers to changes in the substrate specificity of an enzyme. The substrate specificity of an enzyme is determined by comparing the catalytic efficiencies it exhibits with different substrates. These determinations find particular use in assessing the efficiency of mutant enzymes, as it is generally desired to produce variant enzymes that exhibit greater ratios of k_{cat}/Kₘ for substrates of interest. However, it is not intended that the present invention be limited to any particular substrate composition or substrate specificity.

As used herein, "surface property" is used in reference to electrostatic charge, as well as properties such as the hydrophobicity and hydrophilicity exhibited by the surface of a protein.

As used herein, the term "net charge" is defined as the sum of all charges present in a molecule. "Net charge changes" are made to a parent protein molecule to provide a variant that has a net charge that differs from that of the parent molecule (*i.e.,* the variant has a net charge that is not the same as that of the parent molecule). For example, substitution of a neutral amino acid with a negatively charged amino acid or a positively charged amino acid with a neutral amino acid results in net charge of -1 with respect to the parent molecule. Substitution of a positively charged amino acid with a negatively charged amino acid results in a net charge of -2 with respect to the parent. Substitution of a neutral amino acid with a positively charged amino acid or a negatively charged amino acid with a neutral amino acid results in net charge of +1 with respect to the parent. Substitution of a negatively charged amino acid with a positively charged amino acid results in a net charge of +2 with respect to the parent. The net charge of a parent protein can also be altered by deletion and/or insertion of charged amino acids

The terms "thermally stable" and "thermostable" and "thermostability" refer to proteases that retain a specified amount of enzymatic activity after exposure to identified temperatures over a given period of time under conditions prevailing during the proteolytic, hydrolyzing, cleaning or other process of the invention, while being exposed to altered temperatures. "Altered temperatures" encompass increased or decreased temperatures.

The term "enhanced stability" in the context of an oxidation, chelator, thermal and/or pH stable protease refers to a higher retained proteolytic activity over time as compared to other proteases (*e.g*., thermolysin proteases) and/or wild-type enzymes.

The term "diminished stability" in the context of an oxidation, chelator, thermal and/or pH stable protease refers to a lower retained proteolytic activity over time as compared to other proteases (*e.g*., thermolysin proteases) and/or wild-type enzymes.

The term "cleaning activity" refers to a cleaning performance achieved by a variant protease or reference protease under conditions prevailing during the proteolytic, hydrolyzing, cleaning, or other process of the inventionAs disclosed herein, cleaning performance of a variant protease or reference protease may be determined by using various assays for cleaning one or more various enzyme sensitive stains on an item or surface (*e.g*., a stain resulting from food, grass, blood, ink, milk, oil, and/or egg protein). Cleaning performance of a variant or reference protease can be determined by subjecting the stain on the item or surface to standard wash condition(s) and assessing the degree to which the stain is removed by using various chromatographic, spectrophotometric, or other quantitative methodologies. Exemplary cleaning assays and methods are known in the art and include, but are not limited to those described in WO 99/34011 and U.S. Pat. 6,605,458 , as well as those cleaning assays and methods included in the Examples provided below. The cleaning performance of variant proteases of the present invention is determined using the assays described in Example 1.

The term "cleaning effective amount" of a variant protease or reference protease refers to the amount of protease that achieves a desired level of enzymatic activity in a specific cleaning composition. Such effective amounts are readily ascertained by one of ordinary skill in the art and are based on many factors, such as the particular protease used, the cleaning application, the specific composition of the cleaning composition, and whether a liquid or dry (*e.g*., granular, tablet, bar) composition is required, etc.

The term "cleaning adjunct material" refers to any liquid, solid, or gaseous material included in cleaning composition other than a variant protease of the invention. Each cleaning adjunct material is typically selected depending on the particular type and form of cleaning composition (*e.g*., liquid, granule, powder, bar, paste, spray, tablet, gel, foam, or other composition). Preferably, each cleaning adjunct material is compatible with the protease enzyme used in the composition.

The term "enhanced performance" in the context of cleaning activity refers to an increased or greater cleaning activity by an enzyme on certain enzyme sensitive stains such as egg, milk, grass, ink, oil, and/or blood, as determined by usual evaluation after a standard wash cycle and/or multiple wash cycles.

The term "diminished performance" in the context of cleaning activity refers to a decreased or lesser cleaning activity by an enzyme on certain enzyme sensitive stains such as egg, milk, grass or blood, as determined by usual evaluation after a standard wash cycle.

Cleaning performance can be determined by comparing the variant proteases of the present invention with reference proteases in various cleaning assays concerning enzyme sensitive stains such as grass, blood, ink, oil, and/or milk as determined by usual spectrophotometric or analytical methodologies after standard wash cycle conditions. The cleaning performance of variant proteases of the present invention is determined using the assays described in Example 1.

As used herein, the term "consumer product" means fabric and home care product. As used herein, the term "fabric and home care product" or "fabric and household care product" includes products generally intended to be used or consumed in the form in which they are sold and that are for treating fabrics, hard surfaces and any other surfaces, and cleaning systems all for the care and cleaning of inanimate surfaces, as well as fabric conditioner products and other products designed specifically for the care and maintenance of fabrics, and air care products, including: air care including air fresheners and scent delivery systems, car care, pet care, livestock care, personal care, jewelry care, dishwashing, fabric conditioning (including softening and/or freshening), laundry detergency, laundry and rinse additive and/or care, pre-treatment cleaning compositions, hard surface cleaning and/or treatment including floor and toilet bowl cleaners, glass cleaners and/or treatments, tile cleaners and /or treatments, ceramic cleaners and/or treatments, and other cleaning for consumer or institutional use. "Fabric and home care product" includes consumer and institutional products.

As used herein, the term "non-fabric and home care products" refers to compositions that are added to other compositions to produce an end product that may be a fabric and home care product.

As used herein, the term "institutional cleaning composition" refers to products suitable for use in institutions including but not limited to schools, hospitals, factories, stores, corporations, buildings, restaurants, office complexes and buildings, processing and/or manufacturing plants, veterinary hospitals, factory farms, factory ranches, etc.

As used herein, the term "cleaning and/or treatment composition" is a subset of fabric and home care products that includes, unless otherwise indicated, compositions suitable for cleaning and/or treating items. Such products include, but are not limited to, products for treating fabrics, hard surfaces and any other surfaces in the area of fabric and home care, including: air care including air fresheners and scent delivery systems, car care, dishwashing, fabric conditioning (including softening and/or freshening), laundry detergency, laundry and rinse additive and/or care, hard surface cleaning and/or treatment including floor and toilet bowl cleaners, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use: car or carpet shampoos, bathroom cleaners including toilet bowl cleaners; as well as cleaning auxiliaries such as bleach additives and "stain-stick" or pre-treat types, substrate-laden products such as dryer added sheets.

Indeed, as used herein, "cleaning composition" or "cleaning formulation" of the invention refers to any composition of the invention useful for removing or eliminating a compound (*e.g*., undesired compound) from an object, item or surface to be cleaned, including, but not limited to for example, a fabric, fabric item, dishware item, tableware item, glassware item, contact lens, other solid substrate, hair (shampoo) (including human or animal hair), skin (soap or and cream), teeth (mouthwashes, toothpastes), surface of an item or object (*e.g*., hard surfaces, such as the hard surface of a table, table top, wall, furniture item, floor, ceiling, non-dishware item, non-tableware item, etc.), filters, membranes (*e.g*., filtration membranes, including but not limited to ultrafiltration membranes), etc. The term encompasses any material and/or added compound selected for the particular type of cleaning composition desired and the form of the product (*e.g*., liquid, gel, granule, spray, or other composition), as long as the composition is compatible with the protease and other enzyme(s) used in the composition. The specific selection of cleaning composition materials are readily made by considering the surface, object, item, or fabric to be cleaned, and the desired form of the composition for the cleaning conditions during use.

Cleaning compositions and cleaning formulations include any composition that is suited for cleaning, bleaching, disinfecting, and/or sterilizing any object, item, and/or surface. Such compositions and formulations include, but are not limited to for example, liquid and/or solid compositions, including cleaning or detergent compositions (*e.g*., liquid, tablet, gel, bar, granule, and/or solid laundry cleaning or detergent compositions and fine fabric detergent compositions; hard surface cleaning compositions and formulations, such as for glass, wood, ceramic and metal counter tops and windows; carpet cleaners; oven cleaners; fabric fresheners; fabric softeners; and textile, laundry booster cleaning or detergent compositions, laundry additive cleaning compositions, and laundry pre-spotter cleaning compositions; dishwashing compositions, including hand or manual dishwash compositions (*e.g*., "hand" or "manual" dishwashing detergents) and automatic dishwashing compositions (*e.g*., "automatic dishwashing detergents") .

Cleaning composition or cleaning formulations, as used herein, include, unless otherwise indicated, granular or powder-form all-purpose or heavy-duty washing agents, especially cleaning detergents; liquid, granular, gel, solid, tablet, or paste-form all-purpose washing agents, especially the so-called heavy-duty liquid (HDL) detergent or heavy-duty powder detergent (HDD) types; liquid fine-fabric detergents; hand or manual dishwashing agents, including those of the high-foaming type; hand or manual dishwashing, automatic dishwashing, or dishware or tableware washing agents, including the various tablet, powder, solid, granular, liquid, gel, and rinse-aid types for household and institutional use; liquid cleaning and disinfecting agents, including antibacterial hand-wash types, cleaning bars, mouthwashes, denture cleaners, car shampoos, carpet shampoos, bathroom cleaners; hair shampoos and/or hair-rinses for humans and other animals; shower gels and foam baths and metal cleaners; as well as cleaning auxiliaries, such as bleach additives and "stain-stick" or pre-treat types.

As used herein, "fabric cleaning compositions" include hand and machine laundry detergent compositions including laundry additive compositions and compositions suitable for use in the soaking and/or pretreatment of stained fabrics (*e.g*., clothes, linens, and other textile materials).

As used herein, "non-fabric cleaning compositions" include non-textile (*i.e.,* non-fabric) surface cleaning compositions, including, but not limited to for example, hand or manual or automatic dishwashing detergent compositions, oral cleaning compositions, denture cleaning compositions, and personal cleansing compositions.

As used herein, the term "fabric and/or hard surface cleaning and/or treatment composition" is a subset of cleaning and treatment compositions that includes, unless otherwise indicated, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, car or carpet shampoos, bathroom cleaners including toilet bowl cleaners; fabric conditioning products including softening and/or freshening that may be in liquid, solid and/or dryer sheet form ; as well as cleaning auxiliaries such as bleach additives and "stain-stick" or pre-treat types, substrate-laden products such as dryer added sheets. All of such products which are applicable may be in standard, concentrated or even highly concentrated form even to the extent that such products may in certain aspect be non-aqueous.

As used herein, the term "detergent composition" or "detergent formulation" is used in reference to a composition intended for use in a wash medium for the cleaning of soiled or dirty objects, including particular fabric and/or non-fabric objects or items. Such compositions of the present invention are not limited to any particular detergent composition or formulation.

As used herein, the term "bleaching" refers to the treatment of a material (*e.g*., fabric, laundry, pulp, etc.) or surface for a sufficient length of time and/or under appropriate pH and/or temperature conditions to effect a brightening (*i.e.,* whitening) and/or cleaning of the material. Examples of chemicals suitable for bleaching include, but are not limited to, for example, ClO₂, H₂O₂, peracids, NO₂, etc.

As used herein, "wash performance" of a protease (*e.g*., a variant protease of the invention) refers to the contribution of a variant protease to washing that provides additional cleaning performance to the detergent as compared to the detergent without the addition of the variant protease to the composition. Wash performance is compared under relevant washing conditions. In some test systems, other relevant factors, such as detergent composition, sud concentration, water hardness, washing mechanics, time, pH, and/or temperature, can be controlled in such a way that condition(s) typical for household application in a certain market segment (*e.g.*, hand or manual dishwashing, automatic dishwashing, dishware cleaning, tableware cleaning, fabric cleaning, etc.) are imitated.

The term "relevant washing conditions" is used herein to indicate the conditions, particularly washing temperature, time, washing mechanics, sud concentration, type of detergent and water hardness, actually used in households in a hand dishwashing, automatic dishwashing, or laundry detergent market segment.

The term "improved wash performance" is used to indicate that a better end result is obtained in stain removal under relevant washing conditions, or that less variant protease, on weight basis, is needed to obtain the same end result relative to the corresponding wild-type or starting parent protease.

As used herein, the term "disinfecting" refers to the removal of contaminants from the surfaces, as well as the inhibition or killing of microbes on the surfaces of items. It is not intended that the present invention be limited to any particular surface, item, or contaminant(s) or microbes to be removed.

The "compact" form of the cleaning compositions herein is best reflected by density and, in terms of composition, by the amount of inorganic filler salt. Inorganic filler salts are conventional ingredients of detergent compositions in powder form. In conventional detergent compositions, the filler salts are present in substantial amounts, typically about 17 to about 35% by weight of the total composition. In contrast, in compact compositions, the filler salt is present in amounts not exceeding about 15% of the total composition..

The position of an amino acid residue in a given amino acid sequence is typically numbered herein using the numbering of the position of the corresponding amino acid residue of the *G. caldoproteolyticus* thermolysin amino acid sequence shown in SEQ ID NO: 3. The *G. caldoproteolyticus* thermolysin amino acid sequence shown in SEQ ID NO: 3, thus serves as a reference sequence. A given amino acid sequence, such as a variant protease amino acid sequence described herein, can be aligned with the *G. caldoproteolyticus* sequence (SEQ ID NO: 3) using an alignment algorithm as described herein, and an amino acid residue in the given amino acid sequence that aligns (preferably optimally aligns) with an amino acid residue in the *G. caldoproteolyticus* sequence can be conveniently numbered by reference to the corresponding amino acid residue in the thermolysin *G. caldoproteolyticus* sequence.

Generally, the nomenclature used herein and many of the laboratory procedures in cell culture, molecular genetics, molecular biology, nucleic acid chemistry, and protein chemistry described below are well known and commonly employed by those of ordinary skill in the art. Methods for production and manipulation of recombinant nucleic acid methods, nucleic acid synthesis, cell culture methods, and transgene incorporation (*e.g*., transfection, electroporation) are known to those skilled in the art and are described in numerous standard texts. Oligonucleotide synthesis and purification steps are typically performed according to specifications. Techniques and procedures are generally performed according to conventional methods well known in the art and various general references that are provided throughout this document. Procedures therein are believed to be well known to those of ordinary skill in the art and are provided for the convenience of the reader.

### Thermolysin enzymes of the invention

As used herein, a thermolysin enzyme includes an enzyme, polypeptide, or protein, or an active fragment thereof, exhibiting a proteolytic activity. This includes members of the peptidase family M4 of which thermolysin (TLN; EC 3.4.24.27) is the prototype.

### Productive Positions of thermolysin enzymes

The stability of thermolysin enzymes of the present invention can be compared to the stability of a standard, for example, the *G. caldoproteolyticus* thermolysin of SEQ ID NO: 3.

The terms "thermal stability" and "thermostability" refer to thermolysins of the present disclosure that retain a specified amount of enzymatic activity after exposure to an identified temperature, often over a given period of time under conditions prevailing during the proteolytic, hydrolyzing, cleaning or other process disclosed herein, for example while exposed to altered temperatures. Altered temperatures include increased or decreased temperatures..

As used herein, improved properties of a variant thermolysin enzyme includes a variant thermolysin enzyme with improved or enhanced wash or cleaning performance, and/or improved or enhanced stability optionally with retained wash or cleaning performance, relative to the corresponding parent thermolysin enzyme (*e.g*., wild-type or naturally-occurring thermolysin enzyme). The improved properties of a variant thermolysin enzyme may comprise improved wash or cleaning performance and/or improved stability.

Productive positions are described as those positions within a molecule that are most useful for making combinatorial variants exhibiting an improved characteristic, where the position itself allows for at least one combinable mutation. Combinable mutations can be described as those substitutions in a molecule that can be used to make combinatorial variants. Combinable mutations are ones that improve at least one desired property of the molecule, while not significantly decreasing either: expression, activity, or stability.

Combinable mutations are ones that improve at least one desired property of the molecule, while not significantly decreasing either: expression, activity, or stability. For example, Combinable mutations in thermolysin can be determined using performance index (PI) values resulting from the assays described in Example 1: Abz-AGLA-Nba protease assay (activity), PAS-38 microswatch assay (activity), detergent stability and thermostability assays, and protein determination (expression).

In addition to Combinable mutations, a second group of mutations for thermolysin is Activity Combinable mutations. Activity Combinable mutations are ones that improve at least one activity property of the molecule, with a performance index greater than or equal to 1.5, while not decreasing either expression or stability PI values below 0.5. These Activity Combinable mutations can be used to modify the molecule in order to achieve a desired property without significantly decreasing other known and desired properties of the molecule (e.g. expression or stability).

Thermolysin enzyme amino acid positions found to be useful positions can have different modifications that are suitable for use in a detergent composition. Modifications can include an insertion, deletion or substitution at the particular position. For example, amino acid positions can have at least 75%, 40% or 15% of the modifications tested at a productive position as suitable modifications, wherein the modification meets at least one of the following suitability criteria:
a) a position wherein the minimum performance indices (PI) relative to Thermolysin parent for PAS-38 microswatch cleaning at pH6 or pH8, activity on Abz-AGLA-Nba, detergent stability and thermostability are greater than or equal to 0.9, and in addition have a PI for any one of these tests that is greater than or equal to 1.0;
b) a position wherein the minimum performance indices (PI) relative to Thermolysin parent for PAS-38 microswatch cleaning at pH6 or pH8, activity on Abz-AGLA-Nba, detergent stability and thermostability are greater than or equal to 0.8, and in addition have a PI for any one of these tests that is greater than or equal to 1.2; or
c) a position wherein the minimum performance indices (PI) relative to Thermolysin parent for PAS-38 microswatch cleaning at pH6 or pH8, activity on Abz-AGLA-Nba, detergent stability and thermostability are greater than or equal to 0.5, and in addition have a PI for any one of these tests that is greater than or equal to 1.5.

### Polypeptides of the Invention

The present invention provides novel polypeptides, which may be collectively referred to as "polypeptides of the invention", provided that such polypeptides are as defined in the claims. Polypeptides of the invention include isolated, recombinant, substantially pure, or non-naturally occurring variant thermolysin enzyme polypeptides, including for example, variant thermolysin enzyme polypeptides, having enzymatic activity (e.g., thermolysin activity), as defined in the claims. In some embodiments, polypeptides of the invention are useful in cleaning applications and can be incorporated into cleaning compositions that are useful in methods of cleaning an item or a surface (*e.g*., of surface of an item) in need of cleaning.

Described are compositions and methods relating to thermolysin cloned from *Geobacillus caldoproteolyticus.* The compositions and methods are based, in part, on the observation that cloned and expressed thermolysin has proteolytic activity in the presence of a detergent composition. Thermolysin also demonstrates excellent stability in detergent compositions. These features of thermolysin makes it well suited for use in a variety of cleaning applications, where the enzyme can hydrolyze proteins in the presence of surfactants and other components found in detergent compositions.

As noted above, the variant thermolysin enzyme polypeptides of the invention have enzymatic activities (*e.g*., thermolysin activities) and thus are useful in cleaning applications, including but not limited to, methods for cleaning dishware items, tableware items, fabrics, and items having hard surfaces (*e.g*., the hard surface of a table, table top, wall, furniture item, floor, ceiling, etc.). Exemplary cleaning compositions comprising one or more variant thermolysin enzyme polypeptides of the invention are described *infra.* The enzymatic activity (*e.g.,* thermolysin enzyme activity) of a variant thermolysin enzyme polypeptide of the invention can be determined readily using procedures well known to those of ordinary skill in the art. The Examples presented *infra* describe methods for evaluating the enzymatic activity, cleaning performance, detergent stability and/or thermostability. The performance of variant thermolysin enzymes of the invention in removing stains (*e.g*., a lipid stain), cleaning hard surfaces, or cleaning laundry, dishware or tableware item(s) can be readily determined using procedures well known in the art and/or by using procedures set forth in the Examples.

### IMethods for Making Modified Variant Proteases of the Invention

A variety of methods are known in the art that are suitable for generating modified polynucleotides of the disclosure that encode variant proteases of the invention, including, but not limited to, for example, site-saturation mutagenesis, scanning mutagenesis, insertional mutagenesis, deletion mutagenesis, random mutagenesis, site-directed mutagenesis, and directed-evolution, as well as various other recombinatorial approaches. Methods for making modified polynucleotides and proteins (*e.g*., variant proteases) include DNA shuffling methodologies, methods based on non-homologous recombination of genes, such as ITCHY *(See,* Ostermeier *et al.,* 7:2139-44 [1999]), SCRACHY *(See,* Lutz *et al.* 98:11248-53 [2001]), SHIPREC *(See,* Sieber *et al.,* 19:456-60 [2001]), and NRR *(See,* Bittker *et al.,* 20:1024-9 [2001]; Bittker *et al.,* 101:7011-6 [2004]), and methods that rely on the use of oligonucleotides to insert random and targeted mutations, deletions and/or insertions *(See,* Ness *et al.,* 20:1251-5 [2002]; Coco *et al.,* 20:1246-50 [2002]; Zha *et al.,* 4:34-9 [2003]; Glaser *et al.,* 149:3903-13 [1992]).

### Cleaning Compositions

Unless otherwise noted, all component or composition levels provided herein are made in reference to the active level of that component or composition, and are exclusive of impurities, for example, residual solvents or by-products, which may be present in commercially available sources. Enzyme components weights are based on total active protein. All percentages and ratios are calculated by weight unless otherwise indicated. All percentages and ratios are calculated based on the total composition unless otherwise indicated. In the exemplified detergent compositions, the enzymes levels are expressed by pure enzyme by weight of the total composition and unless otherwise specified, the detergent ingredients are expressed by weight of the total compositions.

As indicated herein, in some embodiments, the cleaning compositions of the present invention further comprise adjunct materials including, but not limited to, surfactants, builders, bleaches, bleach activators, bleach catalysts, other enzymes, enzyme stabilizing systems, chelants, optical brighteners, soil release polymers, dye transfer agents, dispersants, suds suppressors, dyes, perfumes, colorants, filler salts, hydrotropes, photoactivators, fluorescers, fabric conditioners, hydrolyzable surfactants, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, and pH control agents (*See e.g*., U.S. Pat. Nos. 6,610,642, 6,605,458, 5,705,464, 5,710,115, 5,698,504, 5,695,679, 5,686,014 and 5,646,101). Embodiments of specific cleaning composition materials are exemplified in detail below. In embodiments in which the cleaning adjunct materials are not compatible with the variant proteases of the present invention in the cleaning compositions, then suitable methods of keeping the cleaning adjunct materials and the protease(s) separated (*i.e.,* not in contact with each other) until combination of the two components is appropriate are used. Such separation methods include any suitable method known in the art (*e.g*., gelcaps, encapsulation, tablets, physical separation, etc.).

The cleaning compositions of the present invention are advantageously employed for example, in laundry applications, hard surface cleaning, dishwashing applications, as well as cosmetic applications such as dentures, teeth, hair and skin. In addition, due to the unique advantages of increased effectiveness in lower temperature solutions, the enzymes of the present invention are ideally suited for laundry applications. Furthermore, the enzymes of the present invention find use in granular and liquid compositions.

The variant proteases of the present invention also find use in cleaning additive products.

The present cleaning compositions and cleaning additives require an effective amount of at least one of the protease variants provided herein, alone or in combination with other proteases and/or additional enzymes. The required level of enzyme is achieved by the addition of one or more protease variants of the present invention. Typically the present cleaning compositions comprise at least about 0.0001 weight percent, from about 0.0001 to about 10, from about 0.001 to about 1, or even from about 0.01 to about 0.1 weight percent of at least one of the variant proteases of the present invention.

The cleaning compositions herein are typically formulated such that, during use in aqueous cleaning operations, the wash water will have a pH of from about 5.0 to about 11.5 or even from about 7.5 to about 10.5. Liquid product formulations are typically formulated to have a neat pH from about 3.0 to about 9.0 or even from about 3 to about 5. Granular laundry products are typically formulated to have a pH from about 9 to about 11. Techniques for controlling pH at recommended usage levels include the use of buffers, alkalis, acids, etc., and are well known to those skilled in the art.

Suitable "low pH cleaning compositions" typically have a neat pH of from about 3 to about 5, and are typically free of surfactants that hydrolyze in such a pH environment. Such surfactants include sodium alkyl sulfate surfactants that comprise at least one ethylene oxide moiety or even from about 1 to about 16 moles of ethylene oxide. Such cleaning compositions typically comprise a sufficient amount of a pH modifier, such as sodium hydroxide, monoethanolamine or hydrochloric acid, to provide such cleaning composition with a neat pH of from about 3 to about 5. Such compositions typically comprise at least one acid stable enzyme.

In some embodiments not covered by the present invention, when the variant protease(s) is/are employed in a granular composition or liquid, it is desirable for the variant protease to be in the form of an encapsulated particle to protect the variant protease from other components of the granular composition during storage. In addition, encapsulation is also a means of controlling the availability of the variant protease during the cleaning process. In some embodiments not covered by the present invention, encapsulation enhances the performance of the variant protease(s) and/or additional enzymes. In this regard, the variant proteases of the present invention are encapsulated with any suitable encapsulating material known in the art. In some embodiments not covered by the present invention, the encapsulating material typically encapsulates at least part of the catalyst for the variant protease(s) of the present invention. Typically, the encapsulating material is water-soluble and/or water-dispersible. In some embodiments not covered by the present invention, the encapsulating material has a glass transition temperature (Tg) of 0°C or higher. Glass transition temperature is described in more detail in WO 97/11151. The encapsulating material is typically selected from consisting of carbohydrates, natural or synthetic gums, chitin, chitosan, cellulose and cellulose derivatives, silicates, phosphates, borates, polyvinyl alcohol, polyethylene glycol, paraffin waxes, and combinations thereof. When the encapsulating material is a carbohydrate, it is typically selected from monosaccharides, oligosaccharides, polysaccharides, and combinations thereof. In some typical embodiments not covered by the present invention, the encapsulating material is a starch (*See e.g*., EP 0 922 499; US 4,977,252; US 5,354,559, and US 5,935,826). In some embodiments not covered by the present invention, the encapsulating material is a microsphere made from plastic such as thermoplastics, acrylonitrile, methacrylonitrile, polyacrylonitrile, polymethacrylonitrile and mixtures thereof; commercially available microspheres that find use include, but are not limited to those supplied by EXPANCEL^{®} (Stockviksverken, Sweden), and PM 6545, PM 6550, PM 7220, PM 7228, EXTENDOSPHERES^{®}, LUXSIL^{®}, Q-CEL^{®}, and SPHERICEL^{®} (PQ Corp., Valley Forge, PA).]

As described herein, the variant proteases of the present invention find particular use in the cleaning industry, including, but not limited to laundry and dish detergents. These applications place enzymes under various environmental stresses. The variant proteases of the present invention provide advantages over many currently used enzymes, due to their stability under various conditions.

Indeed, there are a variety of wash conditions including varying detergent formulations, wash water volumes, wash water temperatures, and lengths of wash time, to which proteases involved in washing are exposed. In addition, detergent formulations used in different geographical areas have different concentrations of their relevant components present in the wash water. For example, European detergents typically have about 4500-5000 ppm of detergent components in the wash water, while Japanese detergents typically have approximately 667 ppm of detergent components in the wash water. In North America, particularly the United States, detergents typically have about 975 ppm of detergent components present in the wash water.

A low detergent concentration system includes detergents where less than about 800 ppm of the detergent components are present in the wash water. Japanese detergents are typically considered low detergent concentration system as they have approximately 667 ppm of detergent components present in the wash water.

A medium detergent concentration includes detergents where between about 800 ppm and about 2000ppm of the detergent components are present in the wash water. North American detergents are generally considered to be medium detergent concentration systems as they have approximately 975 ppm of detergent components present in the wash water. Brazil typically has approximately 1500 ppm of detergent components present in the wash water.

A high detergent concentration system includes detergents where greater than about 2000 ppm of the detergent components are present in the wash water. European detergents are generally considered to be high detergent concentration systems as they have approximately 4500-5000 ppm of detergent components in the wash water.

Latin American detergents are generally high suds phosphate builder detergents and the range of detergents used in Latin America can fall in both the medium and high detergent concentrations as they range from 1500 ppm to 6000 ppm of detergent components in the wash water. As mentioned above, Brazil typically has approximately 1500 ppm of detergent components present in the wash water. However, other high suds phosphate builder detergent geographies, not limited to other Latin American countries, may have high detergent concentration systems up to about 6000 ppm of detergent components present in the wash water.

In light of the foregoing, it is evident that concentrations of detergent compositions in typical wash solutions throughout the world varies from less than about 800 ppm of detergent composition ("low detergent concentration geographies"), for example about 667 ppm in Japan, to between about 800 ppm to about 2000 ppm ("medium detergent concentration geographies"), for example about 975 ppm in U.S. and about 1500 ppm in Brazil, to greater than about 2000 ppm ("high detergent concentration geographies"), for example about 4500 ppm to about 5000 ppm in Europe and about 6000 ppm in high suds phosphate builder geographies.

The concentrations of the typical wash solutions are determined empirically. For example, in the U.S., a typical washing machine holds a volume of about 64.4 L of wash solution. Accordingly, in order to obtain a concentration of about 975 ppm of detergent within the wash solution about 62.79 g of detergent composition must be added to the 64.4 L of wash solution. This amount is the typical amount measured into the wash water by the consumer using the measuring cup provided with the detergent.

As a further example, different geographies use different wash temperatures. The temperature of the wash water in Japan is typically less than that used in Europe. For example, the temperature of the wash water in North America and Japan is typically between about 10 and about 30°C (*e.g.*, about 20°C), whereas the temperature of wash water in Europe is typically between about 30 and about 60°C (*e.g*., about 40°C). However, in the interest of saving energy, many consumers are switching to using cold water washing. In addition, in some further regions, cold water is typically used for laundry, as well as dish washing applications.

As a further example, different geographies typically have different water hardness. Water hardness is usually described in terms of the grains per gallon mixed Ca²⁺/Mg²⁺. Hardness is a measure of the amount of calcium (Ca²⁺) and magnesium (Mg²⁺) in the water. Most water in the United States is hard, but the degree of hardness varies. Moderately hard (60-120 ppm) to hard (121-181 ppm) water has 60 to 181 parts per million (parts per million converted to grains per U.S. gallon is ppm # divided by 17.1 equals grains per gallon) of hardness minerals.

| **Water** | **Grains per gallon** | **Parts per million** |
|---|---|---|
| Soft | less than 1.0 | less than 17 |
| Slightly hard | 1.0 to 3.5 | 17 to 60 |
| Moderately hard | 3.5 to 7.0 | 60 to 120 |
| Hard | 7.0 to 10.5 | 120 to 180 |
| Very hard | greater than 10.5 | greater than 180 |

European water hardness is typically greater than about 10.5 (for example about 10.5 to about 20.0) grains per gallon mixed Ca2⁺/Mg²⁺ (*e.g.*, about 15 grains per gallon mixed Ca²⁺/Mg²⁺). North American water hardness is typically greater than Japanese water hardness, but less than European water hardness. For example, North American water hardness can be between about 3 to about 10 grains, about 3 to about 8 grains or about 6 grains. Japanese water hardness is typically lower than North American water hardness, usually less than about 4, for example about 3 grains per gallon mixed Ca²⁺/Mg²⁺.

In some embodiments of the present invention, the cleaning compositions comprise at least one variant protease of the present invention at a level from about 0.00001 % to about 10% by weight of the composition and the balance (*e.g*., about 99.999% to about 90.0%) comprising cleaning adjunct materials by weight of composition. In some other embodiments of the present invention, the cleaning compositions of the present invention comprises at least one variant protease at a level of about 0.0001 % to about 10%, about 0.001% to about 5%, about 0.001% to about 2%, about 0.005% to about 0.5% by weight of the composition and the balance of the cleaning composition (*e.g*., about 99.9999% to about 90.0%, about 99.999 % to about 98%, about 99.995% to about 99.5% by weight) comprising cleaning adjunct materials.

In some embodiments, the cleaning compositions of the present invention comprise one or more additional detergent enzymes, which provide cleaning performance and/or fabric care and/or dishwashing benefits. Examples of suitable enzymes include, but are not limited to, hemicellulases, cellulases, peroxidases, proteases, xylanases, lipases, phospholipases, esterases, cutinases, pectinases, pectate lyases, mannanases, keratinases, reductases, oxidases, phenoloxidases, lipoxygenases, ligninases, pullulanases, tannases, pentosanases, malanases, β-glucanases, arabinosidases, hyaluronidases, chondroitinases, laccases, and amylases, or any combinations or mixtures thereof. In some embodiments, a combination of enzymes is used (*i.e*., a "cocktail") comprising conventional applicable enzymes like protease, lipase, cutinase and/or cellulase in conjunction with amylase is used.

In addition to the protease variants provided herein, any other suitable protease finds use in the compositions of the present invention. Suitable proteases include those of animal, vegetable or microbial origin. In some embodiments, microbial proteases are used. In some embodiments, chemically or genetically modified mutants are included. In some embodiments, the protease is a serine protease, preferably an alkaline microbial protease or a trypsin-like protease. Examples of alkaline proteases include subtilisins, especially those derived from *Bacillus* (*e.g*.*,* subtilisin, *lentus, amyloliquefaciens,* subtilisin Carlsberg, subtilisin 309, subtilisin 147 and subtilisin 168). Additional examples include those mutant proteases described in U.S. Pat. Nos. RE 34,606, 5,955,340, 5,700,676, 6,312,936, and 6,482,628. Additional protease examples include, but are not limited to trypsin (*e.g*., of porcine or bovine origin), and the *Fusarium* protease described in WO 89/06270. In some embodiments, commercially available protease enzymes that find use in the present invention include, but are not limited to MAXATASE^{®}, MAXACAL^{™}, MAXAPEM^{™}, OPTICLEAN^{®}, OPTIMASE^{®}, PROPERASE^{®}, PURAFECT^{®}, PURAFECT^{®} OXP, PURAMAX^{™}, EXCELLASE^{™}, and PURAFAST^{™} (Genencor); ALCALASE^{®}, SAVINASE^{®}, PRIMASE^{®}, DURAZYM^{™}, POLARZYME^{®}, OVOZYME^{®}, KANNASE^{®}, LIQUANASE^{®}, NEUTRASE^{®}, RELASE^{®} and ESPERASE^{®} (Novozymes); BLAP^{™} and BLAP^{™} variants (Henkel Kommanditgesellschaft auf Aktien, Duesseldorf, Germany), and KAP (B. alkalophilus subtilisin; Kao Corp., Tokyo, Japan). Various proteases are described in WO95/23221, WO 92/21760, U.S. Pat. Publ. No. 2008/0090747, and U.S. Pat. Nos. 5,801,039, 5,340,735, 5,500,364, 5,855,625, US RE 34,606, 5,955,340, 5,700,676, 6,312,936, and 6,482,628, and various other patents. In some further embodiments, metalloproteases find use in the present invention, including but not limited to the neutral metalloprotease described in WO 07/044993.

In addition, any suitable lipase finds use in the present invention. Suitable lipases include, but are not limited to those of bacterial or fungal origin. Chemically or genetically modified mutants are encompassed by the present invention. Examples of useful lipases include *Humicola lanuginosa* lipase *(See e.g.,* EP 258 068, and EP 305 216), *Rhizomucor miehei* lipase *(See e.g.,* EP 238 023), *Candida* lipase, such as *C. antarctica* lipase *(e.g.,* the *C. antarctica* lipase A or B; *See e.g.,* EP 214 761), *Pseudomonas* lipases such as *P. alcaligenes* lipase and *P. pseudoalcaligenes* lipase *(See e.g.,* EP 218 272), *P. cepacia* lipase *(See e.g.,* EP 331 376), *P. stutzeri* lipase *(See e.g.,* GB 1,372,034), *P. fluorescens* lipase, *Bacillus* lipase (*e.g*., *B. subtilis* lipase [Dartois et al., Biochem. Biophys. Acta 1131:253-260 [1993]); *B. stearothermophilus* lipase [*See e.g.,* JP 64/744992]; and *B. pumilus* lipase [*See e.g.,* WO 91/16422]).

Furthermore, a number of cloned lipases find use in some embodiments of the present invention, including but not limited to *Penicillium camembertii* lipase *(See,* Yamaguchi et al., Gene 103:61-67 [1991]), *Geotricum candidum* lipase *(See,* Schimada et al., J. Biochem., 106:383-388 [1989]), and various *Rhizopus* lipases such as *R. delemar* lipase *(See,* Hass et al., Gene 109:117-113 [1991]), a R. *niveus* lipase (Kugimiya et al., Biosci. Biotech. Biochem. 56:716-719 [1992]) and *R. oryzae* lipase.

Other types of thermolysin enzymes such as cutinases also find use in some embodiments of the present invention, including but not limited to the cutinase derived from *Pseudomonas mendocina* (*See*, WO 88/09367), and the cutinase derived from *Fusarium solani pisi* (*See,* WO 90/09446).

Additional suitable lipases include commercially available lipases such as M1 LIPASE^{™}, LUMA FAST^{™}, and LIPOMAX^{™} (Genencor); LIPEX^{®}, LIPOLASE^{®} and LIPOLASE^{®} ULTRA (Novozymes); and LIPASE P^{™} "Amano" (Amano Pharmaceutical Co. Ltd., Japan).

In some embodiments invention, the cleaning compositions of the present invention further comprise lipases at a level from about 0.00001 % to about 10% of additional lipase by weight of the composition and the balance of cleaning adjunct materials by weight of composition. In some other embodiments of the present invention, the cleaning compositions of the present invention also comprise lipases at a level of about 0.0001 % to about 10%, about 0.001% to about 5%, about 0.001% to about 2%, about 0.005% to about 0.5% lipase by weight of the composition.

In some embodiments of the present invention, any suitable amylase finds use in the present invention. In some embodiments, any amylase (e.g., alpha and/or beta) suitable for use in alkaline solutions also find use. Suitable amylases include, but are not limited to those of bacterial or fungal origin. Chemically or genetically modified mutants are included in some embodiments. Amylases that find use in the present invention, include, but are not limited to α-amylases obtained from *B*. *licheniformis* (*See e.g*., GB 1,296,839). Commercially available amylases that find use in the present invention include, but are not limited to DURAMYL^{®}, TERMAMYL^{®}, FUNGAMYL^{®}, STAINZYME^{®}, STAINZYME PLUS^{®}, STAINZYME ULTRA^{®}, and BAN^{™} (Novozymes), as well as POWERASE^{™}, RAPIDASE^{®} and MAXAMYL^{®} P (Genencor).

In some embodiments of the present invention, the cleaning compositions of the present invention further comprise amylases at a level from about 0.00001 % to about 10% of additional amylase by weight of the composition and the balance of cleaning adjunct materials by weight of composition. In some other embodiments of the present invention, the cleaning compositions of the present invention also comprise amylases at a level of about 0.0001 % to about 10%, about 0.001% to about 5%, about 0.001% to about 2%, about 0.005% to about 0.5% amylase by weight of the composition.

In some further embodiments, any suitable cellulase finds used in the cleaning compositions of the present invention. Suitable cellulases include, but are not limited to those of bacterial or fungal origin. Chemically or genetically modified mutants are included in some embodiments. Suitable cellulases include, but are not limited to *Humicola insolens* cellulases (*See e.g*., U.S. Pat. No. 4,435,307). Especially suitable cellulases are the cellulases having color care benefits *(See e.g.,* EP 0 495 257). Commercially available cellulases that find use in the present include, but are not limited to CFLLUZYMF^{®}, CAREZYME^{®} (Novozymes), and KAC-500(B)^{™} (Kao Corporation). In some embodiments, cellulases are incorporated as portions or fragments of mature wild-type or variant cellulases, wherein a portion of the N-terminus is deleted (*See e.g*., U.S. Pat. No. 5,874,276). In some embodiments, the cleaning compositions of the present invention further comprise cellulases at a level from about 0.00001 % to about 10% of additional cellulase by weight of the composition and the balance of cleaning adjunct materials by weight of composition. In some other embodiments of the present invention, the cleaning compositions of the present invention also comprise cellulases at a level of about 0.0001 % to about 10%, about 0.001% to about 5%, about 0.001% to about 2%, about 0.005% to about 0.5% cellulase by weight of the composition.

Any mannanase suitable for use in detergent compositions also finds use in the present invention. Suitable mannanases include, but are not limited to those of bacterial or fungal origin. Chemically or genetically modified mutants are included in some embodiments. Various mannanases are known which find use in the present invention (*See e.g*., U.S. Pat. No. 6,566,114, U.S. Pat. No. 6,602,842, and US Patent No. 6,440,991). In some embodiments, the cleaning compositions of the present invention further comprise mannanases at a level from about 0.00001% to about 10% of additional mannanase by weight of the composition and the balance of cleaning adjunct materials by weight of composition. In some embodiments of the present invention, the cleaning compositions of the present invention also comprise mannanases at a level of about 0.0001% to about 10%, about 0.001% to about 5%, about 0.001% to about 2%, about 0.005% to about 0.5% mannanase by weight of the composition.

In some embodiments, peroxidases are used in combination with hydrogen peroxide or a source thereof (*e.g.*, a percarbonate, perborate or persulfate) in the compositions of the present invention. In some alternative embodiments, oxidases are used in combination with oxygen. Both types of enzymes are used for "solution bleaching" (*i.e.,* to prevent transfer of a textile dye from a dyed fabric to another fabric when the fabrics are washed together in a wash liquor), preferably together with an enhancing agent (*See e.g*., WO 94/12621 and WO 95/01426). Suitable peroxidases/oxidases include, but are not limited to those of plant, bacterial or fungal origin. Chemically or genetically modified mutants are included in some embodiments. In some embodiments, the cleaning compositions of the present invention further comprise peroxidase and/or oxidase enzymes at a level from about 0.00001 % to about 10% of additional peroxidase and/or oxidase by weight of the composition and the balance of cleaning adjunct materials by weight of composition. In some other embodiments of the present invention, the cleaning compositions of the present invention also comprise, peroxidase and/or oxidase enzymes at a level of about 0.0001 % to about 10%, about 0.001% to about 5%, about 0.001% to about 2%, about 0.005% to about 0.5% peroxidase and/or oxidase enzymes by weight of the composition.

In some embodiments, additional enzymes find use, including but not limited to perhydrolases (*See e.g*., WO 05/056782). In addition, in some embodiments, mixtures of the above mentioned enzymes are encompassed herein, in particular one or more additional protease, amylase, lipase, mannanase, and/or at least one cellulase. Indeed, it is contemplated that various mixtures of these enzymes will find use in the present invention. It is also contemplated that the varying levels of the variant protease(s) and one or more additional enzymes may both independently range to about 10%, the balance of the cleaning composition being cleaning adjunct materials. The specific selection of cleaning adjunct materials are readily made by considering the surface, item, or fabric to be cleaned, and the desired form of the composition for the cleaning conditions during use (*e.g*., through the wash detergent use).

Examples of suitable cleaning adjunct materials include, but are not limited to, surfactants, builders, bleaches, bleach activators, bleach catalysts, other enzymes, enzyme stabilizing systems, chelants, optical brighteners, soil release polymers, dye transfer agents, dye transfer inhibiting agents, catalytic materials, hydrogen peroxide, sources of hydrogen peroxide, preformed peracids, polymeric dispersing agents, clay soil removal agents, structure elasticizing agents, dispersants, suds suppressors, dyes, perfumes, colorants, filler salts, hydrotropes, photoactivators, fluorescers, fabric conditioners, fabric softeners, carriers, hydrotropes, processing aids, solvents, pigments, hydrolyzable surfactants, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, and pH control agents (*See e.g*., U.S. Pat. Nos. 6,610,642, 6,605,458, 5,705,464, 5,710,115, 5,698,504, 5,695,679, 5,686,014 and 5,646,101). Embodiments of specific cleaning composition materials are exemplified in detail below. In embodiments in which the cleaning adjunct materials are not compatible with the variant proteases of the present invention in the cleaning compositions, then suitable methods of keeping the cleaning adjunct materials and the protease(s) separated (*i.e.,* not in contact with each other) until combination of the two components is appropriate are used. Such separation methods include any suitable method known in the art (*e.g*., gelcaps, encapsulation, tablets, physical separation, etc.).

In some embodiments, an effective amount of one or more variant protease(s) provided herein is included in compositions useful for cleaning a variety of surfaces in need of proteinaceous stain removal. Such cleaning compositions include cleaning compositions for such applications as cleaning hard surfaces, fabrics, and dishes. Indeed, in some embodiments, the present invention provides fabric cleaning compositions, while in other embodiments, the present invention provides non-fabric cleaning compositions. Notably, the present invention also provides cleaning compositions suitable for personal care, including oral care (including dentrifices, toothpastes, mouthwashes, etc., as well as denture cleaning compositions), skin, and hair cleaning compositions. It is intended that the present invention encompass detergent compositions in any form (*i.e.*, liquid, granular, bar, semi-solid, gels, emulsions, tablets, capsules, etc.).

By way of example, several cleaning compositions wherein the variant proteases of the present invention find use are described in greater detail below. In some embodiments in which the cleaning compositions of the present invention are formulated as compositions suitable for use in laundry machine washing method(s), the compositions of the present invention preferably contain at least one surfactant and at least one builder compound, as well as one or more cleaning adjunct materials preferably selected from organic polymeric compounds, bleaching agents, additional enzymes, suds suppressors, dispersants, lime-soap dispersants, soil suspension and anti-redeposition agents and corrosion inhibitors. In some embodiments, laundry compositions also contain softening agents (*i.e.,* as additional cleaning adjunct materials). The compositions of the present invention also find use detergent additive products in solid or liquid form. Such additive products are intended to supplement and/or boost the performance of conventional detergent compositions and can be added at any stage of the cleaning process. In some embodiments, the density of the laundry detergent compositions herein ranges from about 400 to about 1200 g/liter, while in other embodiments, it ranges from about 500 to about 950 g/liter of composition measured at 20°C.

In embodiments not covered by the invention formulated as compositions for use in manual dishwashing methods, the compositions of the invention preferably contain at least one surfactant and preferably at least one additional cleaning adjunct material selected from organic polymeric compounds, suds enhancing agents, group II metal ions, solvents, hydrotropes and additional enzymes.

In some embodiments not covered by the invention, various cleaning compositions such as those provided in U.S, Pat. No. 6,605,458, find use with the variant proteases of the present invention. Thus, in some embodiments not covered by the invention, the compositions comprising at least one variant protease of the present invention is a compact granular fabric cleaning composition, while in other embodiments not covered by the invention, the composition is a granular fabric cleaning composition useful in the laundering of colored fabrics, in further embodiments not covered by the invention, the composition is a granular fabric cleaning composition which provides softening through the wash capacity, in additional embodiments not covered by the invention, the composition is a heavy duty liquid fabric cleaning composition. In some embodiments not covered by the invention, the compositions comprising at least one variant protease of the present invention are fabric cleaning compositions such as those described in U.S. Pat. Nos. 6,610,642 and 6,376,450. In addition, the variant proteases of the present invention find use in granular laundry detergent compositions of particular utility under European or Japanese washing conditions (*See e.g*., U.S. Pat. No. 6,610,642).

In some alternative embodiments, the present invention provides hard surface cleaning compositions comprising at least one variant protease provided herein. Thus, in some embodiments, the compositions comprising at least one variant protease of the present invention is a hard surface cleaning composition such as those described in U.S. Pat. Nos. 6,610,642, 6,376,450, and 6,376,450.

In yet further embodiments, the present invention provides dishwashing compositions comprising at least one variant protease provided herein. Thus, in some embodiments, the compositions comprising at least one variant protease of the present invention is a hard surface cleaning composition such as those in U.S. Pat. Nos. 6,610,642 and 6,376,450. In some still further embodiments, the present invention provides dishwashing compositions comprising at least one variant protease provided herein. In some further embodiments, the compositions comprising at least one variant protease of the present invention comprise oral care compositions such as those in U.S. Pat. No. 6,376,450, and 6,376,450. The formulations and descriptions of the compounds and cleaning adjunct materials contained in the aforementioned US Pat. Nos. 6,376,450, 6,605,458, 6,605,458, and 6,610,642, find use with the variant proteases provided herein.

The cleaning compositions of the present invention are formulated into any suitable form and prepared by any process chosen by the formulator, non-limiting examples of which are described in U.S. Pat. Nos. 5,879,584, 5,691,297, 5,574,005, 5,569,645, 5,565,422, 5,516,448, 5,489,392, and 5,486,303. When a low pH cleaning composition is desired, the pH of such composition is adjusted via the addition of a material such as monoethanolamine or an acidic material such as HCl.

While not essential for the purposes of the present invention, the non-limiting list of adjuncts illustrated hereinafter are suitable for use in the instant cleaning compositions. In some embodiments, these adjuncts are incorporated for example, to assist or enhance cleaning performance, for treatment of the substrate to be cleaned, or to modify the aesthetics of the cleaning composition as is the case with perfumes, colorants, dyes or the like. It is understood that such adjuncts are in addition to the variant proteases of the present invention. The precise nature of these additional components, and levels of incorporation thereof, will depend on the physical form of the composition and the nature of the cleaning operation for which it is to be used. Suitable adjunct materials include, but are not limited to, surfactants, builders, chelating agents, dye transfer inhibiting agents, deposition aids, dispersants, additional enzymes, and enzyme stabilizers, catalytic materials, bleach activators, bleach boosters, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids and/or pigments. In addition to the disclosure below, suitable examples of such other adjuncts and levels of use are found in U.S. Patent Nos. 5,576,282, 6,306,812, and 6,326,348. The aforementioned adjunct ingredients may constitute the balance of the cleaning compositions of the present invention.

In some embodiments, the cleaning compositions according to the present invention comprise at least one surfactant and/or a surfactant system wherein the surfactant is selected from nonionic surfactants, anionic surfactants, cationic surfactants, ampholytic surfactants, zwitterionic surfactants, semi-polar nonionic surfactants and mixtures thereof. In some low pH cleaning composition embodiments (*e.g*., compositions having a neat pH of from about 3 to about 5), the composition typically does not contain alkyl ethoxylated sulfate, as it is believed that such surfactant may be hydrolyzed by such compositions the acidic contents. In some embodiments, the surfactant is present at a level of from about 0.1% to about 60%, while in alternative embodiments the level is from about 1% to about 50%, while in still further embodiments the level is from about 5% to about 40%, by weight of the cleaning composition.

In some embodiments, the cleaning compositions of the present invention comprise one or more detergent builders or builder systems. In some embodiments incorporating at least one builder, the cleaning compositions comprise at least about 1%, from about 3% to about 60% or even from about 5% to about 40% builder by weight of the cleaning composition. Builders include, but are not limited to, the alkali metal, ammonium and alkanolammonium salts of polyphosphates, alkali metal silicates, alkaline earth and alkali metal carbonates, aluminosilicates, polycarboxylate compounds, ether hydroxypolycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, citric acid, oxydisuccinic acid, polymaleic acid, benzene 1,3,5-tricarboxylic acid, carboxymethyloxysuccinic acid, and soluble salts thereof. Indeed, it is contemplated that any suitable builder will find use in various embodiments of the present invention.

In some embodiments, the builders form water-soluble hardness ion complexes (e.g., sequestering builders), such as citrates and polyphosphates (*e.g*., sodium tripolyphosphate and sodium tripolyphospate hexahydrate, potassium tripolyphosphate, and mixed sodium and potassium tripolyphosphate, etc.). It is contemplated that any suitable builder will find use in the present invention, including those known in the art (*See e.g*., EP 2 100 949).

In some embodiments, the cleaning compositions of the present invention contain at least one chelating agent. Suitable chelating agents include, but are not limited to copper, iron and/or manganese chelating agents and mixtures thereof. In embodiments in which at least one chelating agent is used, the cleaning compositions of the present invention comprise from about 0.1% to about 15% or even from about 3.0% to about 10% chelating agent by weight of the subject cleaning composition.

In some still further embodiments, the cleaning compositions provided herein contain at least one deposition aid. Suitable deposition aids include, but are not limited to, polyethylene glycol, polypropylene glycol, polycarboxylate, soil release polymers such as polytelephthalic acid, clays such as kaolinite, montmorillonite, atapulgite, illite, bentonite, halloysite, and mixtures thereof.

As indicated herein, in some embodiments, anti-redeposition agents find use in some embodiments of the present invention. In some embodiments, non-ionic surfactants find use. For example, in automatic dishwashing embodiments, non-ionic surfactants find use for surface modification purposes, in particular for sheeting, to avoid filming and spotting and to improve shine. These non-ionic surfactants also find use in preventing the re-deposition of soils. In some embodiments, the anti-redeposition agent is a non-ionic surfactant as known in the art (*See e.g*., EP 2 100 949).

In some embodiments, the cleaning compositions of the present invention include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. In embodiments in which at least one dye transfer inhibiting agent is used, the cleaning compositions of the present invention comprise from about 0.0001% to about 10%, from about 0.01% to about 5%, or even from about 0.1% to about 3% by weight of the cleaning composition.

In some embodiments, silicates are included within the compositions of the present invention. In some such embodiments, sodium silicates (*e.g*., sodium disilicate, sodium metasilicate, and crystalline phyllosilicates) find use. In some embodiments, silicates are present at a level of from about 1% to about 20%. In some embodiments, silicates are present at a level of from about 5% to about 15% by weight of the composition.

In some still additional embodiments, the cleaning compositions of the present invention also contain dispersants. Suitable water-soluble organic materials include, but are not limited to the homo- or co-polymeric acids or their salts, in which the polycarboxylic acid comprises at least two carboxyl radicals separated from each other by not more than two carbon atoms.

In some further embodiments not covered by the invention, the enzymes used in the cleaning compositions are stabilized by any suitable technique. In some embodiments not covered by the invention, the enzymes employed herein are stabilized by the presence of water-soluble sources of calcium and/or magnesium ions in the finished compositions that provide such ions to the enzymes. In some embodiments not covered by the invention, the enzyme stabilizers include oligosaccharides, polysaccharides, and inorganic divalent metal salts, including alkaline earth metals, such as calcium salts. It is contemplated that various techniques for enzyme stabilization will find use in the present invention. Examples of suitable oligosaccharides and polysaccharides (*e.g*., dextrins) are known in the art (*See e.g*., WO 07/145964).

In some embodiments, bleaches, bleach activators and/or bleach catalysts are present in the compositions of the present invention. In some embodiments, the cleaning compositions of the present invention comprise inorganic and/or organic bleaching compound(s). Inorganic bleaches include, but are not limited to perhydrate salts (*e.g*., perborate, percarbonate, perphosphate, persulfate, and persilicate salts). In some embodiments, inorganic perhydrate salts are alkali metal salts. In some embodiments, inorganic perhydrate salts are included as the crystalline solid, without additional protection, although in some other embodiments, the salt is coated. Any suitable salt known in the art finds use in the present invention (*See e.g*., EP 2 100 949).

In some embodiments, bleach activators are used in the compositions of the present invention. Bleach activators are typically organic peracid precursors that enhance the bleaching action in the course of cleaning at temperatures of 60°C and below. Bleach activators suitable for use herein include compounds which, under perhydrolysis conditions, give aliphatic peroxoycarboxylic acids having preferably from about 1 to about 10 carbon atoms, in particular from about 2 to about 4 carbon atoms, and/or optionally substituted perbenzoic acid. Additional bleach activators are known in the art and find use in the present invention (*See e.g*., EP 2 100 949).

In addition, in some embodiments and as further described herein, the cleaning compositions of the present invention further comprise at least one bleach catalyst. In some embodiments, the manganese triazacyclononane and related complexes find use, as well as cobalt, copper, manganese, and iron complexes. Additional bleach catalysts find use in the present invention (*See e.g*., US 4,246,612, 5,227,084, 4,810410, WO 99/06521, and EP 2 100 949).

In some embodiments, the cleaning compositions of the present invention contain one or more catalytic metal complexes. In some embodiments, a metal-containing bleach catalyst finds use. In some embodiments, the metal bleach catalyst comprises a catalyst system comprising a transition metal cation of defined bleach catalytic activity, (*e.g*., copper, iron, titanium, ruthenium, tungsten, molybdenum, or manganese cations), an auxiliary metal cation having little or no bleach catalytic activity (*e.g*., zinc or aluminum cations), and a sequestrate having defined stability constants for the catalytic and auxiliary metal cations, particularly ethylenediaminetetraacetic acid, ethylenediaminetetra (methylenephosphonic acid) and water-soluble salts thereof are used (*See e.g*., US Patent No. 4,430,243). In some embodiments, the cleaning compositions of the present invention are catalyzed by means of a manganese compound. Such compounds and levels of use are well known in the art (*See e.g*., US Patent No. 5,576,282). In additional embodiments, cobalt bleach catalysts find use in the cleaning compositions of the present invention. Various cobalt bleach catalysts are known in the art (*See e.g*., US Patent Nos. 5,597,936 and 5,595,967) and are readily prepared by known procedures.

In some additional embodiments, the cleaning compositions of the present invention include a transition metal complex of a macropolycyclic rigid ligand (MRL). As a practical matter, and not by way of limitation, in some embodiments, the compositions and cleaning processes provided by the present invention are adjusted to provide on the order of at least one part per hundred million of the active MRL species in the aqueous washing medium, and in some embodiments, provide from about 0.005 ppm to about 25 ppm, more preferably from about 0.05 ppm to about 10 ppm, and most preferably from about 0.1 ppm to about 5 ppm, of the MRL in the wash liquor.

In some embodiments, transition-metals in the instant transition-metal bleach catalyst include, but are not limited to manganese, iron and chromium. MRLs also include, but are not limited to special ultra-rigid ligands that are cross-bridged (*e.g*., 5,12-diethyl-1,5,8,12-tetraazabicyclo[6.6.2]hexadecane). Suitable transition metal MRLs are readily prepared by known procedures (*See e.g*., WO 2000/32601, and US Patent No. 6,225,464).

In some embodiments, the cleaning compositions of the present invention comprise metal care agents. Metal care agents find use in preventing and/or reducing the tarnishing, corrosion, and/or oxidation of metals, including aluminum, stainless steel, and non-ferrous metals (e.g., silver and copper). Suitable metal care agents include those described in EP 2 100 949, WO 9426860 and WO 94/26859). In some embodiments, the metal care agent is a zinc salt. In some further embodiments, the cleaning compositions of the present invention comprise from about 0.1% to about 5% by weight of one or more metal care agent.

In some embodiments, the cleaning composition is a high density liquid (HDL) composition having a variant thermolysin protease. The HDL liquid laundry detergent can comprise a detersive surfactant (10%-40%) comprising anionic detersive surfactant (selected from a group of linear or branched or random chain, substituted or unsubstituted alkyl sulphates, alkyl sulphonates, alkyl alkoxylated sulphate, alkyl phosphates, alkyl phosphonates, alkyl carboxylates, and/or mixtures thereof); and optionally non-ionic surfactant (selected from a group of linear or branched or random chain, substituted or unsubstituted alkyl alkoxylated alcohol, for example a C₈-C₁₈ alkyl ethoxylated alcohol and/or C₆-C₁₂ alkyl phenol alkoxylates), optionally wherein the weight ratio of anionic detersive surfactant (with a hydrophilic index (HIc) of from 6.0 to 9) to non-ionic detersive surfactant is greater than 1: 1.

The composition can comprise optionally, a surfactancy boosting polymer consisting of amphiphilic alkoxylated grease cleaning polymers (selected from a group of alkoxylated polymers having branched hydrophilic and hydrophobic properties, such as alkoxylated polyalkylenimines in the range of 0.05wt%-10wt%) and/or random graft polymers (typically comprising of hydrophilic backbone comprising monomers selected from the group consisting of: unsaturated C₁-C₆ carboxylic acids, ethers, alcohols, aldehydes, ketones, esters, sugar units, alkoxy units, maleic anhydride, saturated polyalcohols such as glycerol, and mixtures thereof; and hydrophobic side chain(s) selected from the group consisting of: C₄-C₂₅ alkyl group, polypropylene, polybutylene, vinyl ester of a saturated C₁-C₆ mono-carboxylic acid, C₁-C₆ alkyl ester of acrylic or methacrylic acid, and mixtures thereof.

The composition can comprise additional polymers such as soil release polymers (include anionically end-capped polyesters, for example SRP1, polymers comprising at least one monomer unit selected from saccharide, dicarboxylic acid, polyol and combinations thereof, in random or block configuration, ethylene terephthalate-based polymers and co-polymers thereof in random or block configuration, for example Repel-o-tex SF, SF-2 and SRP6, Texcare SRA100, SRA300, SRN100, SRN170, SRN240, SRN300 and SRN325, Marloquest SL), anti-redeposition polymers (0.1 wt% to 10wt%, include carboxylate polymers, such as polymers comprising at least one monomer selected from acrylic acid, maleic acid (or maleic anhydride), fumaric acid, itaconic acid, aconitic acid, mesaconic acid, citraconic acid, methylenemalonic acid, and any mixture thereof, vinylpyrrolidone homopolymer, and/or polyethylene glycol, molecular weight in the range of from 500 to 100,000 Da); cellulosic polymer (including those selected from alkyl cellulose, alkyl alkoxyalkyl cellulose, carboxyalkyl cellulose, alkyl carboxyalkyl cellulose examples of which include carboxymethyl cellulose, methyl cellulose, methyl hydroxyethyl cellulose, methyl carboxymethyl cellulose, and mixures thereof) and polymeric carboxylate (such as maleate/acrylate random copolymer or polyacrylate homopolymer).

The composition can further comprise saturated or unsaturated fatty acid, preferably saturated or unsaturated C₁₂-C₂₄ fatty acid (0 wt% to 10 wt%); deposition aids (examples for which include polysaccharides, preferably cellulosic polymers, poly diallyl dimethyl ammonium halides (DADMAC), and co-polymers of DAD MAC with vinyl pyrrolidone, acrylamides, imidazoles, imidazolinium halides, and mixtures thereof, in random or block configuration, cationic guar gum, cationic cellulose such as cationic hydoxyethyl cellulose, cationic starch, cationic polyacylamides, and mixtures thereof.

The composition can further comprise dye transfer inhibiting agents examples of which include manganese phthalocyanine, peroxidases, polyvinylpyrrolidone polymers, polyamine N-oxide polymers, copolymers of N-vinylpyrrolidone and N-vinylimidazole, polyvinyloxazolidones and polyvinylimidazoles and/or mixtures thereof; chelating agents examples of which include ethylenediamine-tetraacetic acid (EDTA); diethylene triamine penta methylene phosphonic acid (DTPMP); hydroxy-ethane diphosphonic acid (HEDP); ethylenediamine N,N'-disuccinic acid (EDDS); methyl glycine diacetic acid (MGDA); diethylene triamine penta acetic acid (DTPA); propylene diamine tetracetic acid (PDT A); 2-hydroxypyridine-N-oxide (HPNO); or methyl glycine diacetic acid (MGDA); glutamic acid N,N-diacetic acid (N,N-dicarboxymethyl glutamic acid tetrasodium salt (GLDA); nitrilotriacetic acid (NTA); 4,5-dihydroxy-m-benzenedisulfonic acid; citric acid and any salts thereof; N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP) and derivatives thereof.

The composition can further comprise enzymes (0.01 wt% active enzyme to 0.03wt% active enzyme) selected from a group of proteases; amylases; lipases; cellulases; choline oxidases; peroxidases/oxidases; pectate lyases; mannanases; cutinases; laccases; phospholipases; lysophospholipases; acyltransferase; perhydrolase; arylesterase and any mixture thereof. The composition may comprise an enzyme stabilizer (examples of which include polyols such as propylene glycol or glycerol, sugar or sugar alcohol, lactic acid, reversible protease inhibitor, boric acid, or a boric acid derivative, e.g., an aromatic borate ester, or a phenyl boronic acid derivative such as 4-formylphenyl boronic acid).

The composition can further comprise silicone or fatty-acid based suds suppressors; hueing dyes, calcium and magnesium cations, visual signaling ingredients, anti-foam (0.001 wt% to about 4.0 wt%), and/or structurant/thickener (0.01 wt% to 5wt%, selected from the group consisting of diglycerides and triglycerides, ethylene glycol distearate, microcrystalline cellulose, cellulose based materials, microfiber cellulose, biopolymers, xanthan gum, gellan gum, and mixtures thereof).

Suitable detersive surfactants also include cationic detersive surfactants (selected from a group of alkyl pyridinium compounds, alkyl quarternary ammonium compounds, alkyl quarternary phosphonium compounds, alkyl ternary sulphonium compounds, and/or mixtures thereof); zwitterionic and/or amphoteric detersive surfactants (selected from a group of alkanolamine sulpho-betaines); ampholytic surfactants; semi-polar non-ionic surfactants and mixtures thereof.

The composition can be any liquid form, for example a liquid or gel form, or any combination thereof. The composition may be in any unit dose form, for example a pouch.

In some embodiments not covered by the invention, the cleaning composition is a high density powder (HDD) composition having a variant thermolysin protease. The HDD powder laundry detergent can comprise a detersive surfactant including anionic detersive surfactants (selected from a group of linear or branched or random chain, substituted or unsubstituted alkyl sulphates, alkyl sulphonates, alkyl alkoxylated sulphate, alkyl phosphates, alkyl phosphonates, alkyl carboxylates and/or mixtures thereof), non-ionic detersive surfactant (selected from a group of linear or branched or random chain, substituted or unsubstituted C₈-C₁₈ alkyl ethoxylates, and/or C₆-C₁₂ alkyl phenol alkoxylates), cationic detersive surfactants (selected from a group of alkyl pyridinium compounds, alkyl quaternary ammonium compounds, alkyl quaternary phosphonium compounds, alkyl ternary sulphonium compounds, and mixtures thereof), zwitterionic and/or amphoteric detersive surfactants (selected from a group of alkanolamine sulpho-betaines); ampholytic surfactants; semi-polar non-ionic surfactants and mixtures thereof; builders (phosphate free builders [for example zeolite builders examples of which include zeolite A, zeolite X, zeolite P and zeolite MAP in the range of 0 wt% to less than 10 wt%]; phosphate builders [examples of which include sodium tri-polyphosphate in the range of 0 wt% to less than 10 wt%]; citric acid, citrate salts and nitrilotriacetic acid or salt thereof in the range of less than 15 wt%); silicate salt (sodium or potassium silicate or sodium meta-silicate in the range of 0wt% to less than 10 wt%, or layered silicate (SKS-6)); carbonate salt (sodium carbonate and/or sodium bicarbonate in the range of 0 wt% to less than 10 wt%); and bleaching agents (photobleaches, examples of which include sulfonated zinc phthalocyanines, sulfonated aluminum phthalocyanines, xanthenes dyes, and mixtures thereof; hydrophobic or hydrophilic bleach activators (examples of which include dodecanoyl oxybenzene sulfonate, decanoyl oxybenzene sulfonate, decanoyl oxybenzoic acid or salts thereof, 3,5,5-trimethy hexanoyl oxybenzene sulfonate, tetraacetyl ethylene diamine-TAED, and nonanoyloxybenzene sulfonate-NOBS, nitrile quats, and mixtures thereof; hydrogen peroxide; sources of hydrogen peroxide (inorganic perhydrate salts examples of which include mono or tetra hydrate sodium salt of perborate, percarbonate, persulfate, perphosphate, or persilicate); preformed hydrophilic and/or hydrophobic peracids (selected from a group consisting of percarboxylic acids and salts, percarbonic acids and salts, perimidic acids and salts, peroxymonosulfuric acids and salts) & mixtures thereof and/or bleach catalyst (such as imine bleach boosters examples of which include iminium cations and polyions; iminium zwitterions; modified amines; modified amine oxides; N-sulphonyl imines; N-phosphonyl imines; N-acyl imines; thiadiazole dioxides; perfluoroimines; cyclic sugar ketones and mixtures thereof; metal-containing bleach catalyst for example copper, iron, titanium, ruthenium, tungsten, molybdenum, or manganese cations along with an auxiliary metal cations such as zinc or aluminum and a sequestrate such as ethylenediaminetetraacetic acid, ethylenediaminetetra(methylenephosphonic acid) and water-soluble salts thereof).

The composition can further comprise enzymes selected from a group of proteases; amylases; lipases; cellulases; choline oxidases; peroxidases/oxidases; pectate lyases; mannanases; cutinases; laccases; phospholipases; lysophospholipases; acyltransferase; perhydrolase; arylesterase and any mixture thereof.

The composition can further comprise additional detergent ingredients including perfume microcapsules, starch encapsulated perfume accord, hueing agents, additional polymers including fabric integrity and cationic polymers, dye lock ingredients, fabric-softening agents, brighteners (for example C.I. Fluorescent brighteners), flocculating agents, chelating agents, alkoxylated polyamines, fabric deposition aids, and/or cyclodextrin.

In some embodiments not covered by the invention, the cleaning composition is an automatic dishwashing (ADW) detergent composition having a variant thermolysin protease. The ADW detergent can comprise two or more non-ionic surfactants selected from a group of ethoxylated non-ionic surfactants, alcohol alkoxylated surfactants, epoxy-capped poly(oxyalkylated) alcohols, or amine oxide surfactants present in amounts from 0 to 10% by weight; builders in the range of 5-60% comprising either phosphate (mono-phosphates, di-phosphates, tri-polyphosphates or oligomeric-poylphosphates, preferred sodium tripolyphosphate-STPP or phosphate-free builders [amino acid based compounds, examples of which include MGDA (methyl-glycine-diacetic acid), and salts and derivatives thereof, GLDA (glutamic-N,Ndiacetic acid) and salts and derivatives thereof, IDS (iminodisuccinic acid) and salts and derivatives thereof, carboxy methyl inulin and salts and derivatives thereof and mixtures thereof, nitrilotriacetic acid (NTA), diethylene triamine penta acetic acid (DTPA), B-alaninediacetic acid (B-ADA) and their salts], homopolymers and copolymers of poly-carboxylic acids and their partially or completely neutralized salts, monomeric polycarboxylic acids and hydroxycarboxylic acids and their salts in the range of 0.5% to 50% by weight; sulfonated/carboxylated polymers (provide dimensional stability to the product) in the range of about 0.1 % to about 50% by weight; drying aids in the range of about 0.1 % to about 10% by weight (selected from polyesters, especially anionic polyesters optionally together with further monomers with 3 to 6 functionalities which are conducive to polycondensation, specifically acid, alcohol or ester functionalities, polycarbonate-, polyurethane- and/or polyurea-polyorganosiloxane compounds or precursor compounds thereof of the reactive cyclic carbonate and urea type); silicates in the range from about 1% to about 20% by weight (sodium or potassium silicates for example sodium disilicate, sodium meta-silicate and crystalline phyllosilicates); bleach-inorganic (for example perhydrate salts such as perborate, percarbonate, perphosphate, persulfate and persilicate salts) and organic (for example organic peroxyacids including diacyl and tetraacylperoxides, especially diperoxydodecanedioc acid, diperoxytetradecanedioc acid, and diperoxyhexadecanedioc acid); bleach activators- organic peracid precursors in the range from about 0.1% to about 10% by weight; bleach catalysts (selected from manganese triazacyclononane and related complexes, Co, Cu, Mn and Fe bispyridylamine and related complexes, and pentamine acetate cobalt(III) and related complexes); metal care agents in the range from about 0.1% to 5% by weight (selected from benzatriazoles, metal salts and complexes, and/or silicates); enzymes in the range from about 0.01 to 5.0mg of active enzyme per gram of automatic dishwashing detergent composition (selected from a group of proteases; amylases; lipases; cellulases; choline oxidases; peroxidases/oxidases; pectate lyases; mannanases; cutinases; laccases; phospholipases; lysophospholipases; acyltransferase; perhydrolase; arylesterase and any mixture thereof); and enzyme stabilizer components (selected from oligosaccharides, polysaccharides and inorganic divalent metal salts).

The tables below show representative detergent composition useful as compositions having a variant thermolysin variant of the present invention.

| **HDL Detergent Composition** | |
|---|---|
| **Ingredient** | **wt%** |
| Enzyme (s) (Protease + Lipase + Amylase) | 3 |
| Linear alkyl benzene sulphonic acid (HLAS) | 10 |
| C12-14 alkyl ethoxylated alcohol having an average degree of ethoxylation of 9 (AE9) | 2 |
| C12-14 alkyl ethoxylated sulphonic acid having an average degree of ethoxylation of 3 (HAES) | 23 |
| C16-17 alkyl mid chain branched alkyl sulphate | 4 |
| Amine oxide | 1 |
| C12-18 fatty acid | 2 |
| PE20 polymer | 3 |
| Polyethylene imine polymer | 3 |
| Chelant | 1.4 |
| FW A 15 Brightener | 0.4 |
| p-glycol (solvent) | 8 |
| DEG (solvent) | 0.5 |
| Ethanol | 3 |
| Monoethanolamine | 6 |
| Water | 26 |
| NaOH | 0.3 |
| Perfume | 1 |
| Silicone suds suppressor | 0.06 |
| Violet DD dye | 0.01 |
| Other dyes | 0.03 |
| Hydrogenated castor oil (structurant/thickener) | 0.1 |
| Mica | 0.2 |
| Calcium formate | 0.1 |
| Sodium formate | 0.2 |
| Miscellaneous | to 100 |

| **HDD Detergent Compositions** | | | | |
|---|---|---|---|---|
| **Ingredient** | **Composition A** | **Composition B** | **Composition C** | **Composition D** |
| Enzyme (Lipase + other enzymes) | 0.8 wt% | 0.8 wt% | 0.8 wt% | 0.8 wt% |
| Linear alkyl benzene sulphonate | 9 wt% | 9 wt% | 12 wt% | 8 wt% |
| Alkyl ethoxylated sulphate having an average degree of ethoxylation of from 0.5 to 3 | 3 wt% | 2 wt% | 1 wt% | 2 wt% |
| Cationic detersive surfactant | 0.5 wt% | 0.5 wt% | 0.5 wt% | 0.5 wt% |
| Sodium sulphate | 55 wt% | 55 wt% | 55 wt% | 55 wt% |
| Sodium carbonate | 8 wt% | 10 wt% | 5 wt% | 8 wt% |
| Glycerol carbonate | 9 wt% | 12 wt% | 8 wt% | 10 wt% |
| Oxaziridiniuym-based bleach catalyst | 0.005wt% | 0.005wt% | 0.005wt% | 0.005wt% |
| Sodium silicate | 3 wt% | 0 wt% | 3 wt% | 0 wt% |
| Carboxylate polymer | 2wt% | 2wt% | 2wt% | 2wt% |
| Brightener | 0.02 wt% | 0.02 wt% | 0.02 wt% | 0.02 wt% |
| Cellulosic polymer | 0.3 wt% | 0.3 wt% | 0.3 wt% | 0.3 wt% |
| Misc & Moisture | to 100 wt% | to 100 wt% | to 100 wt% | to 100 wt% |

| **HDD Detergent Compositions** | | | | | | |
|---|---|---|---|---|---|---|
| **Ingredient** | **1 (wt%)** | **2 (wt%)** | **3 (wt%)** | **4 (wt%)** | **5 (wt%)** | **6 (wt%)** |
| Sodium linear alkylbenzene sulfonate with average aliphatic chain length C11-12 | 10.3 | 10.7 | 14 | 17 | 12.2 | 8.3 |
| Sodium lauryl sulfate | 0 | 3.5 | 0 | 1.4 | 1.2 | 0 |
| Sodium C12-14 alcohol ethoxy-3-sulfate | 0 | 0 | 0.8 | 0 | 0 | 3 |
| C13-15 oxo alcohol ethoxylate with average 7 moles of ethoxylation (Lutensol^{®} A07) | 1.57 | 0 | 0 | 0 | 1.2 | 0 |
| C10-Guerbet (2-propylheptan-I-ol) alcohol ethoxylate with average 7 moles of ethoxylation (Lutensol^{®} XP70) | 0 | 1.5 | 0 | 0 | 1.2 | 0 |
| C16-18 alcohol ethoxylate with average 7 moles of ethoxylation | 0 | 0.5 | 0 | 0 | 0.3 | 0 |
| C12-18 alcohol ethoxylate with average 5 moles of ethoxylation | 0 | 0.3 | 0 | 0 | 0 | 0 |
| C12-14 alkyl hydroxyethyl dimethyl ammonium chloride (Praepagen^{®} HY) | 0 | 0 | 0.7 | 0.54 | 0.1 | 1 |
| Sodium tripolyphosphate | 0 | 0 | 0.6 | 0 | 1 | 0 |
| Zeolite A (builder) | 2.7 | 3.4 | 0 | 0 | 0.5 | 1.6 |
| Citric Acid | 1.8 | 2 | 0 | 1.4 | 0 | 2 |
| Sodium citrate | 0 | 1.9 | 0 | 0 | 0 | 0 |
| Sodium bicarbonate | 29 | 35 | 36.7 | 34 | 53 | 22 |
| Sodium sesquicarbonate dihydrate | 0 | 0 | 1.2 | 0 | 0 | 0 |
| Sodium carbonate | 1.2 | 0 | 1.9 | 0 | 0 | 0 |
| Sodium polyacrylate (MW 4000, Sokalan PA25 CL) | 0 | 0 | 1 | 0 | 0 | 0 |
| Sodium polyacrylate (MW 8000, Sokalan PA30 CL) | 1.45 | 1.6 | 0 | 0.97 | 1 | 0 |
| Sodium polyacrylate/maleate copolymer MW 70,000, 70:30 ratio, Sokalan^{®} CPS | 0 | 0 | 0.3 | 0 | 0 | 3 |
| Polyethylene glycol/vinyl acetate random graft copolymer | 0 | 0 | 0.8 | 1 | 1 | 0 |
| Carboxymethyl cellulose (Finnfix^{®} GDA) | 1 | 0.9 | 0 | 0 | 0 | 0 |
| Carboxymethyl cellulose (Finnfix^{®} V) | 0 | 0 | 0 | 0.3 | 1.1 | 0.92 |
| Hydrophobically modified carboxymethyl cellulose (Finnfix^{®} SH-1) | 0 | 0 | 0.5 | 0 | 0 | 0 |
| C. I. Fluorescent Brightener 260 | 0.1 | 0.13 | 0.1 | 0.03 | 0.05 | 0.18 |
| C. I. Fluorescent Brightener 351 (Tinopal^{®} CBS) | 0 | 0.06 | 0.08 | 0 | 0 | 0 |
| Diethylenetriamine pentaacetic acid | 0 | 0 | 0.2 | 0.1 | 0.2 | 0 |
| Tetrasodium S,S-ethylenediamine disuccinate | 0 | 0 | 0 | 0.3 | 0 | 0.3 |
| Diethylenetriamine penta (methylene phosphonic acid), heptasodium salt | 0 | 0.2 | 0 | 0 | 0 | 0 |
| 1-Hydroxyethane -1,1-diphosphonic acid | 0.1 | 0.2 | 0.3 | 0 | 0.2 | 0.4 |
| 2-Phosphonobutane 1,2,4- tricarboxylic acid (Bayhibit^{®} AM) | 0 | 0 | 0 | 0.4 | 0 | 0 |
| MgS04 | 0 | 0 | 0 | 0.8 | 0 | 0.4 |
| Sodium percarbonate | 9 | 12 | 7 | 6 | 8 | 9 |
| Propylene glycol diacetate | 7 | 10 | 10.8 | 0 | 0 | 0 |
| Triethylene glycol diacetate | 0 | 0 | 0 | 5 | 7 | 3.9 |
| Oxaziridinium-based bleach booster | 0.03 | 0 | 0.03 | 0.02 | 0.05 | 0.02 |
| Protease 1 | 4.3 | 3.3 | 6.3 | 5.7 | 3.3 | 0 |
| Protease 2 | 0 | 0 | 0 | 0 | 0 | 2.2 |
| Amyalse | 2.2 | 1.51 | 1 | 2.2 | 1.9 | 3.3 |
| Lipase | 0 | 0 | 3.6 | 0 | 0 | 2.7 |
| Endoglucanase 1 | 0 | 0 | 5.3 | 3.3 | 0 | 0 |
| Endoglucanase 2 | 2.1 | 1.3 | 0 | 0 | 0 | 2.4 |
| Mannanase | 1.3 | 1.54 | 1.3 | 0 | 1.2 | 1.9 |
| Perhydrolase 1 | 2 | 0 | 1.8 | 0 | 2.1 | 1.9 |
| Perhydrolase 2 | 0 | 4.1 | 0 | 2.3 | 0 | 0 |
| Direct Violet 9 | 0 | 0 | 0.0003 | 0.0004 | 0 | 0 |
| Solvent Violet 13 | 0 | 0 | 0.002 | 0 | 0 | 0 |
| Texcare^{®} SRA300F | 0.3 | 1.2 | 0 | 1 | 0.33 | 0.3 |
| Dye lock | 0.02 | 0.02 | 0 | 0 | 0 | 0 |
| (Tinolux^{®} BMC) | 0 | 0 | 0 | 0 | 0 | 0.0015 |
| C.I. Food Red 14 | 0 | 0 | 0.001 | 0 | 0 | 0.001 |
| Suds suppressor granule | 0.2 | 0.2 | 0 | 0 | 0.3 | 0 |
| Moisture | 7 | 6.3 | 8.9 | 9.1 | 4.3 | 4.6 |
| Perfume | 0.2 | 0.3 | 0.4 | 0.3 | 0.2 | 0.3 |
| Sodium sulfate | Balance to 100% | Balance to 100% | Balance to 100% | Balance to 100% | Balance to 100% | Balance to 100% |

| **Automatic Dishwashing (ADW) Detergent Compositions** | | | | |
|---|---|---|---|---|
| **Formulation** | **1** | **2** | **3** | **4** |
| **Ingredient** | **Level %wt** | **Level %wt** | **Level %wt** | **Level %wt** |
| Solid ADW detergent composition | | | | |
| STPP | 35 | 0 | 0 | 56 |
| Carbonate | 24 | 45 | 40 | 18.5 |
| Methylglycine diacetic acid (83% active) | 0 | 15 | 20 | 0 |
| Silicate | 7 | 7 | 7 | 1.5 |
| TEAD (Tetraacety lethy lenediamine) | 0.5 | 0.5 | 0.5 | 3.8 |
| Zinc carbonate | 0.5 | 0.5 | 0.5 | 0 |
| SLF18 | 1.5 | 1.5 | 1.5 | 0 |
| Plurafac LF224 | | | | 0.6 |
| Penta Amine Acetato-cobalt(III) nitrate (1 % active) | 0.5 | 0.5 | 0.5 | 0.6 |
| Percarbonate | 15 | 15 | 15 | 11 |
| Sulphonated polymer | 10 | 4 | 3 | 5.1 |
| Amylase (14.4mg/g active) | 1.3 | 1.8 | 1.5 | 0.7 |
| Processing aids, perfume and sodium sulphate | To balance | To balance | To balance | To balance |
| Liquid automatic dishwashing detergent com 12osition | | | | |
| Dipropylene glycol | 45 | 45 | 45 | 25 |
| SLF18 | 45 | 45 | 45 | 0 |
| Neodol1-9 | 3 | 3 | 3 | 2.6 |
| Lutensol T07 | | | | 30 |
| Plurafac LF224 | | | | 32.4 |
| Amine Oxide | | | | 3.6 |
| Glycerine | 2 | 2 | 2 | 4 |
| Processing aids and Dyes | To balance | To balance | To balance | To balance |
| Second Liquid automatic dishwashing detergent composition (part of three compartment unit dose) | | | | |

| **HDL Detergent Compositions** | | | | | |
|---|---|---|---|---|---|
| **Compound** | **Formulations** | | | | |
| | **I** | **II** | **III** | **IV** | **V** |
| LAS | 24 | 32 | 6 | 3 | 6 |
| NaC₁₆-C₁₇ HSAS | - | - | - | 5 | - |
| C₁₂-C₁₅AE₁.₈S | - | - | 8 | 7 | 5 |
| C₈-C₁₀ propyl dimethyl amine | 2 | 2 | 2 | 2 | 1 |
| C₁₂-C₁₄ alkyl dimethyl amine oxide | - | - | - | - | 2 |
| C₁₂-C₁₅ AS alkyl sulphate | - | - | 17 | - | 8 |
| C12-C14 alkyl N-methyl glucamide (CFAA) surfactant | - | 5 | 4 | 4 | 3 |
| C₁₂-C₁₄ Fatty alcohol ethoxylate | 12 | 6 | 1 | 1 | 1 |
| C₁₂-C₁₈ Fatty acid | 3 | - | 4 | 2 | 3 |
| Citric acid (anhydrous) | 4.5 | 5 | 3 | 2 | 1 |
| DETPMP | - | - | 1 | 1 | 0.5 |
| Monoethanolamine | 5 | 5 | 5 | 5 | 2 |
| Sodium hydroxide | - | - | 2.5 | 1 | 1.5 |
| 1 N HCl aqueous solution | #1 | #1 | - | - | - |
| Propanediol | 12.7 | 14.5 | 13.1 | 10 | 8 |
| Ethanol | 1.8 | 2.4 | 4.7 | 5.4 | 1 |
| DTPA | 0.5 | 0.4 | 0.3 | 0.4 | 0.5 |
| Pectin Lyase | - | - | - | 0.005 | - |
| Amylase | 0.001 | 0.002 | - | - | - |
| Cellulase | - | - | 0.0002 | - | 0.0001 |
| Lipase | 0.1 | - | 0.1 | - | 0.1 |
| Metalloprotease 1 (optional) | 0.05 | 0.3 | - | 0.5 | 0.2 |
| Metalloprotease 2 | - | - | 0.08 | - | - |
| Protease A (optional) | - | - | - | - | 0.1 |
| Aldose Oxidase | - | - | 0.3 | - | 0.003 |
| ZnCl2 | 0.1 | 0.05 | 0.05 | 0.05 | 0.02 |
| Ca formate | 0.05 | 0.07 | 0.05 | 0.06 | 0.07 |
| DETBCHD | - | - | 0.02 | 0.01 | - |
| SRP1 (anionically end capped polyesters) | 0.5 | 0.5 | - | 0.3 | 0.3 |
| Boric acid | - | - | - | - | 2.4 |
| Sodium xylene sulfonate | - | - | 3 | - | - |
| Sodium cumene sulfonate | - | - | - | 0.3 | 0.5 |
| DC 3225C | 1 | 1 | 1 | 1 | 1 |
| 2-butyl-octanol | 0.03 | 0.04 | 0.04 | 0.03 | 0.03 |
| Brightener 1 | 0.12 | 0.1 | 0.18 | 0.08 | 0.1 |
| Balance to 100% perfume / dye and/or water | | | | | |
| #1: Add IN HCl aq. soln to adjust the neat pH of the formula in the range from about 3 to about 5. The pH of Examples above (I)-(II) is about 5 to about 7, and of (III)-(V) is about 7.5 to about 8.5. | | | | | |

| **HDL Detergent Compositions** | | | | | | |
|---|---|---|---|---|---|---|
| **Compound** | **Formulations** | | | | | |
| | **I** | **II** | **III** | **IV** | **V** | **VI** |
| LAS | 11.5 | 11.5 | 9 | - | 4 | - |
| C₁₂-C₁₅AE_{2.85}S | - | - | 3 | 18 | - | 16 |
| C₁₄-C₁₅E₂.₅S | 11.5 | 11.5 | 3 | - | 16 | - |
| C₁₂-C₁₃E₉ | - | - | 3 | 2 | 2 | 1 |
| C₁₂-C₁₃E₇ | 3.2 | 3.2 | - | - | - | - |
| C12-C14 alkyl N-methyl glucamide (CFAA) surfactant | - | - | - | 5 | - | 3 |
| TPKFA (C12-C14 topped whole cut fatty acids) | 2 | 2 | - | 2 | 0.5 | 2 |
| Citric Acid (Anhydrous) | 3.2 | 3.2 | 0.5 | 1.2 | 2 | 1.2 |
| Ca formate | 0.1 | 0.1 | 0.06 | 0.1 | - | - |
| Na formate | 0.5 | 0.5 | 0.06 | 0.1 | 0.05 | 0.05 |
| ZnCl2 | 0.1 | 0.05 | 0.06 | 0.03 | 0.05 | 0.05 |
| Sodium Cumene Sulfonate | 4 | 4 | 1 | 3 | 1.2 | - |
| Borate | 0.6 | 0.6 | 1.5 | - | - | - |
| Sodium Hydroxide | 6 | 6 | 2 | 3.5 | 4 | 3 |
| Ethanol | 2 | 2 | 1 | 4 | 4 | 3 |
| 1,2 Propanediol | 3 | 3 | 2 | 8 | 8 | 5 |
| Monoethanolamine | 3 | 3 | 1.5 | 1 | 2.5 | 1 |
| TEPAE (tetraethylene pentaamine ethoxylate) | 2 | 2 | - | 1 | 1 | 1 |
| Metalloprotease 1 (optional) | 0.03 | 0.05 | - | 0.03 | - | 0.02 |
| Metalloprotease 2 | - | - | 0.01 | - | 0.08 | - |
| Protease A (optional) | - | - | 0.01 | - | - | - |
| Lipase | - | - | - | 0.002 | - | - |
| Amylase | - | - | - | - | 0.002 | - |
| Cellulase | - | - | - | - | - | 0.0001 |
| Pectin Lyase | 0.005 | 0.005 | - | | - | - |
| Aldose Oxidase | 0.05 | - | - | 0.05 | - | 0.02 |
| Galactose oxidase | - | 0.04 | | | | |
| pentaamine acetate cobalt (III) salt PAAC | 0.03 | 0.03 | 0.02 | - | - | - |
| DETBCHD | - | - | - | 0.02 | 0.01 | - |
| SRP1 (anionically end capped polyesters) | 0.2 | 0.2 | - | 0.1 | - | - |
| DTPA | - | - | - | 0.3 | - | - |
| polyvinyl pyridine-N-Oxide (PVNO) | - | - | - | 0.3 | - | 0.2 |
| Brightener 1 | 0.2 | 0.2 | 0.07 | 0.1 | - | - |
| Silicone antifoam | 0.04 | 0.04 | 0.02 | 0.1 | 0.1 | 0.1 |
| Balance to 100% perfume/dye and/or water | | | | | | |

| **Liquid Hand Dishwashing (Hand Dish Liquid) Detergent Compositions** | | | | | | |
|---|---|---|---|---|---|---|
| **Compound** | **Formulations** | | | | | |
| | **I** | **II** | **III** | **IV** | **V** | **VI** |
| C₁₂-C₁₅AE_{1.8}S | 30 | 28 | 25 | - | 15 | 10 |
| LAS | - | - | - | 5 | 15 | 12 |
| Paraffin Sulfonate | - | - | - | 20 | - | - |
| C₁₀-C₁₈ Alkyl Dimethyl Amine Oxide | 5 | 3 | 7 | - | - | - |
| Betaine | 3 | - | 1 | 3 | 1 | - |
| C₁₂ poly-hydroxy fatty acid amide | - | - | - | 3 | - | 1 |
| C₁₄ poly-OH fatty acid amide | - | 1.5 | - | - | - | - |
| C₁₁E₉ | 2 | - | 4 | - | - | 20 |
| DTPA | - | - | - | - | 0.2 | - |
| Tri-sodium Citrate dihydrate (builder) | 0.25 | - | - | 0.7 | - | - |
| Diamine (Dimethyl aminopropyl amine; 1,6-hezane diamine; 1,3-propane diamine; 2-methyl-1,5-pentane diamine; 1,3-pentanediamine; 1-methyl-diaminopropane) | 1 | 5 | 7 | 1 | 5 | 7 |
| MgCl₂ | 0.25 | - | - | 1 | - | - |
| Metalloprotease 1 (optional) | 0.02 | 0.01 | - | 0.01 | - | 0.05 |
| Metalloprotease 2 | - | - | 0.03 | - | 0.02 | - |
| Protease A (optional) | - | 0.01 | - | - | - | - |
| Amylase | 0.001 | - | - | 0.002 | - | 0.001 |
| Aldose Oxidase | 0.03 | - | 0.02 | - | 0.05 | - |
| Sodim Cumene Sulfonate | - | - | - | 2 | 1.5 | 3 |
| pentaamine acetate cobalt (III) salt | 0.01 | 0.01 | 0.02 | - | - | - |
| DETBCHD | - | - | - | 0.01 | 0.02 | 0.01 |
| Balance to 100% perfume / dye and/or water | | | | | | |
| The pH of Examples (I)-(VI) is about 8 to about 11. | | | | | | |

| **Liquid Automatic Dish Washing Detergent Compositions** | | | | | |
|---|---|---|---|---|---|
| **Compound** | **Formulations** | | | | |
| | **I** | **II** | **III** | **IV** | **V** |
| STPP (sodium tripoly phosphate) | 16.00 | 16.00 | 18.00 | 16.00 | 16.00 |
| Potassium Sulfate | - | 10.00 | 8.00 | - | 10.00 |
| 1,2 propanediol | 6.00 | 0.50 | 2.00 | 6.00 | 0.50 |
| Boric Acid | - | - | - | 4.00 | 3.00 |
| CaCl₂ dihydrate | 0.04 | 0.04 | 0.04 | 0.04 | 0.04 |
| Nonionic surfactant | 0.50 | 0.50 | 0.50 | 0.50 | 0.50 |
| Metalloprotease 1 (optional) | 0.10 | 0.03 | - | 0.03 | - |
| Metalloprotease 2 | - | - | 0.05 | - | 0.06 |
| Protease B (optional) | - | - | - | 0.01 | - |
| Amylase | 0.02 | - | 0.02 | 0.02 | - |
| Aldose Oxidase | - | 0.15 | 0.02 | - | 0.01 |
| Galactose Oxidase | - | - | 0.01 | - | 0.01 |
| pentaamine acetate cobalt (III) salt PAAC (bleach catalyst) | 0.01 | - | - | 0.01 | - |
| DETBCHD | - | 0.01 | - | - | 0.01 |
| Balance to 100% perfume / dye and/or water | | | | | |

| **Granular and/or Tablet Detergent Compositions** | | | | | |
|---|---|---|---|---|---|
| **Compound** | **Formulations** | | | | |
| | **I** | **II** | **III** | **IV** | **V** |
| C₁₄-C₁₅AS or TAS (sodium tallow alkyl sulfate) | 8 | 5 | 3 | 3 | 3 |
| LAS | 8 | - | 8 | - | 7 |
| C₁₂-C₁₅AE₃S | 0.5 | 2 | 1 | - | - |
| C₁₂-C₁₅E₅ or E₃ | 2 | - | 5 | 2 | 2 |
| QAS (quarternary ammonium salt) | - | - | - | 1 | 1 |
| Zeolite A | 20 | 18 | 11 | - | 10 |
| SKS-6 (dry add) (layered silicate) | - | - | 9 | - | - |
| MA/AA (acrylate/maleate copolymer) | 2 | 2 | 2 | - | - |
| AA (polyacrylate polymer) | - | - | - | - | 4 |
| 3Na Citrate 2H₂O | - | 2 | - | - | - |
| Citric Acid (Anhydrous) | 2 | - | 1.5 | 2 | - |
| DTPA | 0.2 | 0.2 | - | - | - |
| EDDS | - | - | 0.5 | 0.1 | - |
| HEDP | - | - | 0.2 | 0.1 | - |
| PB 1 (sodium perborate monohydrate) | 3 | 4.8 | - | - | 4 |
| Percarbonate | - | - | 3.8 | 5.2 | - |
| NOBS | 1.9 | - | - | - | - |
| NACA OBS | - | - | 2 | - | - |
| TAED | 0.5 | 2 | 2 | 5 | 1 |
| BB1 (3-(3,4-Dihydroisoquinolinium)propane sulfonate (DIPS)) | 0.06 | - | 0.34 | - | 0.14 |
| BB2 3-(3,4-Dihydroisoquinolinium)-decane-2-sulfate | - | 0.14 | - | 0.2 | - |
| Anhydrous sodium carbonate | 15 | 18 | - | 15 | 15 |
| Sulfate | 5 | 12 | 5 | 17 | 3 |
| Silicate | - | 1 | - | - | 8 |
| Metalloprotease 1 (optional) | 0.03 | - | 0.1 | 0.06 | - |
| Metalloprotease 2 | - | 0.05 | - | - | 0.1 |
| Protease B (optional) | - | 0.01 | - | - | - |
| Protease C (optional) | - | - | - | 0.01 | - |
| Lipase | - | 0.008 | - | - | - |
| Amylase | 0.001 | - | - | - | 0.001 |
| Cellulase | - | 0.0014 | - | - | - |
| Pectin Lyase | 0.001 | 0.001 | 0.001 | 0.001 | 0.001 |
| Aldose Oxidase | 0.03 | - | 0.05 | - | - |
| pentaamine acetate cobalt (III) salt PAAC | - | 0.01 | - | - | 0.05 |
| Balance to 100% Moisture and/or Minors^{∗} | | | | | |
| ^{∗} Perfume, dye, brightener / SRP1 / Na carboxymethylcellulose/ photobleach / MgSO4 / PVPVI/ suds suppressor /high molecular PEG/clay. | | | | | |

| **High Density Automatic Dish Washing Detergent Compositions** | | | | | | |
|---|---|---|---|---|---|---|
| **Compound** | **Formulations** | | | | | |
| | **I** | **II** | **III** | **IV** | **V** | **VI** |
| STPP (sodium tripoly phosphate) | - | 45 | 45 | - | - | 40 |
| 3Na Citrate 2H₂O | 17 | - | - | 50 | 40.2 | - |
| Na Carbonate | 17.5 | 14 | 20 | - | 8 | 33.6 |
| Bicarbonate | - | - | - | 26 | - | - |
| Silicate | 15 | 15 | 8 | - | 25 | 3.6 |
| Metasilicate | 2.5 | 4.5 | 4.5 | - | - | - |
| PB 1 (sodium perborate monohydrate) | - | - | 4.5 | - | - | - |
| PB4 (sodium perborate tetrahydrate) | - | - | - | 5 | - | - |
| Percarbonate | - | - | - | - | - | 4.8 |
| BB1 (3-(3,4-Dihydroisoquinolinium)propane sulfonate (DIPS)) | - | 0.1 | 0.1 | - | 0.5 | - |
| BB2 3-(3,4-Dihydroisoquinolinium)-decane-2-sulfate | 0.2 | 0.05 | - | 0.1 | - | 0.6 |
| Nonionic detergent | 2 | 1.5 | 1.5 | 3 | 1.9 | 5.9 |
| HEDP | 1 | - | - | - | - | - |
| DETPMP | 0.6 | - | - | - | - | - |
| pentaamine acetate cobalt (III) salt PAAC | 0.03 | 0.05 | 0.02 | - | - | - |
| Paraffin oil Winog 70 | 0.5 | 0.4 | 0.4 | 0.6 | - | - |
| Metalloprotease 1 (optional) | 0.072 | 0.053 | - | 0.026 | - | 0.01 |
| Metalloprotease 2 | - | - | 0.053 | - | 0.059 | - |
| Protease B (optional) | - | - | - | - | - | 0.01 |
| Amylase | 0.012 | - | 0.012 | - | 0.021 | 0.006 |
| Lipase | - | 0.001 | - | 0.005 | - | - |
| Pectin Lyase | 0.001 | 0.001 | 0.001 | - | - | - |
| Aldose Oxidase | 0.05 | 0.05 | 0.03 | 0.01 | 0.02 | 0.01 |
| BTA (benzotriazole) | 0.3 | 0.2 | 0.2 | 0.3 | 0.3 | 0.3 |
| Polycarboxylate | 6 | - | - | - | 4 | 0.9 |
| Perfume | 0.2 | 0.1 | 0.1 | 0.2 | 0.2 | 0.2 |
| Balance to 100% Moisture and/or Minors^{∗} | | | | | | |
| ^{∗}Brightener / dye / SRP1 / Na carboxymethylcellulose/ photobleach / MgSO4 / PVPVI/ suds suppressor /high molecular PEG/clay. The pH of Examples (I) through (VI) is from about 9.6 to about 11.3. | | | | | | |

| **Tablet Detergent Compositions** | | | | | | | | |
|---|---|---|---|---|---|---|---|---|
| **Compound** | **Formulations** | | | | | | | |
| | **I** | **II** | **III** | **IV** | **V** | **VI** | **VII** | **VIII** |
| STPP (sodium tripoly phosphate) | - | 48.8 | 44.7 | 38.2 | - | 42.4 | 46.1 | 46 |
| 3Na Citrate 2H₂O | 20 | - | - | - | 35.9 | - | - | - |
| Na Carbonate | 20 | 5 | 14 | 15.4 | 8 | 23 | 20 | - |
| Silicate | 15 | 14.8 | 15 | 12.6 | 23.4 | 2.9 | 4.3 | 4.2 |
| Lipase | 0.001 | - | 0.01 | - | 0.02 | - | - | - |
| Protease B | 0.01 | - | - | - | - | - | - | - |
| Protease C | - | - | - | - | - | 0.01 | - | - |
| Metalloprotease 1 (optional) | 0.01 | 0.08 | - | 0.04 | - | 0.023 | - | 0.05 |
| Metalloprotease 2 | - | - | 0.05 | - | 0.052 | - | 0.023 | - |
| Amylase | 0.012 | 0.012 | 0.012 | - | 0.015 | - | 0.017 | 0.002 |
| Pectin Lyase | 0.005 | - | - | 0.002 | - | - | - | - |
| Aldose Oxidase | - | 0.03 | - | 0.02 | 0.02 | - | 0.03 | - |
| PB 1 (sodium perborate monohydrate) | - | - | 3.8 | - | 7.8 | - | - | 4.5 |
| Percarbonate | 6 | - | - | 6 | - | 5 | - | - |
| BB1 (3-(3,4-Dihydroisoquinolinium)propane sulfonate (DIPS)) | 0.2 | - | 0.5 | - | 0.3 | 0.2 | - | - |
| BB2 3-(3,4-Dihydroisoquinolinium)-decane-2-sulfate | - | 0.2 | - | 0.5 | - | - | 0.1 | 0.2 |
| Nonionic surfactant | 1.5 | 2 | 2 | 2.2 | 1 | 4.2 | 4 | 6.5 |
| pentaamine acetate cobalt (III) salt PAAC | 0.01 | 0.01 | 0.02 | - | - | - | - | - |
| DETBCHD | - | - | - | 0.02 | 0.02 | - | - | - |
| TAED | - | - | - | - | - | 2.1 | - | 1.6 |
| HEDP | 1 | - | - | 0.9 | - | 0.4 | 0.2 | - |
| DETPMP | 0.7 | - | - | - | - | - | - | - |
| Paraffin oil Winog 70 | 0.4 | 0.5 | 0.5 | 0.5 | - | - | 0.5 | - |
| BTA (benzotriazole) | 0.2 | 0.3 | 0.3 | 0.3 | 0.3 | 0.3 | 0.3 | - |
| Polycarboxylate | 4 | - | - | - | 4.9 | 0.6 | 0.8 | - |
| PEG 400-30,000 | - | - | - | - | - | 2 | - | 2 |
| Glycerol | - | - | - | - | - | 0.4 | - | 0.5 |
| Perfume | - | - | - | 0.05 | 0.2 | 0.2 | 0.2 | 0.2 |
| Balance to 100% Moisture and/or Minors^{∗} | | | | | | | | |
| ^{∗}Brightener / SRP1 / Na carboxymethylcellulose/ photobleach / MgSO4 / PVPVI/ suds suppressor /high molecular PEG/clay. | | | | | | | | |
| The pH of Examples (I) through (VII) is from about 10 to about 11.5; pH of (VIII) is from 8-10. The tablet weight of Examples (I) through (VIII) is from about 20 grams to about 30 grams. | | | | | | | | |

| **Liquid Hard Surface Detergent Compositions** | | | | | | | |
|---|---|---|---|---|---|---|---|
| **Compound** | **Formulations** | | | | | | |
| | **I** | **II** | **III** | **IV** | **V** | **VI** | **VII** |
| C₉-C₁₁E₅ | 2.4 | 1.9 | 2.5 | 2.5 | 2.5 | 2.4 | 2.5 |
| C₁₂-C₁₄E₅ | 3.6 | 2.9 | 2.5 | 2.5 | 2.5 | 3.6 | 2.5 |
| C₇-C₉E₆ | - | - | - | - | 8 | - | - |
| C₁₂-C₁₄E₂₁ | 1 | 0.8 | 4 | 2 | 2 | 1 | 2 |
| LAS | - | - | - | 0.8 | 0.8 | - | 0.8 |
| Sodim Cumene Sulfonate | 1.5 | 2.6 | - | 1.5 | 1.5 | 1.5 | 1.5 |
| Isachem ^{®} AS (branched alcohol alkyl sulfate) | 0.6 | 0.6 | - | - | - | 0.6 | - |
| Na₂CO₃ | 0.6 | 0.13 | 0.6 | 0.1 | 0.2 | 0.6 | 0.2 |
| 3Na Citrate 2H₂O | 0.5 | 0.56 | 0.5 | 0.6 | 0.75 | 0.5 | 0.75 |
| NaOH | 0.3 | 0.33 | 0.3 | 0.3 | 0.5 | 0.3 | 0.5 |
| Fatty Acid | 0.6 | 0.13 | 0.6 | 0.1 | 0.4 | 0.6 | 0.4 |
| 2-butyl octanol | 0.3 | 0.3 | - | 0.3 | 0.3 | 0.3 | 0.3 |
| PEG DME-2000^{®} | 0.4 | - | 0.3 | 0.35 | 0.5 | - | - |
| PVP (vinylpyrrolidone homopolymer) | 0.3 | 0.4 | 0.6 | 0.3 | 0.5 | - | - |
| MME PEG (2000) ^{®} | - | - | - | - | - | 0.5 | 0.5 |
| Jeffamine ^{®} ED-2001 (capped polyethylene glycol) | - | 0.4 | - | - | 0.5 | - | - |
| pentaamine acetate cobalt (III) salt PAAC | - | - | - | 0.03 | 0.03 | 0.03 | - |
| DETBCHD | 0.03 | 0.05 | 0.05 | - | - | - | - |
| Metalloprotease 1 (optional) | 0.07 | - | 0.08 | 0.03 | - | 0.01 | 0.04 |
| Metalloprotease 2 | - | 0.05 | - | - | 0.06 | - | - |
| Protease B (optional) | - | - | - | - | - | 0.01 | - |
| Amylase | 0.12 | 0.01 | 0.01 | - | 0.02 | - | 0.01 |
| Lipase | - | 0.001 | - | 0.005 | - | 0.005 | - |
| Pectin Lyase | 0.001 | - | 0.001 | - | - | - | 0.002 |
| ZnCl2 | 0.02 | 0.01 | 0.03 | 0.05 | 0.1 | 0.05 | 0.02 |
| Calcium Formate | 0.03 | 0.03 | 0.01 | - | - | - | - |
| PB 1 (sodium perborate monohydrate) | - | 4.6 | - | 3.8 | - | - | - |
| Aldose Oxidase | 0.05 | - | 0.03 | - | 0.02 | 0.02 | 0.05 |
| Balance to 100% perfume / dye and/or water | | | | | | | |
| The pH of Examples (I) through (VII) is from about 7.4 to about 9.5. | | | | | | | |

| **HDL Detergent Compositions** | | | | |
|---|---|---|---|---|
| **Ingredient** | **Composition** | | | |
| | **(wt% of composition)** | | | |
| | **1** | **2** | **3** | **4** |
| C₁₂₋₁₅ Alkylethoxy(1.8)sulfate | 14.7 | 11.6 | | 16.31 |
| C_{11.8} Alkylbenzene sulfonate | 4.3 | 11.6 | 8.3 | 7.73 |
| C₁₆₋₁₇ Branched alkyl sulfate | 1.7 | 1.29 | | 3.09 |
| C₁₂₋₁₄ Alkyl -9-ethoxylate | 0.9 | 1.07 | | 1.31 |
| C₁₂ dimethylamine oxide | 0.6 | 0.64 | | 1.03 |
| Citric acid | 3.5 | 0.65 | 3 | 0.66 |
| C₁₂₋₁₈ fatty acid | 1.5 | 2.32 | 3.6 | 1.52 |
| Sodium Borate (Borax) | 2.5 | 2.46 | 1.2 | 2.53 |
| Sodium C₁₂₋₁₄ alkyl ethoxy 3 sulfate | | | 2.9 | |
| C₁₄₋₁₅ alkyl 7-ethoxylate | | | 4.2 | |
| C₁₂₋₁₄ Alkyl -7-ethoxylate | | | 1.7 | |
| Ca formate | 0.09 | 0.09 | | 0.09 |
| A compound having the following general structure: bis((C₂H₅O)(C₂H₄O)n)(CH₃)-N⁺-CₓH₂ₓ-N⁺-(CH₃)-bis((C₂H₅O)(C₂H₄O)n), wherein n = from 20 to 30, and x = from 3 to 8, or sulphated or sulphonated variants thereof | | | 1.2 | |
| Random graft co-polymer¹ | | 1.46 | 0.5 | |
| Ethoxylated Polyethylenimine ² | 1.5 | 1.29 | | 1.44 |
| Diethylene triamine pentaacetic acid | 0.34 | 0.64 | | 0.34 |
| Diethylene triamine penta(methylene phosphonic acid) | | | 0.3 | |
| Tinopal AMS-GX | | 0.06 | | |
| Tinopal CBS-X | 0.2 | 0.17 | | 0.29 |
| Amphiphilic alkoxylated grease cleaning polymer ³ | 1.28 | 1 | 0.4 | 1.93 |
| Ethanol | 2 | 1.58 | 1.6 | 5.4 |
| Propylene Glycol | 3.9 | 3.59 | 1.3 | 4.3 |
| Diethylene glycol | 1.05 | 1.54 | | 1.15 |
| Polyethylene glycol | 0.06 | 0.04 | | 0.1 |
| Monoethanolamine | 3.05 | 2.41 | 0.4 | 1.26 |
| NaOH | 2.44 | 1.8 | | 3.01 |
| Sodium Cumene Sulphonate | | | 1 | |
| Sodium Formate | | 0.11 | | 0.09 |
| Water, Aesthetics (Dyes, perfumes) and Minors (Enzymes, solvents, structurants) | balance | balance | balance | balance |
| 1 Random graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units. | | | | |
| 2 Polyethylenimine (MW = 600) with 20 ethoxylate groups per -NH. | | | | |
| 3 Amphiphilic alkoxylated grease cleaning polymer is a polyethylenimine (MW = 600) with 24 ethoxylate groups per -NH and 16 propoxylate groups per -NH | | | | |

| **Light-Duty Liquid Dishwashing Detergent Compositions** | | | | |
|---|---|---|---|---|
| **Composition** | **1** | **2** | **3** | **4** |
| Linear Alkylbenzene Sulfonate (1) | - | | - | - |
| Alkyl Ethoxy Sulfate (2) | 18% | 17% | 17% | 18% |
| Paraffin Sulfonate (C15) | - | - | - | - |
| CAP= coco amido propyl Betaine | - | - | 9% | 5% |
| Nonionic (3) | - | - | 1% | - |
| Amine Oxide (4) | 6% | 5.50% | - | 4% |
| Alkylpolyglucoside | | | | 4% |
| Alcohol (5) | - | - | 5% | 7% |
| Pura= polypropyleneglycol | 1% | 0.80% | - | - |
| Citrate | - | - | 0.30% | 0.60% |
| Salt (6) | 1.20% | 1.00% | - | 0.50% |
| SCS= sodium cumene sulfonate | - | - | 0.80% | - |
| glycerol | 15% | 5% | 3% | - |
| Na-lactate | - | - | - | 5% |
| cationic polymer (7) | 0.10% | 0.10% | 0.30% | 0.20% |
| Protease of this invention | 0.0075 | 0.005 | 0.0025 | 0.03 |
| Glycol distearate from Euperlan^{®} Cognis | 0.4 | 0 | 0.4 | 0 |
| Hydrogenated Castor Oil Thixcin^{®} Elementis | 0 | 0.1 | 0 | 0.1 |
| Mica (BASF Mearlin superfine) | 0 | 0.05 | 0 | 0.05 |
| Minors^{∗} | Balance to 100% with water | | | |
| pH | 9 | 9 | 6 | 6 |
| Optional Minors^{∗}: dyes, opacifier, perfumes, preservatives, hydrotropes, processing aids, and/or stabilizers. | | | | |
| (1) Linear Alkylbenzene Sulfonate: LAS: C11.4 | | | | |
| (2) Alkyl Ethoxy Sulfate: AExS : | | | | |
| (3) Nonionic: AlkylEthoxylate | | | | |
| (4) Di-methyl coco alkyl amine oxide | | | | |
| (5) Alcohol: Ethanol | | | | |
| (6) Salt: NaCl | | | | |
| (7) cationically modified hydroxyethyl cellulose (Polyquaternium-10 - UCARE LR-400 ex Amerchol). | | | | |

| **Liquid laundry detergent compositions suitable for front-loading automatic washing machines** | | | | | | | | |
|---|---|---|---|---|---|---|---|---|
| | **Composition** | | | | | | | |
| **Ingredient** | **(wt% of composition)** | | | | | | | |
| | **1** | **2** | **3** | **4** | **5** | **6** | **7** | **8** |
| Alkylbenzene sulfonic acid | 7 | 11 | 4.5 | 1.2 | 1.5 | 12.5 | 5.2 | 4 |
| Sodium C₁₂₋₁₄ alkyl ethoxy 3 sulfate | 2.3 | 3.5 | 4.5 | 4.5 | 7 | 18 | 1.8 | 2 |
| C₁₄₋₁₅ alkyl 8-ethoxylate | 5 | 8 | 2.5 | 2.6 | 4.5 | 4 | 3.7 | 2 |
| C₁₂ alkyl dimethyl amine oxide | - | - | 0.2 | - | - | - | - | - |
| C₁₂₋₁₄ alkyl hydroxyethyl dimethyl ammonium chloride | - | - | - | 0.5 | - | - | - | - |
| C₁₂₋₁₈ Fatty acid | 2.6 | 4 | 4 | 2.6 | 2.8 | 11 | 2.6 | 1.5 |
| Citric acid | 2.6 | 3 | 1.5 | 2 | 2.5 | 3.5 | 2.6 | 2 |
| Protease ^{∗} | 0.05 | 0.03 | 0.0 4 | 0.03 | 0.04 | 0.03 | 0.03 | 0.02 |
| Amylase | 0.1 | 0.2 | 0.1 5 | - | 0.05 | 0.5 | 0.1 | 0.2 |
| Mannanase | 0.05 | 0.1 | 0.0 5 | - | - | 0.1 | 0.04 | - |
| Random graft co-polymer¹ | 1 | 0.2 | 1 | 0.4 | 0.5 | 2.7 | 0.3 | 1 |
| A compound having the following general structure: bis((C₂H₅O)(C₂H₄O)n)(CH₃)-N⁺-CₓH₂ₓ-N⁺-(CH₃)-bis((C₂H₅O)(C₂H₄O)n), wherein n = from 20 to 30, and x = from 3 to 8, or sulphated or sulphonated variants thereof | 0.4 | 2 | 0.4 | 0.6 | 1.5 | 1.8 | 0.7 | 0.3 |
| Ethoxylated Polyethylenimine ² | - | - | - | - | - | 0.5 | - | - |
| Amphiphilic alkoxylated grease cleaning polymer ³ | 0.1 | 0.2 | 0.1 | 0.2 | 0.3 | 0.3 | 0.2 | 0.3 |
| Diethoxylated poly (1,2 propylene terephthalate) | - | - | - | - | - | - | 0.3 | - |
| Diethylenetriaminepenta(methylenephospho nic) acid | 0.2 | 0.3 | - | - | 0.2 | - | 0.2 | 0.3 |
| Hydroxyethane diphosphonic acid | - | - | 0.4 5 | - | - | 1.5 | - | 0.1 |
| FWA (fluorescent whitening agent) | 0.1 | 0.2 | 0.1 | - | - | 0.2 | 0.05 | 0.1 |
| Solvents (1,2 propanediol, ethanol), | 3 | 4 | 1.5 | 1.5 | 2 | 4.3 | 2 | 1.5 |
| Hydrogenated castor oil derivative | 0.4 | 0.4 | 0.3 | 0.1 | 0.3 | - | 0.4 | 0.5 |
| Boric acid | 1.5 | 2.5 | | 1.5 | 1.5 | 0.5 | 1.5 | 1.5 |
| Na formate | - | - | - | 1 | - | - | - | - |
| Reversible protease inhibitor⁴ | - | - | 0.0 02 | - | - | - | - | - |
| Perfume | 0.5 | 0.7 | 0.5 | 0.5 | 0.8 | 1.5 | 0.5 | 0.8 |
| Perfume MicroCapsules slurry (30%am) | 0.2 | 0.3 | 0.7 | 0.2 | 0.05 | 0.4 | 0.9 | 0.7 |
| Ethoxylated thiophene Hueing Dye⁵ | 0.005 | 0.007 | 0.0 1 | 0.00 8 | 0.00 8 | 0.00 7 | 0.00 7 | 0.00 8 |
| Buffers (sodium hydroxide, Monoethanolamine) | To pH 8.2 | | | | | | | |
| Water and minors (antifoam, aesthetics) | To 100% | | | | | | | |
| ¹ Random graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units. | | | | | | | | |
| ² Polyethylenimine (MW = 600) with 20 ethoxylate groups per -NH. | | | | | | | | |
| ³ Amphiphilic alkoxylated grease cleaning polymer is a polyethylenimine (MW = 600) with 24 ethoxylate groups per -NH and 16 propoxylate groups per -N H | | | | | | | | |
| ⁵Ethoxylated thiophene Hueing Dye is as described in US 7,208,459 B2. | | | | | | | | |
| ^{∗} Remark: all enzyme levels expressed as % enzyme raw material, except for protease which is expressed as % of active protein added to the product.. | | | | | | | | |
| ⁴ Reversible Protease inhibitor of structure: | | | | | | | | |
| 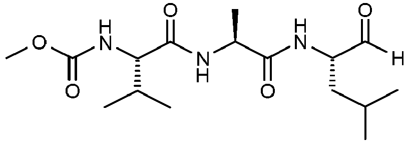 | | | | | | | | |

| **Liquid laundry detergent compositions suitable for top-loading automatic washing machines** | | | | | | | | |
|---|---|---|---|---|---|---|---|---|
| **Ingredient** | **Composition** | | | | | | | |
| | **(wt% of composition)** | | | | | | | |
| | **1** | **2** | **3** | **4** | **5** | **6** | **7** | **8** |
| C₁₂₋₁₅ Alkylethoxy( 1. 8)sulfate | 20.1 | 15.1 | 20 | 15.1 | 13.7 | 16.7 | 10 | 9.9 |
| C_{11.8} Alkylbenzene sulfonate | 2.7 | 2 | 1 | 2 | 5.5 | 5.6 | 3 | 3.9 |
| C₁₆₋₁₇ Branched alkyl sulfate | 6.5 | 4.9 | | 4.9 | 3 | 9 | 2 | |
| C₁₂₋₁₄ Alkyl -9-ethoxylate | 0.8 | 0.8 | 0.8 | 0.8 | 8 | 1.5 | 0.3 | 11.5 |
| C₁₂ dimethylamine oxide | | | 0.9 | | | | | |
| Citric acid | 3.8 | 3.8 | 3.8 | 3.8 | 3.5 | 3.5 | 2 | 2.1 |
| C₁₂₋₁₈ fatty acid | 2 | 1.5 | 2 | 1.5 | 4.5 | 2.3 | | 0.9 |
| Protease ^{∗} | 0.1 | 0.2 | 0.1 | 0.1 | 0.1 | 0.1 | 0.1 | 0.1 |
| Amylase 1 | 0.7 | 0.3 | 0.6 | 0.3 | 0.6 | 0.4 | | |
| Amylase 2 | | | | | | | | 1.1 |
| Mannanase | 0.1 | | | | | 0.1 | | |
| Pectate Lyase | 0.1 | | | | | 0.2 | | |
| Borax | 3 | 3 | | | 2 | 3 | 3 | 3.3 |
| Na & Ca formate | 0.2 | 0.2 | | 0.2 | 0.2 | | 0.7 | |
| A compound having the following general structure: bis((C₂H₅O)(C₂H₄O)n)(CH ₃)-N⁺-CₓH₂ₓ-N⁺-(CH₃)-bis((C₂H₅O)(C₂H₄O)n), wherein n = from 20 to 30, and x = from 3 to 8, or sulphated or sulphonated variants thereof | 1.6 | 1.6 | 3 | 1.6 | 2 | 1.6 | 1.3 | 1.2 |
| Random graft co-polymer¹ | 0.4 | 0.2 | 1 | 0.5 | 0.6 | 1 | 0.8 | 1 |
| Diethylene triamine pentaacetic acid | 0.4 | 0.4 | 0.4 | 0.4 | 0.2 | 0.3 | 0.8 | |
| Tinopal AMS-GX (brightener) | 0.2 | 0.2 | 0.2 | 0.2 | 0.2 | 0.3 | 0.1 | |
| Tinopal CBS-X (brightener) | | | | | | 0.1 | | 0.2 |
| Amphiphilic alkoxylated grease cleaning polymer ³ | 1 | 1.3 | 1.3 | 1.4 | 1 | 1.1 | 1 | 1 |
| Texcare 240N (Clariant) | | | | 1 | | | | |
| | **1** | **2** | **3** | **4** | **5** | **6** | **7** | **8** |
| Ethanol | 2.6 | 2.6 | 2.6 | 2.6 | 1.8 | 3 | 1.3 | |
| Propylene Glycol | 4.6 | 4.6 | 4.6 | 4.6 | 3 | 4 | 2.5 | |
| Diethylene glycol | 3 | 3 | 3 | 3 | 3 | 2.7 | 3.6 | |
| Polyethylene glycol | 0.2 | 0.2 | 0.2 | 0.2 | 0.1 | 0.3 | 0.1 | 1.4 |
| Monoethanolamine | 2.7 | 2.7 | 2.7 | 2.7 | 4.7 | 3.3 | 1.7 | 0.4 |
| Triethanolamine | | | | | | | | 0.9 |
| NaOH | to pH 8.3 | to pH 8.3 | to pH 8.3 | to pH 8.3 | to pH 8.3 | to pH 8.3 | to pH 8.3 | to pH 8.5 |
| Suds suppressor | | | | | | | | |
| Dye | 0.01 | 0.01 | 0.01 | | 0.01 | 0.01 | 0.01 | 0 |
| Perfume | 0.5 | 0.5 | 0.5 | 0.5 | 0.7 | 0.7 | 0.8 | 0.6 |
| Perfume MicroCapsules slurry (30%am) | 0.2 | 0.5 | 0.2 | 0.3 | 0.1 | 0.3 | 0.9 | 1 |
| Ethoxylated thiophene Hueing Dye⁵ | 0.003 | 0.002 | 0.002 | 0.005 | 0.002 | 0.004 | 0.004 | 0.003 |
| Water | Balance | Balance | Balance | Balance | Balance | Balance | Balance | Balance |
| ¹ Random graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units. | | | | | | | | |
| ³ Amphiphilic alkoxylated grease cleaning polymer is a polyethylenimine (MW = 600) with 24 ethoxylate groups per -NH and 16 propoxylate groups per -NH | | | | | | | | |
| ⁵Ethoxylated thiophene Hueing Dye is as described in US 7,208,459 B2. | | | | | | | | |
| ^{∗} Remark: all enzyme levels expressed as % enzyme raw material, except for protease which is expressed as % of active protein added to the product.. | | | | | | | | |

| **Granular detergent compositions** | | | | | | |
|---|---|---|---|---|---|---|
| **Component** | **1** | **2** | **3** | **4** | **5** | **6** |
| Linear alkylbenzenesulfonate with aliphatic carbon chain length C₁₁-C₁₂ | 15 | 12 | 20 | 10 | 12 | 13 |
| Other surfactants | 1.6 | 1.2 | 1.9 | 3.2 | 0.5 | 1.2 |
| Phosphate builder(s) | 2 | 3 | 4 | | | |
| Zeolite | | 1 | | 1 | 4 | 1 |
| Silicate | 4 | 5 | 2 | 3 | 3 | 5 |
| Sodium Carbonate | 2 | 5 | 5 | 4 | 0 | 3 |
| Polyacrylate (MW 4500) | 1 | 0.6 | 1 | 1 | 1.5 | 1 |
| Carboxymethyl cellulose (Finnfix BDA ex CPKelco) | 1 | - | 0.3 | - | 1.1 | - |
| Cellulase | 0.23 | 0.17 | 0.5 | 0.2 | 0.2 | 0.6 |
| Protease | 0.23 | 0.17 | 0.5 | 0.2 | 0.2 | 0.6 |
| Amylase | 0.23 | 0.17 | 0.5 | 0.2 | 0.2 | 0.6 |
| Fluorescent Brightener(s) | 0.16 | 0.06 | 0.16 | 0.18 | 0.16 | 0.16 |
| Diethylenetriamine pentaacetic acid or Ethylene diamine tetraacetic acid | 0.6 | | 0.6 | 0.25 | 0.6 | 0.6 |
| MgSO₄ | 1 | 1 | 1 | 0.5 | 1 | 1 |
| Bleach(es) and Bleach activator(s) | 6.88 | | 6.12 | 2.09 | 1.17 | 4.66 |
| Ethoxylated thiophene Hueing Dye⁵ | 0.002 | 0.001 | 0.003 | 0.003 | - | - |
| Direct Violet 9 ex Ciba Specialty Chemicals | | | | 0.0006 | 0.0004 | 0.0006 |
| Sulfate/Citric Acid/ Sodium Bicarbonate/Moisture/perfume | Balance to 100% | | | | | |
| ⁵Ethoxylated thiophene Hueing Dye is as described in US 7,208,459 B2. | | | | | | |

| **Granular Laundry Detergent Compositions and Their Components** | | | | | | |
|---|---|---|---|---|---|---|
| **Component** | **Detergent Compositions** | | | | | |
| | **1** | **2** | **3** | **4** | **5** | **6** |
| Linear alkylbenzenesulfonate with aliphatic carbon chain length C₁₁-C₁₂ | 15 | 12 | 20 | 10 | 12 | 13 |
| Other surfactants | 1.6 | 1.2 | 1.9 | 3.2 | 0.5 | 1.2 |
| Phosphate builder(s) | 2 | 3 | 4 | | | |
| Zeolite | | 1 | | 1 | 4 | 1 |
| Silicate | 4 | 5 | 2 | 3 | 3 | 5 |
| Sodium Carbonate | 2 | 5 | 5 | 4 | 0 | 3 |
| Polyacrylate (MW 4500) | 1 | 0.6 | 1 | 1 | 1.5 | 1 |
| Carboxymethyl cellulose | 1 | - | 0.3 | - | 1.1 | - |
| Cellulase (15.6mg/g) | 0.23 | 0.17 | 0.5 | 0.2 | 0.2 | 0.6 |
| Protease | 0.23 | 0.17 | 0.05 | 0.2 | 0.03 | 0.1 |
| Amylase (14mg/g) | 0.23 | 0.17 | 0.5 | 0.2 | 0.2 | 0.6 |
| Mannanase (4mg/g) | 0.1 | | | 0.1 | | 0.1 |
| Lipase (18.6mg/g) | 0.2 | | 0.1 | | 0.3 | |
| Fluorescent Brightener(s) | 0.16 | 0.06 | 0.16 | 0.18 | 0.16 | 0.16 |
| Diethylenetriamine pentaacetic acid or Ethylene diamine tetraacetic acid | 0.6 | | 0.6 | 0.25 | 0.6 | 0.6 |
| MgSO₄ | 1 | 1 | 1 | 0.5 | 1 | 1 |
| Bleach(es) and Bleach activator(s) | 6.88 | | 6.12 | 2.09 | 1.17 | 4.66 |
| Ethoxylated thiophene Hueing Dye⁵ | 0.002 | 0.001 | 0.003 | 0.003 | - | - |
| Direct Violet 9 ex Ciba Specialty Chemicals | | | | 0.0006 | 0.0004 | 0.0006 |
| Sulfate/Citric Acid/ Sodium Bicarbonate/ Moisture/perfume | Balance to 100% | | | | | |
| ⁵Ethoxylated thiophene Hueing Dye is as described in US 7,208,459 B2. | | | | | | |

| **Component** | **Detergent Composition** | | | | | |
|---|---|---|---|---|---|---|
| | **7** | **8** | **9** | **10** | **11** | |
| Surfactants | | | | | | |
| C₁₆₋₁₇ Branched alkyl sulfate | 3.55 | 15.8 | | | | |
| C₁₂₋₁₄ alkyl sulphate | | | 1.5 | | | |
| Sodium linear alkylbenzenesulfonate with aliphatic chain length C₁₁-C₁₂ | 9.6 | | 10.6 | 7.5 | 9 | |
| Sodium C_{14/15} alcohol ethoxy - 3 - sulfate | 1.15 | | | 2.88 | | |
| Sodium C_{14/15} alkyl sulphate | 2.37 | | | | | |
| C_{14/15} alcohol ethoxylate with average 7 moles of ethoxylation | | | | 1.17 | 1 | |
| mono-C₈₋₁₀ alkyl mono-hydroxyethyl di-methyl quaternary ammonium chloride | | | | | 0.45 | |
| Dimethyl hydroxyl ethyl lauryl ammonium chloride | | | 0.18 | | | |
| Zeolite A | 13.9 | 4.7 | 0.01 | 2.9 | 1.8 | |
| Sodium Silicate 1.6.ratio | 4 | 0.2 | | 4 | 4 | |
| Sodium Silicate 2.35.ratio | | | 8 | | | |
| Citric Acid | | | | 2.5 | 1.4 | |
| Sodium tripolyphosphate | | | 5 | | | |
| Sodium Carbonate | 24.1 | 30 | 16.9 | 24.4 | 21 | |
| Nonanoyloxybenzenesuplhonate | 5.78 | 2.81 | 0.96 | | | |
| Oxaziridinium-based bleach booster | | | | 0.03 | 0.017 | |
| Tetrasodium S,S,-ethylenediaminedisuccinate | | | | 0.2 | | |
| Diethylenetriamine penta (methylene phosphonic acid), heptasodium salt | 0.61 | | | | 0.33 | |
| Hydroxyethane dimethylene phosphonic acid | | | | 0.29 | 0.45 | |
| Ethylene diamine tetraacetate | | | 0.27 | | | |
| MgSO4 | | | 0.47 | 0.5994 | 0.782 | |
| Sodium Percarbonate | 7 | 4.4 | | 15.9 | 19.1 | |
| Tetra Acetyl Ethylene Diamine | | | | 3.3 | 4.6 | |
| Sodium Perborate Monohydrate | | | 1.2 | | | |
| Carboxymethyl cellulose | 0.1 | | 0.17 | 1.69 | 0.23 | |
| (e.g., Finnfix BDA ex CPKelco) | | | | | | |
| Sodium Acrylic acid/maleic acid co-polymer (70/30) | 0.0236 | 3.8 | | 2 | 2.5 | |
| Sodium polyacrylate (Sokalan PA30 CL) | 4 | | 0.84 | | | |
| Terephthalate polymer | | | | 0.23 | | |
| Polyethylene glycol/vinyl acetate random graft co polymer | | | 0.89 | 0.89 | 0.91 | |
| Photobleach- zinc phthalocyanine tetrasulfonate | | | 0.005 | 0.001 | 0.002 | |
| C.I. Fluorescent Brightener 260 | 0.11 | 0.15 | 0.04 | 0.23 | 0.15 | |
| C.I. Fluorescent Brightener 351 (Tinopal^{®} CBS) | | | 0.1 | | | |
| Suds suppressor granule | | 0.25 | | 0.07 | 0.04 | |
| Hydrophobically modified carboxy methyl cellulose (Finnifix^{®} SH-1) | | | 0.019 | 0.028 | | |
| Bentonite | | | 8.35 | | | |
| Miscellaneous (Dyes, perfumes, process aids, moisture and sodium sulphate) | Balance | Balance | Balance | Balance | Balance | |

| **Unit Dose Detergent Compositions** | | | | | |
|---|---|---|---|---|---|
| **Ingredients** | **1** | **2** | **3** | **4** | **5** |
| Alkylbenzene sulfonic acid C 11-13, 23.5% 2-phenyl isomer | 14.5 | 14.5 | 14.5 | 14.5 | 14.5 |
| C₁₂₋₁₄ alkyl ethoxy 3 sulfate | 7.5 | 7.5 | 7.5 | 7.5 | 7.5 |
| C₁₂₋₁₄ alkyl 7-ethoxylate | 13 | 13 | 13 | 13 | 13 |
| Citric Acid | 0.6 | 0.6 | 0.6 | 0.6 | 0.6 |
| Fatty Acid | 14.8 | 14.8 | 14.8 | 14.8 | 14.8 |
| Enzymes (as % raw material not active) | 1.7 | 1.7 | 1.7 | 1.7 | 1.7 |
| Protease of this invention (as % active) | 0.05 | 0.1 | 0.02 | 0.03 | 0.03 |
| Ethoxylated Polyethylenimine¹ | 4 | 4 | 4 | 4 | 4 |
| Series 1 GG36 protease (as % active) | 0.02 | 0 | 0.01 | 0.02 | 0.03 |
| Hydroxyethane diphosphonic acid | 1.2 | 1.2 | 1.2 | 1.2 | 1.2 |
| Brightener | 0.3 | 0.3 | 0.3 | 0.3 | 0.3 |
| P-diol | 15.8 | 13.8 | 13.8 | 13.8 | 13.8 |
| Glycerol | 6.1 | 6.1 | 6.1 | 6.1 | 6.1 |
| MEA (monoethanolamide) brightener stabilizer | 8 | 8 | 8 | 8 | 8 |
| TIPA (triisopropanolamine) | - | - | 2 | - | - |
| TEA (triethanolamine) | - | 2 | - | - | - |
| Cumene sulphonate | - | - | - | - | 2 |
| cyclohexyl dimethanol | - | - | - | 2 | - |
| Water | 10 | 10 | 10 | 10 | 10 |
| Structurant | 0.14 | 0.14 | 0.14 | 0.14 | 0.14 |
| Perfume | 1.9 | 1.9 | 1.9 | 1.9 | 1.9 |
| Buffers (monoethanolamine) | To pH 8.0 | | | | |
| Solvents (1,2 propanediol, ethanol) | To 100% | | | | |
| ¹ Polyethylenimine (MW = 600) with 20 ethoxylate groups per -NH. | | | | | |

| **Multiple Compartment Unit Dose Detergent Compositions** | | |
|---|---|---|
| **Base Composition 1** | **%** | |
| **Ingredients** | | |
| Glycerol (min 99) | 5.3 | |
| 1,2-propanediol | 10 | |
| Citric Acid | 0.5 | |
| Monoethanolamine | 10 | |
| Caustic soda | - | |
| Dequest 2010 | 1.1 | |
| Potassium sulfite | 0.2 | |
| Nonionic Marlipal C24EO7 | 20.1 | |
| HLAS (surfactant) | 24.6 | |
| Optical brightener FWA49 | 0.2 | |
| C12-15 Fatty acid | 16.4 | |
| Polymer Lutensit Z96 | 2.9 | |
| Polyethyleneimine ethoxylate PEI600 E20 | 1.1 | |
| MgCl2 | 0.2 | |
| Solvents (1,2 propanediol, ethanol) | To 100% | |

| **Multi-compartment formulations** | | | | | | | |
|---|---|---|---|---|---|---|---|
| **Composition** | **1** | | | | **2** | | |
| **Compartment** | **A** | **B** | **C** | | **A** | **B** | **C** |
| Volume of each compartment | 40 ml | 5 ml | 5 ml | | 40 ml | 5 ml | 5 ml |
| Active material in Wt.% | | | | | | | |
| Perfume | 1.6 | 1.6 | 1.6 | | 1.6 | 1.6 | 1.6 |
| Dyes | < 0.01 | < 0.01 | < 0.01 | | < 0.01 | < 0.01 | < 0.01 |
| TiO2 | 0.1 | - | - | | - | 0.1 | - |
| Sodium Sulfite | 0.4 | 0.4 | 0.4 | | 0.3 | 0.3 | 0.3 |
| Acusol 305, Rohm&Haas | 1.2 | | | | 2 | - | - |
| Hydrogenated castor oil | 0.14 | 0.14 | 0.14 | | 0.14 | 0.14 | 0.14 |
| **Base Composition 1** | Add to 100% | Add to 100% | Add to 100% | | Add to 100% | Add to 100% | Add to 100% |

| **Phosphate-Free Detergent: IEC-60436 WFK Type B (pH=10.4 in 3g/l)** | |
|---|---|
| **Component** | **Wt %** |
| Sodium citrate dehydrate | 30 |
| Maleic acid/ Acrylic acid copolymer sodium Salt SOKALAN^{®} CP5 BASF | 12 |
| Sodium perborate monohydrate | 5 |
| TAED | 2 |
| Sodium disilicate: Protil A (Cognis) | 25 |
| Linear fatty alcohol ethoxylate | 2 |
| Sodium carbonate anhydrous | add to 100 |

| **Phosphate-Containing Detergent: IEC-60436 WFK Type C (pH=10.5 in 3 g/l)** | |
|---|---|
| **Component** | **Wt %** |
| Sodium tripolyphosphate | 23 |
| Sodium citrate dehydrate | 22.3 |
| Maleic acid/ Acrylic acid copolymer sodium salt | 4 |
| Sodium perborate monohydrate | 6 |
| TAED | 2 |
| Sodium disilicate: Protil A (Cognis) | 5 |
| Linear fatty alcohol ethoxylate | 2 |
| Sodium carbonate anhydrous | add to 100 |

| L**iquid laundry detergent compositions suitable for top-loading automatic washing machines (1 &2) and front loading washing machines (3).** | | | |
|---|---|---|---|
| **Ingredient** | **Composition** | | |
| | **(wt% of composition)** | | |
| | **1** | **2** | **3** |
| C₁₂₋₁₅ Alkylethoxy(1.8)sulfate | 14.7 | 11.6 | |
| C_{11.8} Alkylbenzene sulfonate | 4.3 | 11.6 | 8.3 |
| C₁₆₋₁₇ Branched alkyl sulfate | 1.7 | 1.29 | |
| C₁₂₋₁₄ Alkyl -9-ethoxylate | 0.9 | 1.07 | |
| C₁₂ dimethylamine oxide | 0.6 | 0.64 | |
| Citric acid | 3.5 | 0.65 | 3 |
| C₁₂₋₁₈ fatty acid | 1.5 | 2.32 | 3.6 |
| Sodium Borate (Borax) | 2.5 | 2.46 | 1.2 |
| Sodium C₁₂₋₁₄ alkyl ethoxy 3 sulfate | | | 2.9 |
| C₁₄₋₁₅ alkyl 7-ethoxylate | | | 4.2 |
| C₁₂₋₁₄ Alkyl -7-ethoxylate | | | 1.7 |
| Ca formate | 0.09 | 0.09 | |
| A compound having the following general structure: bis((C₂H₅O)(C₂H₄O)n)(CH₃)-N⁺-CₓH₂ₓ-N⁺-(CH₃)-bis((C₂H₅O)(C₂H₄O)n), wherein n = from 20 to 30, and x = from 3 to 8, or sulphated or sulphonated variants thereof | | | 1.2 |
| Random graft co-polymer¹ | | 1.46 | 0.5 |
| Ethoxylated Polyethylenimine ² | 1.5 | 1.29 | |
| Diethylene triamine pentaacetic acid | 0.34 | 0.64 | |
| Diethylene triamine penta(methylene phosphonic acid) | | | 0.3 |
| Tinopal AMS-GX | | 0.06 | |
| Tinopal CBS-X | 0.2 | 0.17 | |
| Amphiphilic alkoxylated grease cleaning polymer ³ | 1.28 | 1 | 0.4 |
| Ethanol | 2 | 1.58 | 1.6 |
| Propylene Glycol | 3.9 | 3.59 | 1.3 |
| Diethylene glycol | 1.05 | 1.54 | |
| Polyethylene glycol | 0.06 | 0.04 | |
| Monoethanolamine | 3.05 | 2.41 | 0.4 |
| NaOH | 2.44 | 1.8 | |
| Sodium Cumene Sulphonate | | | 1 |
| Sodium Formate | | 0.11 | |
| Water, Aesthetics (Dyes, perfumes) and Minors (Enzymes, solvents, structurants) | balance | balance | balance |
| ¹ Random graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units. | | | |
| ² Polyethylenimine (MW = 600) with 20 ethoxylate groups per -NH. | | | |
| ³ Amphiphilic alkoxylated grease cleaning polymer is a polyethylenimine (MW = 600) with 24 ethoxylate groups per -NH and 16 propoxylate groups per -NH | | | |

| **Granular laundry detergent compositions suitable for top-loading automatic washing machines (1-3) and front loading washing machines (4-5). The protease of this invention is separately added to these formulations.** | | | | | |
|---|---|---|---|---|---|
| **Ingredients** | **1** | **2** | **3** | **4** | **5** |
| C₁₆₋₁₇ Branched alkyl sulfate | 3.55 | | | | |
| C₁₂₋₁₄ alkyl sulphate | | | 1.5 | | |
| Sodium linear alkylbenzenesulfonate with aliphatic chain length C₁₁-C₁₂ | 9.6 | 15.8 | 10.6 | 7.5 | 9 |
| Sodium C_{14/15} alcohol ethoxy - 3 - sulfate | 1.15 | | | 2.88 | |
| Sodium C_{14/15} alkyl sulphate | 2.37 | | | | |
| C_{14/15} alcohol ethoxylate with average 7 moles of ethoxylation | | | | 1.17 | 1 |
| mono-C₈₋₁₀ alkyl mono-hydroxyethyl di-methyl quaternary ammonium chloride | | | | | 0.45 |
| Di methyl hydroxyl ethyl lauryl ammonium chloride | | | 0.18 | | |
| Zeolite A | 13.9 | 4.7 | 0.01 | 2.9 | 1.8 |
| Sodium Silicate 1.6.ratio | 4 | 0.2 | | 4 | 4 |
| Sodium Silicate 2.35.ratio | | | 8 | | |
| Citric Acid | | | | 2.5 | 1.4 |
| Sodium tripolyphosphate | | | 5 | | |
| Sodium Carbonate | 24.1 | 30 | 16.9 | 24.4 | 21 |
| Nonanoyloxybenzenesuplhonate | 5.78 | 2.81 | 0.96 | | |
| Oxaziridinium-based bleach booster | | | | 0.03 | 0.017 |
| Tetrasodium S,S,-ethylenediaminedisuccinate | | | | 0.2 | |
| Diethylenetriamine penta (methylene phosphonic acid), heptasodium salt | 0.61 | | | | 0.33 |
| Hydroxyethane dimethylene phosphonic acid | | | | 0.29 | 0.45 |
| Ethylene diamine tetraacetate | | | 0.27 | | |
| MgSO4 | | | 0.47 | 0.5994 | 0.782 |
| Sodium Percarbonate | 7 | 4.4 | | 15.9 | 19.1 |
| Tetra Acetyl Ethylene Diamine | | | | 3.3 | 4.6 |
| Sodium Perborate Monohydrate | | | 1.2 | | |
| Carboxymethyl cellulose (e.g. Finnfix BDA ex CPKelco) | 0.1 | | 0.17 | 1.69 | 0.23 |
| Sodium Acrylic acid/maleic acid co-polymer (70/30) | 0.0236 | 3.8 | | 2 | 2.5 |
| Sodium polyacrylate (Sokalan PA30 CL) | 4 | | 0.84 | | |
| Terephthalate polymer | | | | 0.23 | |
| Polyethylene glycol/vinyl acetate random graft co polymer | | | 0.89 | 0.89 | 0.91 |
| Photobleach- zinc phthalocyanine tetrasulfonate | | | 0.005 | 0.001 | 0.002 |
| C.I.Fluorescent Brightener 260 | 0.11 | 0.15 | 0.04 | 0.23 | 0.15 |
| C.I.Fluorescent Brightener 351 (Tinopal ^{®} CBS) | | | 0.1 | | |
| Suds suppressor granule | | 0.25 | | 0.07 | 0.04 |
| Hyrdophobically modified carboxy methyl cellulose (Finnifix ^{®} SH-1) | | | 0.019 | 0.028 | |
| Bentonite | | | 8.35 | | |
| Miscellaneous (Dyes, perfumes, process aids, moisture and sodium sulphate) | Balance | Balance | Balance | Balance | Balance |

| **Granular Laundry Detergent Compositions and Their Components. The protease of this invention is separately added to these formulations.** | | | | | | | | | | | | | | |
|---|---|---|---|---|---|---|---|---|---|---|---|---|---|---|
| | **Detergent Composition** | | | | | | | | | | | | | |
| **Component Surfactants** | **A** | **B** | **C** | **D** | **E** | **F** | **G** | **H** | **I** | **J** | **K** | **L** | **M** | **N** |
| C₁₀ Nonionic | | | | 0.1843 | | 0.1142 | 0.2894 | 0.1885 | 0.1846 | 0.1885 | 0.1979 | 0.1979 | 0.1979 | 0.1979 |
| C₁₆₋₁₇ Branched alkyl sulfate | 3.53 | 3.53 | 3.53 | | | | | | | | | | | |
| C₁₂₋₁₄ alkyl sulphate | | | | | | | | | | | | | | |
| Sodium linear alkylbenzenesulfonate with aliphatic chain length C₁₁-C₁₂ | 8.98 | 8.98 | 8.98 | 13.58 | 14.75 | 12.94 | 15.69 | 9.01 | 8.42 | 9.51 | 8.92 | 8.92 | 11.5 | 11.5 |
| Sodium C_{14/15} alcohol ethoxy - 3 - sulfate | 1.28 | 1.28 | 1.28 | | | | | | | | 1.62 | 1.62 | 1.125 | 1.125 |
| Sodium C_{14/15} alkyl sulphate | 2.36 | 2.36 | 2.36 | | | | | | | | | | | |
| C_{12/14} alcohol ethoxylate with average 7 moles of ethoxylation | | | | | | 2.9 | | | | | | | | |
| C_{12/14} alcohol ethoxylate with average 3 moles of ethoxylation | | | | | | | | | 2.44 | | | | | |
| C_{14/15} alcohol ethoxylate with average 7 moles of ethoxylation | | | | | | | | 0.97 | 1.17 | 0.97 | 1 | 1 | 1.5 | 1.5 |
| mono-C₈₋₁₀ alkyl mono-hydroxyethyl di-methyl quaternary ammonium chloride | | | | | | | | 0.45 | | | | | | |
| Di methyl hydroxyl ethyl lauryl ammonium chloride | | | | 0.1803 | | | 0.195 | | | 0.45 | | | | |
| Zeolite A | 15.31 | 15.31 | 15.31 | | 4.47 | 2.01 | 0.39 | 1.83 | 2.58 | 0.59 | 1.63 | 1.63 | 2 | 2 |
| Bentonite | | | | 8.35 | | | | | | | | | | |
| Sodium Silicate 1.6.ratio | | | | | 0.16 | | | 4.53 | 5.62 | 4.53 | 4.75 | 4.75 | 4.75 | 4.75 |
| Sodium Silicate 2.0.ratio | 3.72 | 3.72 | 3.72 | 8.41 | | | 10.1 | | | | | | 0.06 | 0.06 |
| Sodium Silicate 2.35.ratio | | | | | | 7.05 | | | | | | | | |
| Citric Acid | | | | 0.0066 | | | | 1.4 | 1.84 | 1 | 1.1 | 1.1 | 1.1 | 1.1 |
| Sodium tripolyphosphate | | | | 5.06 | | | 5.73 | | | | | | | |
| Sodium Carbonate | 26.1 | 26.18 | 26.1 | 15.9 | 29 | 12.65 | 15.93 | 21 | 27.31 | 20.2 | 23.3 | 23.3 | 23.3 | 23.3 |
| Nonanoyl oxybenzene suplhonate | 5.78 | 5.78 | 5.78 | 1.17 | 1.86 | | 1.73 | | | | | | | |
| Oxaziridinium-based bleach booster | 0.037 | 0.037 | 0.037 | | | | | 0.0168 | 0.0333 | 0.024 | 0.021 | 0.021 | 0.015 | 0.015 |
| Tetrasodium S,S,-ethylene diaminedisuccinate | | | | | | | | | | | 0.26 | 0.26 | 0.26 | 0.26 |
| Diethylenetriamine penta (methylene phosphonic acid), heptasodium salt | 0.62 | 0.62 | 0.62 | | | | | 0.327 | | 0.3272 | | | | |
| Hydroxyethane dimethylene phosphonic acid | | | | | | | | 0.45 | 0.2911 | 0.45 | 0.47 | 0.47 | 0.47 | 0.47 |
| Ethylene diamine tetraacetate | | | | 0.2701 | | | 0.28 | | 0.1957 | | | | | |
| MgSO4 | 0.056 | 0.056 | 0.056 | 0.47 | | | 0.54 | 0.79 | 0.6494 | 0.793 | 0.83 | 0.83 | 0.82 | 0.82 |
| Sodium Percarbonate | | 7.06 | 7.06 | | 3.64 | | | 19.1 | 15.85 | 22.5 | 19.35 | 19.35 | 19.35 | 19.35 |
| Tetra Acetyl Ethylene Diamine | | | | | | | | 4.554 | 3.71 | 5.24 | 4.51 | 4.51 | 4.51 | 4.51 |
| Sodium Perborate Monohydrate | | | | 1.47 | | | 5.55 | | | | | | | |
| Carboxymethyl cellulose (e.g. Finnfix BDA ex CPKelco) | 0.38 | 0.38 | 0.38 | 0.173 | | 0.62 | 0.21 | 0.23 | 1.07 | 0.2622 | 1.01 | 1.01 | 1.01 | 1.01 |
| Sodium Acrylic acid/maleic acid copolymer (70/30) | 3.79 | 3.78 | 3.79 | | 3.64 | 0.4 | 2.61 | 2.5 | 2 | 1.75 | 1.84 | 1.84 | 1.84 | 1.84 |
| Sodium polyacrylate (SokalanPA30 CL) | 3.78 | 3.78 | 3.78 | 0.842 | | | | 0.0055 | 0.011 | 0.008 | 0.007 | 0.007 | 0.005 | 0.005 |
| Terephthalate polymer | | | | | | | | | 0.231 | | 0.179 | 0.179 | 0.179 | 0.179 |
| Polyethylene glycol/vinyl acetate random graft co polymer | | | | 0.89 | | 0.55 | 1.4 | 0.911 | 0.8924 | 0.911 | 0.96 | 0.96 | 0.96 | 0.96 |
| Photobleach- zinc phthalocyanine tetrasulfonate | | | | | | | | | | | | | | |
| C.I.Fluorescent Brightener 260 | 0.1125 | 0.1125 | 0.1125 | 0.043 | 0.15 | 0.1174 | 0.048 | 0.1455 | 0.2252 | 0.1455 | 0.153 | 0.153 | 0.171 | 0.171 |
| C.I.Fluorescent Brightener 351 (Tinopal ^{®} CBS) | | | | 0.0952 | | | 0.1049 | | | | | | | |
| Suds suppressor granule | 0.015 | 0.015 | 0.015 | | 0.031 | | | 0.04 | 0.0658 | 0.04 | 0.042 | 0.042 | 0.042 | 0.042 |
| Hydrophobically modified carboxy methyl cellulose (Finnifix ^{®} SH-1) | | | | | | | | | | | | | | |
| Bentonite | | | | | | | | | | | | | | |
| Miscellaneous (Dyes, perfumes, process aids, moisture and sodium sulphate) | Balance | Balance | Balance | Balance | Balance | Balance | Balance | Balance | Balance | Balance | Balance | Balance | Balance | Balance |

| **Dishwashing Detergent Gel Compositions** | | | | | |
|---|---|---|---|---|---|
| **Ingredients** | **1** | **2** | **3** | **4** | **5** |
| | (wt%) | (wt%) | (wt%) | (wt%) | (wt%) |
| Polytergent ^{®} SLF-18 | 1 | 1.3 | 0.8 | 1 | 0.9 |
| Sodium Benzoate (33% active) | 0.61 | 0.61 | 0.61 | 0.6 | 0.6 |
| Xanthan gum | 1 | 0.8 | 1.2 | 1 | 1.1 |
| Sodium Sulphate | 10 | 10 | 10 | 8 | 10 |
| Perfume | 0.03 | 0.05 | 0.03 | 0.06 | 0.1 |
| Sodium Silicate | | | | | 2 |
| Citric Acid (50% active) | 12.5 | | 12 | | |
| GLDA | | 7 | | 8 | |
| Protease 1 (44 mg active/g | 0.7 | | 0.3 | | |
| 4-Formyl-Phenyl BoronicAcid | | | 0.05 | | |
| Protease 2 (10 mg/g) encapsulated | | 2 | | 0.6 | |
| Protease 3 (48 mg active/g) | | | | | 0.5 |
| Protease 4 (123 mg active/g) | | | | | |
| Ethanol | | | | 0.3 | |
| Potassium Hydroxide (45% active) | 14.6 | 14.6 | 14.6 | 14 | |
| Calcium Chloride (25% active) | 1.8 | 1.8 | 1.8 | 1.1 | 0.4 |
| Dye | 0.05 | 0.05 | 0.05 | 0.05 | 0.02 |
| Proxcel GXL ^{™} (19% active) | 0.05 | 0.05 | 0.05 | 0.05 | 0.05 |
| Acusol ^{™} 8209 | 0.34 | 0.34 | 0.3 | 0.35 | 0.3 |
| Acusol ^{™} 425N (50% active) | 3 | 3 | 3.5 | 2.5 | 2 |
| Amylases (25 mg/g active) | 0.2 | 0.5 | 0.4 | 0.3 | 0.1 |
| Water & other adjunct ingredients | Balance to 100% | Balance to 100% | Balance to 100% | Balance to 100% | Balance to 100% |

| **Powder Automatic Dishwashing Compositions** | |
|---|---|
| **Composition 1** | |
| **Ingredients** | **Wt%** |
| Nonionic surfactant | 0.4-2.5% |
| Sodium metasilicate | 0-20% |
| Sodium disilicate | 0-20% |
| Sodium triphosphate | 0-40% |
| Sodium carbonate | 0-20% |
| Sodium perborate | 2-9% |
| Tetraacetyl ethylene diamine (TAED) | 1-4% |
| Sodium sulfate | 5-33% |
| Enzymes | 0.0001-0.1% |

| **Composition** 2 | |
|---|---|
| **Ingredients** | **Wt%** |
| Nonionic surfactant (e.g. alcohol ethoxylate) | 1-2% |
| Sodium disilicate | 2-30% |
| Sodium carbonate | 10-50% |
| Sodium phosphonate | 0-5% |
| Trisodium citrate dehydrate | 9-30% |
| Nitrilotrisodium acetate (NTA) | 0-20% |
| Sodium perborate monohvdrate | 5-10% |
| Tetraacetvl ethylene diamine (TAED) | 1-2% |
| Polyacrylate polymer (e.g. maleic acid/acrylic acid copolymer) | 6-25% |
| Enzymes | 0.0001-0.1% |
| Perfume | 0.1-0.5% |
| Water | 5--10 |

| **Composition 3** | |
|---|---|
| **Ingredients** | **Wt%** |
| Nonionic surfactant | 0.5-2.0% |
| Sodium disilicate | 25-40% |
| Sodium citrate | 30-55% |
| Sodium carbonate | 0-29% |
| Sodium bicarbonate | 0-20% |
| Sodium perborate monohydrate | 0-15% |
| Tetraacetyl ethylene diamine (TAED) | 0-6% |
| Maleic acid/acrylic acid copolymer | 0-5% |
| Clay | 1-3% |
| Polyamino acids | 0-20% |
| Sodium polyacrylate | 0-8% |
| Enzymes | 0.0001-0.1 % |

| **Powder Automatic Dishwashing Compositions** | |
|---|---|
| **Composition 4** | |
| **Ingredients** | **Wt%** |
| Nonionic surfactant | 1-2% |
| Zeolite MAP | 0-42% |
| Sodium disilicate | 0-34% |
| Sodium citrate | 0-12% |
| Sodium carbonate | 0-20% |
| Sodium perborate monohydrate | 7-15% |
| Tetraacetyl ethylene diamine (TAED) | 0-3% |
| Polymer | 0-4% |
| Maleic acid/acrylic acid copolymer | 0-5% |
| Organic phosphonate | 0-4% |
| Clay | 1-2% |
| Enzymes | 0.0001-0.1% |
| Sodium sulfate | Balance |

| **Composition 5** | |
|---|---|
| **Ingredients** | **Wt%** |
| Nonionic surfactant | 1-7% |
| Sodium disilicate | 18-30% |
| Trisodium citrate | 10-24% |
| Sodium carbonate | 12-20% |
| Monopersulfate (2 KHSOsoKHS04 °K2S04 ) | 15-21% |
| Bleach stabilizer | 0.1-2% |
| Maleic acid/acrylic acid copolymer | 0-6% |
| Diethylene triarnine pentaacetate, pentasodium salt | 0-2.5% |
| Enzymes | 0.0001-0.1% |
| Sodium sulfate, water | Balance |

| **Powder and Liquid Dishwashing Composition with Cleaning Surfactant System** | |
|---|---|
| **Ingredients** | **Wt%** |
| Nonionic surfactant | 0-1.5% |
| Octadecyl dimethylamine N-oxide dihydrate | 0-5% |
| 80:20 wt C18/C16 blend of octadecyl dimethylamine N-oxide dihydrate and hexadecyldimethyl amine Noxide dehydrate | 0-4% |
| 70:30 wt C18/C16 blend ofoctadecyl bis (hydroxyethyl)amine N-oxide anhydrous and hexadecyl bis (hydroxyethyl)amine N-oxide anhydrous | 0-5% |
| C13-C1S alkyl ethoxysulfate with an average degree of ethoxylation of 3 | 0-10% |
| C12-C1S alkyl ethoxysulfate with an average degree of ethoxylation of 3 | 0-5% |
| C13-CIS ethoxylated alcohol with an average degree of ethoxylation of 12 | 0-5% |
| A blend of C 12-C IS ethoxylated alcohols with an average degree of ethoxylation of 9 | 0-6.5% |
| A blend of C 13-C IS ethoxylated alcohols with an average degree of ethoxylation of 30 | 0-4% |
| Sodium disilicate | 0-33% |
| Sodium tripolyphosphate | 0-46% |
| Sodium citrate | 0-28% |
| Citric acid | 0-29% |
| Sodium carbonate | 0-20% |
| Sodium perborate monohydrate | 0-11.5% |
| Tetraacetyl ethylene diamine (TAED) | 0-4% |
| Maleic acid/acrylic acid copolymer | 0-7.5% |
| Sodium sulfate | 0-12.5% |
| Enzymes | 0.0001-0.1 % |

| **Non-Aqueous Liquid Automatic Dishwashing Composition** | |
|---|---|
| **Ingredients** | **Wt%** |
| Liquid nonionic surfactant (e.g. alcohol ethoxylates) | 2.0-10.0% |
| Alkali metal silicate | 3.0-15.0% |
| Alkali metal phosphate | 0-40.0% |
| Liquid carrier selected from higher glycols, polyglycols, polyoxides, glycol ethers | 25.0-45.0% |
| Stabilizer (e.g. a partial ester of phosphoric acid and a C16-C18 alkanol) | 0.5-7.0% |
| Foam suppressor (e.g. silicone) | 0-1.5% |
| Enzymes | 0.0001-0.1 % |

| **Non-Aqueous Liquid Dishwashing Composition** | |
|---|---|
| **Ingredients** | **Wt%** |
| Liquid nonionic surfactant (e.g. alcohol ethoxylates) | 2.0-10.0% |
| Sodium silicate | 3.0-15.0% |
| Alkali metal carbonate | 7.0-20.0% |
| Sodium citrate | 0.0-1.5% |
| Stabilizing system (e.g. mixtures of finely divided silicone and low molecular weight dialkyl polyglycol ethers) | 0.5-7.0% |
| Low molecule weight polyacrylate polymer | 5.0-15.0% |
| Clay gel thickener (e.g. bentonite) | 0.0-10.0% |
| Hydroxypropyl cellulose polymer | 0.0-0.6% |
| Enzymes | 0.0001-0.1 % |
| Liquid carrier selected from higher lycols, polyglycols, polyoxides and glycol ethers | Balance |

| **Thixotropic Liquid Automatic Dishwashing Composition** | |
|---|---|
| **Ingredients** | **Wt%** |
| C 12-C 14 fatty acid | 0-0.5% |
| Block co-polymer surfactant | 1.5-15.0% |
| Sodium citrate | 0-12% |
| Sodium tripolyphosphate | 0-15% |
| Sodium carbonate | 0-8% |
| Aluminium tristearate | 0-0.1% |
| Sodium cumene sulfonate | 0-1.7% |
| Polyacrylate thickener | 1.32-2.5% |
| Sodium polyacrylate | 2.4-6.0% |
| Boric acid | 0-4.0% |
| Sodium formate | 0-0.45% |
| Calcium formate | 0-0.2% |
| Sodium n-decydiphenyl oxide disulfonate | 0-4.0% |
| Monoethanol amine (MEA) | 0-1.86% |
| Sodium hydroxide (50%) | 1.9-9.3% |
| 1,2-Propanediol | 0-9.4% |
| Enzymes | 0.0001-0.1% |
| Suds suppressor, dye, perfumes, water | Balance |

| **Liquid Automatic Dishwashing Composition** | |
|---|---|
| **Ingredients** | **Wt%** |
| Alcohol ethoxylate | 0-20% |
| Fatty acid ester sulfonate | 0-30% |
| Sodium dodecyl sulfate | 0-20% |
| Alkyl polyglycoside | 0-21% |
| Oleic acid | 0-10% |
| Sodium disilicate monohydrate | 0-33% |
| Sodium citrate dihydrate | 0-33% |
| Sodium stearate | 0-2.5% |
| Sodium perborate monohydrate | 0-13% |
| Tetraacetyl ethylene diamine (TAED) | 0-8% |
| Maleic acid/acrylic acid copolymer | 4-8% |
| Enzymes | 0.0001-0.1% |

| **Liquid Automatic Dishwashing Composition Containing Protected Bleach Particles** | |
|---|---|
| **Ingredients** | **Wt%** |
| Sodium silicate | 5-10% |
| Tetrapotassium pyrophosphate | 0-25% |
| Sodium triphosphate | 0-2% |
| Potassium carbonate | 4-8% |
| Protected bleach particles, e.g. chlorine | 5-10% |
| Polymeric thickener | 0.7-1.5% |
| Potassium hydroxide | 0-2% |
| Enzymes | 0.0001-0.1 % |
| Water | Balance |

| | **Composition of Model Detergent A:** | | **Composition of Model Detergent B:** | |
|---|---|---|---|---|
| **Compound** | **Amount g/100 g** | **% active ingredient** | **Amount g/100 g** | **% active ingredient** |
| **Surfactants** | | | | |
| Na-LAS (92%) (NacconoI90G) (anionic) (linear alkylbenzene sulfonate) | 10.87 | 10 | 10.87 | 10 |
| STEOL CS-370E (70%) (anionic), CH3(CH2)m-(OCH2CH2)3--OS03-, where m~ 11-13 | 7.14 | 5 | 7.14 | 5 |
| Bio-soft N25-7 (99.5%) (nonionic),: CH3(CH2)m-(OCH2CH2h--OH, where and m~ 11-14 | 5 | 5 | 5 | 5 |
| Oleic acid (fatty acid) | 2 | 2 | 2 | 2 |
| **Solvents** | | | | |
| H20 | 62 | 65 | 62 | 65 |
| Ethanol | 0.5 | 0.5 | 0.5 | 0.5 |
| STS (sodium p-toluene sulfonate (40%» | 3.75 | 1.5 | 3.75 | 1.5 |
| Mono propylene glycol | 2 | 2 | 2 | 2 |
| **Builder** | | | | |
| Tri -sodium -citrate | 4 | 4 | 0 | 0 |
| Diethylene triamine penta acetic acid (DTPA) | 0 | 0 | 1.5 | 1.5 |
| Triethanolamine (TEA) | 0.5 | 0.5 | 0.5 | 0.5 |
| **Stabilizer** | | | | |
| Boric Acid | 1.5 | 1.5 | 1.5 | 1.5 |
| **Minors** | | | | |
| 10N NaOH (for adjustment to pH 8.5) | 0.8 | 0.8 | 0.8 | 0.8 |

| **Liquid Detergent and Cleaning Agent Compositions** | | | | | | | | |
|---|---|---|---|---|---|---|---|---|
| **Ingredients** | **E1** | **E2** | **E3** | **C1** | **C2** | **C3** | **C4** | **C5** |
| Gellan gum | 0.2 | 0.2 | 0.15 | 0.15 | | | | |
| Xanthan gum | | | 0.15 | | 0.15 | 0.5 | 0.2 | |
| Polyacrylate (Carbopol Aqua 30) | 0.4 | 0.4 | | | | | 0.6 | 0.6 |
| C₁₂₋₁₄-fatly alcohol with 7 EO | 22 | 10 | 10 | 10 | 10 | 10 | 10 | 10 |
| C₉₋₁₃-alkylbenzene sulfonate, Na salt | | 10 | 10 | 10 | 10 | 10 | 10 | 10 |
| C₁₂₋₁₄ -alkylpolyglycoside | 1 | | | | | | | |
| Citric acid | 1.6 | 3 | 3 | 3 | 3 | 3 | 3 | 3 |
| Dequest ^{®} 2010 Hydroxyethylidene-1,1-diphosphonic acid, tetrasodium salt (from Solutia) | 0.5 | 1 | 1 | 1 | 1 | 1 | 1 | 1 |
| Sodium lauryl ether sulfate with 2 EO | 10 | 5 | 5 | 5 | 5 | 5 | 5 | 5 |
| Monoethanolamine | 3 | 3 | 3 | 3 | 3 | 3 | 3 | 3 |
| C₁₂₋₁₈-fatty acid | 7.5 | 7.5 | 7.5 | 7.5 | 7.5 | 7.5 | 7.5 | 7.5 |
| Propylene glycol | | 6.5 | 6.5 | 6.5 | 6.5 | 6.5 | 6.5 | 6.5 |
| Sodium cumene sulfonate | | 2 | 2 | 2 | 2 | 2 | 2 | 2 |
| Enzymes, dyes, stabilizers | + | + | + | + | + | + | + | + |
| Microcapsules with about 2000 µm diameter | 0.5 | 0.5 | 0.5 | 0.5 | 0.5 | 0.5 | 0.5 | 0.5 |
| Water | To 100 | To 100 | To 100 | To 100 | To 100 | To 100 | To 100 | To 100 |
| Flow limit (Pas) | 0.58 | 1.16 | 1.16 | no | no | no | yes | no |

| **All purpose Alkaline detergent Compositions (all-purpose. glass. kitchen) Hard surface cleaning detergent composition** | | | | |
|---|---|---|---|---|
| **Composition [% by wt.]** | **E1** | **E2** | **E3** | **E4** |
| Fatty alcohol ethoxylate C12-7EO | 1 | 3 | 5 | 0.5 |
| Alkylbenzenesulfonic acid Na salt | 3 | 1 | 2 | 4 |
| Octyl sulfate | 3 | 2 | 2 | 2 |
| Sodium carbonate | 1.5 | 0.5 | 1.0 | 1.5 |
| Citric acid | 0.5 | 0.5 | 0.5 | 0.5 |
| Fatty acid | 0.5 | 0.5 | 0.5 | 1.0 |
| Ethanol | 5 | 3 | 5 | 3 |
| Perfume | 0.2 | 0.2 | 0.2 | 0.2 |
| Water | To 100 | To 100 | To 100 | To 100 |

| **Acidic Detergent Compositions (bath, toilet)** | | | | |
|---|---|---|---|---|
| **Composition [% by wt.]** | **E5** | **E6** | **E7** | **E8** |
| Fatty alcohol ether sulfate C12-2EO sodium salt | 2 | 3 | 5 | 2 |
| Ethanol | 3 | 3 | 3 | 3 |
| Citric acid | 3 | 10 | 3 | 10 |
| Thickener xanthan Kelzan ASX -T | | 0.05 | | 0.05 |
| Perfume | 0.1 | 0.1 | 0.1 | 0.1 |
| Water | To 100 | To 100 | To 100 | To 100 |

| **Cleaning Paste Composition** | |
|---|---|
| **Composition [% by wt.]** | **E9** |
| C 12 Fatty alcohol sulfate | 20 |
| C16-18 Fatty alcohol ethoxylate 25 EO | 20 |
| C 12-18 Fatty acid monoethanolamide | 10 |
| Sodium sulfate | 40 |
| Sodium carbonate | 5 |
| Cellulose | 4.899 |
| Dye | 0.001 |
| Perfume | 0.1 |

| **Self Foaming Cleaning Powder Composition** | |
|---|---|
| **Composition [% by wt.]** | **E10** |
| C 12 Fatty alcohol sulfate | 2 |
| Sodium sulfate | 37.899 |
| Sodium carbonate | 25 |
| Citric Acid | 35 |
| Dye | 0.001 |
| Perfume | 0.1 |

| **Compositions of a Clear Aqueous Detergent and Cleaning Agent having a flow limit** | | | | | | |
|---|---|---|---|---|---|---|
| **Ingredients** | **V1** | **E1** | **E2** | **E3** | **E4** | **E5** |
| 1,2 Propane diol | 8 | 0 | 2 | 6 | 4 | 2 |
| Dipropylene glycol | 0 | 8 | 6 | 2 | 4 | 2 |
| Polyacrylate (Carbopol Aqua 30) | 3 | 3 | 3 | 3 | 3 | |
| Polyacrylate (Polygel W301) | - | - | - | - | - | 1.8 |
| C₁₂₋₁₄-fatty alcohol with 7 EO | 10 | 10 | 10 | 10 | 10 | 10 |
| C₉₋₁₃-alkylbenzene sulfonate, Na salt | 10 | 10 | 10 | 10 | 10 | - |
| Citric Acid | 3 | 3 | 3 | 3 | 3 | 2 |
| Dequest ^{®} 2010 Hydroxyethylidene-1, 1-diphosphonic acid, tetrasodium salt (ex Solutia) | 1 | 1 | 1 | 1 | 1 | - |
| Dequest ^{®} 2066 Diethylene triamine penta (methylenephosphonic acid) hepta Na salt (ex Solutia) | - | - | - | - | - | 0.7 |
| Sodium lauryl ether sulfate with 2 EO | 10 | 10 | 10 | 10 | 10 | 5 |
| Monoethanolamine | 3 | 3 | 3 | 3 | 3 | 2 |
| C₁₂₋₁₈-fatty acid Na salt | 5.5 | 5.5 | 5.5 | 5.5 | 5.5 | 5.5 |
| Enzymes, dyes, stabilizers | + | + | + | + | + | + |
| Microcapsules with about 2000 µm diameter | 0.5 | 0.5 | 0.5 | 0.5 | 0.5 | 0.5 |
| Water | To 100 | To 100 | To 100 | To 100 | To 100 | To 100 |
| Flow limit (Pas) | 0.4 | 0.6 | 0.6 | 0.8 | 1.0 | 0.6 |
| Appearance | Cloudy | Clear | Clear | Clear | Clear | Clear |

| **Liquid Laundry Detergent** | |
|---|---|
| **Ingredients** | **Wt%** |
| ABS (alkyl benzene sulphonate) | 10 |
| FAEOS | 5 |
| C_{12/14} 7EO | 10 |
| C_{12/18} Fatty Acid | 5 |
| Glycerol | 5 |
| Sodium citrate | 3 |
| Protease/Amylase/Cellulase | 1 |
| Tinopal^{®} DMS-X (optical brightener manufactured by Ciba) | 0.2 |
| Water | To 100 |

| **Granular Laundry Detergent** | |
|---|---|
| **Ingredients** | **Wt%** |
| ABS (alkyl benzenesulphonate) | 11 |
| C_{13/15} 7EO | 3 |
| Sodium carbonate | 20 |
| Sodium hydrogencarbonate | 5 |
| Sodium sulphate | 25 |
| Sodium silicate | 5 |
| Sodium percarbonate | 13 |
| TAED | 5 |
| Sodium polyacrylate | 4.5 |
| Enzymes (protease, amylase, and cellulose) | 3.5 |
| Water | To 100 |

| **Aqueous Liquid Washing Product Formulations (without-FWM1 and with-FWM2 0.5% hyperbranched polyesteramide** | | |
|---|---|---|
| **Formulation** | **FWM1** | **FWM2** |
| C₁₂₋₁₄-fatty alcohol with 2 EO | 5 | 5 |
| LAS | 10 | 10 |
| C₁₂₋₁₈-fatty alcohol with 7 EO | 10 | 10 |
| C₁₂₋₁₈ soap | 8 | 8 |
| Citrate | 4 | 4 |
| 1,2-propanediol | 5 | 5 |
| Hybrane^{®} SIP 2100 (manufactured by DSM) | | 0.5 |

| **Liquid Laundry Detergent Compositions** | | | |
|---|---|---|---|
| **Detergent Composition** | **Wt%** | | |
| | E1 | E2 | E3 |
| C_{12- 14} fatty alcohol with 7 EO | 5 | 4 | 10 |
| C_{9 -13} alkylbenzene sulfonate, Na salt | 10 | 10 | 10 |
| Sodium lauryl ether sulfate with 2 EO | - | - | 8 |
| Active substance (specific polycarbonate-, polyurethane-, and/or polyureapolyorganosiloxane compounds or precursor compounds thereof of the reactive cyclic carbonate and urea type | 1 | 1 | 1 |
| Polyacrylate thickener | - | - | 1 |
| Sodium percarbonate | 15 | 18 | - |
| TAED | 3 | 3 | - |
| C_{12- 18} fatty acid, Na salt | 1 | 1.5 | 7.5 |
| PVA/Maleic acid copolymer | 4.5 | 2 | - |
| Citric acid, Na salt | 2.5 | - | 2 |
| Phosphonic acid, Na salt | 0.5 | 0.5 | 1 |
| Sodium carbonate | 10 | 20 | - |
| Propane diol | - | - | 6.5 |
| Zeolite A | 25 | 25 | - |
| Boric Acid Sodium salt | - | - | 1.2 |
| Silicone defoamer | 2.5 | 1.3 | 0.1 |
| Enzymes (protease, amylase, cellulase) | + | + | + |
| Colorant | + | + | + |
| Perfume | 0.5 | 0.2 | 0.8 |
| Water | - | - | To 100 |
| Sodium sulfate | - | To 100 | - |
| Sodium bicarbonate | To 100 | - | - |

| **Example formulations of preferred phosphate-free automatic dishwashing agents** | | | | |
|---|---|---|---|---|
| **Ingredient** | **Formulation 1 (wt%)** | **Formulation 2 (wt%)** | **Formulation 3 (wt%)** | **Formulation 4 (wt%)** |
| Citrate | 5 to 60 | 10 to 55 | 15 to 50 | 15 to 50 |
| Sodium percarbonate | 1 to 20 | 2 to 15 | 4 to 10 | 4 to 10 |
| Bleach catalyst | 0.01 to 3 | 0.02 to 2 | 0.02 to 2 | 0.02 to 1 |
| Copolymer¹ | 0.1 to 30 | 0.5 to 25 | 1.0 to 20 | 1.0 to 20 |
| Nonionic surfactant² | 1 to 10 | 2 to 8 | 2 to 8 | 3 to 6 |
| Misc | To 100 | To 100 | To 100 | To 100 |

| **Ingredient** | **Formulation 5 (wt%)** | **Formulation 6 (wt%)** | **Formulation 7 (wt%)** | **Formulation 8 (wt%)** |
|---|---|---|---|---|
| Citrate | 5 to 60 | 10 to 55 | 15 to 50 | 15 to 50 |
| Sodium percarbonate | 1 to 20 | 2 to 15 | 4 to 10 | 4 to 10 |
| Phosphonate | 2 to 8 | 2 to 8 | 2 to 8 | 2 to 8 |
| Copolymer¹ | 0.1 to 30 | 0.5 to 25 | 1.0 to 20 | 1.0 to 20 |
| Nonionic surfactant² | 1 to 10 | 2 to 8 | 2 to 8 | 3 to 6 |
| Misc | To 100 | To 100 | To 100 | To 100 |

| **Example formulations of preferred phosphate-free automatic dishwashing agents** | | | | |
|---|---|---|---|---|
| **Ingredient** | **Formulation 9 (wt%)** | **Formulation 10 (wt%)** | **Formulation 11 (wt%)** | **Formulation 12 (wt%)** |
| Citrate | 5 to 60 | 10 to 55 | 15 to 50 | 15 to 50 |
| Sodium percarbonate | 1 to 20 | 2 to 15 | 4 to 10 | 4 to 10 |
| Enzyme | 0.1 to 6 | 0.2 to 5 | 0.4 to 5 | 0.4 to 5 |
| Copolymer¹ | 0.1 to 30 | 0.5 to 25 | 1.0 to 20 | 1.0 to 20 |
| Nonionic surfactant² | 1 to 10 | 2 to 8 | 2 to 8 | 3 to 6 |
| Misc | To 100 | To 100 | To 100 | To 100 |

| **Example formulations of preferred phosphate-free automatic dishwashing agents** | | | | |
|---|---|---|---|---|
| **Ingredient** | **Formulation 13 (wt%)** | **Formulation 14 (wt%)** | **Formulation 15 (wt%)** | **Formulation 16 (wt%)** |
| Citrate | 5 to 60 | 10 to 55 | 15 to 50 | 15 to 50 |
| Carbonate/hydrogen carbonate | 2 to 40 | 2 to 40 | 2 to 40 | 2 to 40 |
| Silicate | 0 to 15 | 0 to 15 | 0 to 15 | 0.1 to 10 |
| Phosphonate | 0 to 14 | 0 to 14 | 0 to 14 | 2 to 8 |
| Sodium percarbonate | 1 to 20 | 2 to 15 | 4 to 10 | 4 to 10 |
| Bleach catalyst | 0.01 to 3 | 0.02 to 2 | 0.02 to 2 | 0.02 to 1 |
| Copolymer¹ | 0.1 to 30 | 0.5 to 25 | 1.0 to 20 | 1.0 to 20 |
| Nonionic surfactant² | 1 to 10 | 2 to 8 | 2 to 8 | 3 to 6 |
| Enzyme | 0.1 to 6 | 0.2 to 5 | 0.4 to 5 | 0.4 to 5 |
| Misc | To 100 | To 100 | To 100 | To 100 |

### ¹Copolymer comprising

i) monomers from the group of mono- or polyunsaturated carboxylic acids
ii) monomers of the general formula R¹(R²)C=C(R³)-X-R⁴, in which R¹ to R³ mutually independently denote -H, -CH₃ or -C₂H₅, X denotes an optionally present spacer group which is selected from -CH₂-,-C(O)O- and -C(O)-NH-, and R⁴ denotes a straight chain or branched saturated alkyl residue with 2 to 22 carbon atoms or denotes an unsaturated, preferably aromatic residue with 6 to 22 carbon atoms
iii) optionally further monomers
   ²Nonionic surfactant of the general formula R¹-CH(OH)CH₂0-(AO)w-(A'0)ₓ-(A"0)_{y}-(A‴0)_{z}-R₂, in which R¹ denotes a straight-chain or branched, saturated or mono- or polyunsaturated C6 -24 alkyl or alkenyl residue; R² denotes a linear or branched hydrocarbon residue with 2 to 26 carbon atoms; A, A', A" and A‴ mutually independently denote a residue from the group comprising ---CH₂CH₂, -CH₂CH₂---CH₂,-CH₂CH₂--CH(CH₃), CH₂-CH₂-CH₂CH₂, -CH₂-CH-(CH₃)-CH₂-, -CH₂-CH(CH₂-CH₃), w, x, y and z denote values between 0.5 and 120, wherein x, y and/or z may also be 0.

| **Composition of phosphate-free automatic dishwashing detergents** | | |
|---|---|---|
| **Raw material** | **V1** | **E1** |
| Citrate | 23 | 23 |
| MGDA | 8 | 8 |
| Copolymer¹ | 12 | 12 |
| HEDP | 2 | 2 |
| Soda | 28 | 28 |
| Sodium percarbonate | 10 | 10 |
| TAED | 2.4 | 2.4 |
| Protease | 2 | 2 |
| Amylase | 1.8 | 1.8 |
| Non-ionic surfactant² | 5 | - |
| Non-ionic surfactant³ | - | 5 |
| Misc | To 100 | To 100 |

| **Textile Washing Agent** | |
|---|---|
| **Ingredient** | **wt % pure substance** |
| Xanthan | 0.3-0.5 |
| Anti foaming agent | 0.2-0.4 |
| Glycerol | 6-7 |
| Ethanol | 0.3-0.5 |
| FAEOS | 4-7 |
| Non ionic surfactant (FAEO, APG among others) | 24-28 |
| Boric acid | 1 |
| Sodium citrate dihydrate | 1-2 |
| Soda | 2-4 |
| Coconut fatty acids | 14-16 |
| HEDP | 0.5 |
| PVP | 0-0.4 |
| Optical brightener | 0-0.05 |
| Dye | 0-0.001 |
| Perfume | 0-2 |
| Water demineralized | remainder |

| **Example detergent compositions for application to a substrate** | | | | | |
|---|---|---|---|---|---|
| | **Weight Percent (actives %)** | | | | |
| **Ingredients** | **D1** | **D2** | **D3** | **D4** | **D5** |
| Sodium dodecyl benzene sulfonate | 26.09 | 17.30 | 15.60 | 17.70 | 16.70 |
| Sodium alkyl C₁₄₋₁₅/ 7EO ether sulfate | 13.80 | - | - | - | - |
| Linear alcohol ethoxylate C₁₄₋₁₅/ 7EO | 13.44 | 5.4 | 14.6 | 5.5 | 5.2 |
| Polyethylene glycol PEG 75 | 2 | 1.4 | 1.3 | 1.4 | 1.4 |
| Polyoxyethylene (100) stearyl ether | 21.99 | 15.6 | 14.1 | 15.9 | 15.1 |
| Sodium silicate SiO₂/Na₂O ratio 1.6-1.8 | 3.72 | 16.6 | 15 | 17 | 16 |
| Sodium Silicate (Britesil ^{®} C24) | 7 | - | - | - | - |
| Sodium Carbonate | - | 6.5 | 5.9 | 6.7 | 6.3 |
| Sodium tetraborate decahydrate | - | 11.9 | 10.8 | 12.2 | 11.5 |
| Sodium polyacrylate ~4500 MW | - | 1.8 | 1.7 | - | 5.2 |
| EDTA-tetrasodium salt | - | 0.1 | 0.1 | 0.1 | 0.1 |
| Optical brightener (Tinopal ^{®} CBS-X) | 0.15 | 0.1 | 0.09 | 0.1 | 0.1 |
| Dyes and fragrances | 0.9 | 0.9 | 0.81 | 1.01 | 0.91 |
| Water | 10.92 | 22.10 | 19.90 | 22.4 | 21.5 |

| **Example fabric conditioning compositions for application to a substrate** | | | | | |
|---|---|---|---|---|---|
| | **Weight Percent (actives %)** | | | | |
| **Ingredients** | **FS1** | **FS2** | **FS3** | **FS4** | **FS5** |
| Di-(hydrogenated tallow) dimethyl ammonium methyl sulfate | 33.6 | 33.2 | 44.4 | 22.2 | 33.2 |
| Unsaturated trialkylglycerides | 16.8 | 16.6 | 22.2 | 11.1 | 16.6 |
| Hydrogenated tallow fatty acid | 16.8 | 16.6 | 22.2 | 11.1 | 16.6 |
| C₁₂₋₁₈ coco fatty acid | 11.2 | 11.1 | - | 11.1 | - |
| C₁₂₋₁₈ fatty alcohol ethoxylate (7EO) | 11.2 | 11.1 | - | - | 16.6 |
| Fragrance oil | 10.4 | 11.4 | 11.2 | 11.2 | 17 |
| | | | | | |

| **Exemplary Automatic Dishwashing Agents** | | | | |
|---|---|---|---|---|
| **Ingredient** | **Wt %** | | | |
| | Formula 1 | Formula 2 | Formula 3 | Formula 4 |
| Citrate | 12-50 | 15-40 | 12-50 | 15-40 |
| Dicarboxylic acid | 1-18 | 1-18 | 2-16 | 4-12 |
| Phosphate | - | - | - | - |
| Bleaching Agent | - | - | - | - |
| Misc | To 100 | To 100 | To 100 | To 100 |

| **Additional Exemplary Automatic Dishwashing Agents** | | | | |
|---|---|---|---|---|
| **Ingredient** | **Wt %** | | | |
| | Formula 1 | Formula 2 | Formula 3 | Formula 4 |
| Citrate | 12-50 | 15-40 | 12-50 | 15-40 |
| Dicarboxylic acid | 1-18 | 1-18 | 2-16 | 4-16 |
| Carbonate | 5-50 | 10-40 | 5-50 | 10-40 |
| Phosphate | - | - | - | - |
| Bleaching Agent | - | - | - | - |
| Misc | To 100 | To 100 | To 100 | To 100 |

| **Additional Exemplary Automatic Dishwashing Agents** | | | | |
|---|---|---|---|---|
| **Ingredient** | **Wt %** | | | |
| | Formula 1 | Formula 2 | Formula 3 | Formula 4 |
| Citrate | 12-50 | 15-40 | 12-50 | 15-40 |
| Dicarboxylic acid | 1-18 | 1-18 | 2-16 | 4-12 |
| Carbonate | 5-50 | 10-30 | 5-50 | 10-30 |
| Phosphonate | 1-8 | 1-8 | 1.2-6 | 1.2-6 |
| Phosphate | - | - | - | - |
| Bleaching Agent | - | - | - | - |
| Misc | To 100 | To 100 | To 100 | To 100 |

| **Preferred Automatic Dishwashing Agents** | | | | |
|---|---|---|---|---|
| **Ingredient** | **Wt %** | | | |
| | Formula 1 | Formula 2 | Formula 3 | Formula 4 |
| Citrate | 12-50 | 15-40 | 12-50 | 15-40 |
| Dicarboxylic acid | 1-18 | 1-18 | 2-16 | 4-12 |
| Carbonate | 0-50 | 0-30 | 0-30 | 0-30 |
| Phosphonate | 0-8 | 0-8 | 0-8 | 0-8 |
| Phosphate | - | - | - | - |
| Bleaching Agent | - | - | - | - |
| Misc | To 100 | To 100 | To 100 | To 100 |

| **Additional Preferred Automatic Dishwashing Agents** | | | | |
|---|---|---|---|---|
| **Ingredient** | **Wt %** | | | |
| | Formula 1 | Formula 2 | Formula 3 | Formula 4 |
| Citrate | 12-50 | 15-40 | 12-50 | 15-40 |
| Maleic acid | 1-18 | 1-18 | 2-16 | 4-12 |
| Carbonate | 5-50 | 10-30 | 5-50 | 10-30 |
| Phosphonate | 1-8 | 1-8 | 1.2-6 | 1.2-6 |
| Phosphate | - | - | - | - |
| Bleaching Agent | - | - | - | - |
| Misc | To 100 | To 100 | To 100 | To 100 |

| **Preferred Automatic Dishwashing Agents** | | | | |
|---|---|---|---|---|
| **Ingredient** | **Wt %** | | | |
| | Formula 1 | Formula 2 | Formula 3 | Formula 4 |
| Citrate | 12-50 | 15-40 | 12-50 | 15-40 |
| Dicarboxylic acid | 1-18 | 1-18 | 2-16 | 4-12 |
| Carbonate | 0-50 | 0-30 | 0-30 | 0-30 |
| Phosphonate | 0-8 | 0-8 | 0-8 | 0-8 |
| Non-ionic surfactant | 0.1-15 | 0.1-15 | 0.5-8 | 0.5-8 |
| Phosphate | - | - | - | - |
| Bleaching Agent | - | - | - | - |
| Misc | To 100 | To 100 | To 100 | To 100 |

| **Additional Preferred Automatic Dishwashing Agents** | | | | |
|---|---|---|---|---|
| **Ingredient** | **Wt %** | | | |
| | Formula 1 | Formula 2 | Formula 3 | Formula 4 |
| Citrate | 12-50 | 15-40 | 12-50 | 15-40 |
| Maleic acid | 1-18 | 1-18 | 2-16 | 4-12 |
| Carbonate | 5-50 | 10-30 | 5-50 | 10-30 |
| Phosphonate | 1-8 | 1-8 | 1.2-6 | 1.2-6 |
| Non-ionic surfactant | 0.1-15 | 0.1-15 | 0.5-8 | 0.5-8 |
| Phosphate | - | - | - | - |
| Bleaching Agent | - | - | - | - |
| Misc | To 100 | To 100 | To 100 | To 100 |

| **Preferred Automatic Dishwashing Agents** | | | | |
|---|---|---|---|---|
| **Ingredient** | **Wt %** | | | |
| | Formula 1 | Formula 2 | Formula 3 | Formula 4 |
| Citrate | 12-50 | 15-40 | 12-50 | 15-40 |
| Dicarboxylic acid | 1-18 | 1-18 | 2-16 | 4-12 |
| Carbonate | 0-50 | 0-30 | 0-30 | 0-30 |
| Phosphonate | 0-8 | 0-8 | 0-8 | 0-8 |
| Sulfo copolymer | 0-20 | 0-20 | 0-20 | 0-20 |
| Non-ionic surfactant | 0-15 | 0-15 | 0-8 | 0-8 |
| Enzyme preparations | 0.1-12 | 0.1-12 | 0.5-8 | 0.5-8 |
| Phosphate | - | - | - | - |
| Bleaching Agent | - | - | - | - |
| Misc | To 100 | To 100 | To 100 | To 100 |

| **Additional Preferred Automatic Dishwashing Agents** | | | | |
|---|---|---|---|---|
| **Ingredient** | **Wt %** | | | |
| | Formula 1 | Formula 2 | Formula 3 | Formula 4 |
| Citrate | 12-50 | 15-40 | 12-50 | 15-40 |
| Maleic acid | 1-18 | 1-18 | 2-16 | 4-12 |
| Carbonate | 5-50 | 10-30 | 5-50 | 10-30 |
| Phosphonate | 1-8 | 1-8 | 1.2-6 | 1.2-6 |
| Sulfo copolymer | 0-20 | 0-20 | 0-20 | 0-20 |
| Non-ionic surfactant | 0.1-15 | 0.1-15 | 0.5-8 | 0.5-8 |
| Enzyme preparations | 0.1-12 | 0.1-12 | 0.5-8 | 0.5-8 |
| Phosphate | - | - | - | - |
| Bleaching Agent | - | - | - | - |
| Misc | To 100 | To 100 | To 100 | To 100 |

| **Preferred Automatic Dishwashing Agents** | | | | |
|---|---|---|---|---|
| **Ingredient** | **Wt %** | | | |
| | Formula 1 | Formula 2 | Formula 3 | Formula 4 |
| Citrate | 12-50 | 15-40 | 12-50 | 15-40 |
| Dicarboxylic acid | 1-18 | 1-18 | 2-16 | 4-12 |
| Carbonate | 0-50 | 0-30 | 0-30 | 0-30 |
| Phosphonate | 0-8 | 0-8 | 0-8 | 0-8 |
| Sulfo copolymer | 0-20 | 0-20 | 0-20 | 0-20 |
| Non-ionic surfactant | 0-15 | 0-15 | 0-8 | 0-8 |
| Enzyme preparations | 0-12 | 0-12 | 0-8 | 0-8 |
| Organic Solvent | 0.1-15 | 0.5-8 | 0.1-15 | 0.5-8 |
| Phosphate | - | - | - | - |
| Bleaching Agent | - | - | - | - |
| Misc | To 100 | To 100 | To 100 | To 100 |

| **Additional Preferred Automatic Dishwashing Agents** | | | | |
|---|---|---|---|---|
| **Ingredient** | **Wt %** | | | |
| | Formula 1 | Formula 2 | Formula 3 | Formula 4 |
| Citrate | 12-50 | 15-40 | 12-50 | 15-40 |
| Dicarboxylic acid | 1-18 | 1-18 | 2-16 | 4-12 |
| Carbonate | 5-50 | 10-30 | 5-50 | 10-30 |
| Phosphonate | 1-8 | 1-8 | 1.2-6 | 1.2-6 |
| Sulfo copolymer | 0-20 | 0-20 | 0-20 | 0-20 |
| Non-ionic surfactant | 0.1-15 | 0.1-15 | 0.5-8 | 0.5-8 |
| Enzyme preparations | 0.1-12 | 0.1-12 | 0.5-8 | 0.5-8 |
| Organic Solvent | 0.1-15 | 0.5-8 | 0.1-15 | 0.5-8 |
| Phosphate | - | - | - | - |
| Bleaching Agent | - | - | - | - |
| Misc | To 100 | To 100 | To 100 | To 100 |

| **Automatic Dishwashing Agents** | | |
|---|---|---|
| **Ingredient** | **Wt %** | |
| | C 1 | E 1 |
| Sodium citrate | 9 | 9 |
| Potassium hydroxide | 7 | 7 |
| Sodium carbonate | 14 | 14 |
| Maleic acid | - | 1 |
| Sulfo polymer | 4.2 | 4.2 |
| HEDP | 1.5 | 1.5 |
| Non-ionic surfactant | 2 | 2 |
| Protease preparation | 2 | 2 |
| Amylase preparation | 0.8 | 0.8 |
| Alkanolamine | 1.5 | 1.5 |
| Thickener | 2 | 2 |
| Water, misc | To 100 | To 100 |

| **Manual Dishwashing Agents** | | | | | | | |
|---|---|---|---|---|---|---|---|
| **Ingredient** | **Wt %** | | | | | | |
| | Invention 1 | Invention 2 | Invention 3 | Invention 4 | Invention 5 | Invention 6 | Invention 7 |
| Fatty alcohol ether sulfate | 10 | 13.33 | 12 | 12 | 13.3 | 13.3 | 13.3 |
| Cocamidopropylbe taine | 2.5 | 3.33 | 3.1 | 3.1 | 3 | 3 | 3 |
| Sce. Alkane sulfonate | 2.5 | 3.33 | 2.9 | 2.9 | 3.7 | 3.7 | 3.7 |
| Fatty alcohol ethoxylate | 9 | 6 | - | - | - | - | - |
| Sodium chloride | 24 | 24 | 22 | 24 | 20 | 24 | 20 |
| Ethanol | - | - | 2 | 2 | 2.5 | 2.5 | 4 |
| Perfume | 0.2 | 0.3 | 0.3 | 0.3 | 0.3 | 0.3 | 0.3 |
| Colorant | 0.2 | 0.2 | 0.2 | 0.2 | 0.2 | 0.2 | 0.2 |
| Water | 51.60 | 49.51 | 57.5 | 55.5 | 57 | 53 | 55.5 |

| **Antibacterially active detergent/cleaning agent** | | | | | | |
|---|---|---|---|---|---|---|
| **Ingredient** | **V1** | **E1** | **E2** | **E3** | **E4** | **E5** |
| C₁₂₋₁₈ fatty alcohol with 7EO | 12 | 12 | 12 | 5 | 5 | - |
| N-cocoalkyl N, N dimethylamine oxide | 1.95 | 1.95 | 1.95 | 2 | 2 | - |
| Esterquat (N-methyl-N-(2 hydroxyethyl)-N-N-(ditallowacyloxyethyl)ammonium methosulfate | - | - | - | - | - | 15 |
| AgNO₃.H₂O | 0.0043 | 0.0043 | 0.0043 | 0.004 | 0.004 | 0.004 |
| C14 fatty acid | 5 | 5 | - | - | - | - |
| Farnesol | 0.02 | 0.02 | 0.02 | 0.02 | 0.02 | 0.02 |
| Coco Fatty acid | 2.5 | 2.5 | 2.5 | 12 | - | - |
| Citric Acid | - | - | - | 1.0 | 0.1 | - |
| H₂O₂ | - | 0.5 | 0.035 | 2 | 5 | 0.5 |
| NaOH | 0.35 | 0.35 | 0.35 | 1.9 | - | - |
| NH₄OH | 0.04 | 0.04 | 0.04 | 0.06 | - | - |
| 2-Propanol | - | - | - | - | - | 1.67 |
| MgCl₂ × 6H₂O | - | - | - | - | - | 0.01 |
| Perfume A | 1.00 | 1.00 | 1.00 | 1.00 | 1.00 | 0.75 |
| Water | To 100 | To 100 | To 100 | To 100 | To 100 | To 100 |
| pH | 8.5 | 8.5 | 8.5 | 8.5 | 5.5 | 2.6 |

| **Detergent containing anti-grey agent** | |
|---|---|
| **Ingredients** | **M1 (wt%)** |
| C₉₋₁₃ alkylbenzenesulfonate sodium salt | 10 |
| Sodium lauryl ether sulfate with 2EO | 5 |
| C₁₂₋₁₈ fatty alcohol with 7EO | 10 |
| C_{12- 14} alkyl polyglycoside | 2 |
| C₁₂₋₁₈ fatty acid sodium salt | 8 |
| Glycerol | 5 |
| Trisodium citrate | 1 |
| Polyacrylate | 2 |
| Active ingredient (anti-grey agent-a polycarbonate-, polyurethane-, and/or polyurea-polyorganosiloxane compound or a precursor compound use in the production thereof) | 1 |
| Enzyme, dye, optical brightener | + |
| Water | To 100 |

| **Example detergent compositions for application to a substrate** | | | | | |
|---|---|---|---|---|---|
| Ingredients | Weight Percent (actives %) | | | | |
| | D1 | D2 | D3 | D4 | D5 |
| Sodium dodecyl benzene sulfonate | 26.09 | 17.30 | 15.60 | 17.70 | 27.00 |
| Sodium alkyl *C*₁₄₋₁₅/7EO ether sulfate | 13.80 | | | | 14.00 |
| Linear alcohol ethoxylate *C*₁₄₋₁₅/7EO | 13.44 | 5.40 | 14.60 | 5.50 | 14.00 |
| Linear alcohol ethoxylate *C*₁₂₋₂₀/7EO | | | | | 23.00 |
| Polyethylene Glycol PEG-75 | 2.00 | 1.40 | 1.30 | 1.40 | 2.00 |
| Polyoxyethylene (100) stearyl ether | 21.99 | 15.60 | 14.10 | 15.90 | |
| Sodium Silicate Si0₂/Na₂0 ratio 1.6-1.8 | 3.72 | 16.60 | 15.00 | 17.00 | |
| Sodium Silicate (Britesil^{®} C24) | 7.00 | | | | 11.00 |
| Sodium Carbonate | | 6.50 | 5.90 | 6.70 | |
| Sodium tetraborate decahydrate | | 11.90 | 10.80 | 12.20 | |
| Sodium polyacrylate -4,500 MW | | 1.80 | 1.70 | | |
| EDTA - tetrasodium salt | | 0.10 | 0.10 | 0.10 | |
| Optical brightener (Tinopal^{®} CBS-X) | 0.15 | 0.10 | 0.09 | 0.10 | 0.20 |
| Dyes and fragrances | 0.90 | 0.90 | 0.81 | 1.01 | 0.35 |
| Water | 10.92 | 22.10 | 19.90 | 22.40 | 9.55 |

| **Example enzyme containing compositions for application to a substrate** | | | | | |
|---|---|---|---|---|---|
| **Ingredients** | **Weight Percent (actives %)** | | | | |
| | **E1** | **E2** | **E3** | **E4** | **E5** |
| Polyethylene Glycol PEG-75 | 98.60 | 99.10 | | | |
| Fatty acid based matrix 1 | | | 98.9 | | 99.10 |
| Fatty acid based matrix 2 | | | | 98.80 | |
| Protease | 0.10 | 0.10 | 0.12 | 0.10 | 0.10 |
| Mannanase | 0.02 | | 0.02 | 0.02 | |
| Amylase | 0.12 | 0.25 | 0.1 | 0.12 | 0.25 |
| Cellulase | 0.08 | | 0.1 | 0.08 | |
| Lipase | 0.08 | | | 0.08 | |
| Pectate Lyase | | | | 0.05 | |
| Enzyme Stabilizers | 1.00 | 0.55 | 0.75 | 0.75 | 0.55 |

Fatty acid based matrix 1 is comprised of 20 wt. % of the sodium salt of coconut fatty acid, 50 wt. % of non polymeric polyols (sorbitol, glycerin, propylene glycol, sucrose and glucose), 15 wt.% of anionic and nonionic surfactants, and 15 wt. % of water.

Fatty acid based matrix 2 is comprised of 20 wt.% of the sodium salt of stearic acid, 3 wt.% of the sodium salt of lauric acid, 3 wt.% of the sodium salt of myristic acid, 50 wt.% of non polymeric polyols (sorbitol, glycerin, and propylene glycol), 2 wt.% of lauric acid, 2 wt.% of stearic acid, 10 wt.% of anionic surfactant, and 10 wt.% of water.

| **Table 1 Detergent Composition** | |
|---|---|
| **Ingredients** | **(% by weight)** |
| Soap (saturated C₁₂₋₂₄ fatty acid soaps and oleic acid soap) | 5.42 |
| Sodium C₁₂₋₁₄ alkyl benzene sulfonate | 22.67 |
| Sodium C₁₄₋₁₆ fatty alcohol sulfate | 4.59 |
| C₁₂₋₁₈ fatty alcohol.5EO | 0.81 |
| Sodium carbonate | 4.55 |
| Zeolite A | 29.86 |
| Sodium silicate | 8.00 |
| Acrylic acid/maleic acid copolymer | 16.16 |
| Opt. brightener | 0.45 |
| Phosphonate | 2.30 |
| NaOH, 50% | 0.63 |
| Water | 3.88 |
| Other salts | 0.68 |
| **Table 2** | |
| Detergent composition | 59.5% |
| Coated bleaching agent (Na percarbonate) | 23.3% |
| Coated bleach activator (TAED) | 7% |
| Citric acid monohydrate | 10.2% |

| **Particulate detergent composition** | |
|---|---|
| Ingredient | % wt |
| sodium dodecylbenzenesulphonate | 8.5 |
| c12-C15 primary alcohol, condensed with 7 moles of ethylene oxide | 4 |
| sodium-hardened rapeseed oil soap | 1.5 |
| sodium triphosphate | 33 |
| sodium carbonate | 5 |
| sodium silicate | 6 |
| sodium sulphate | 20 |
| water | 9 |
| fluorescers, soil-suspending agents,dyes, perfumes | minor amounts |
| sodium perborate | 12 |
| tetraacetyl ethylene diamine (TAED) (granules) | 2 |
| proteolytic enzyme (Savinase ex.Novo) | 0.4 |

| **Detergent composition A** |
|---|
| 9 % anionic detergent |
| 1 % nonionic detergent |
| 21.5 % sodium tripolyphosphate |
| 7 % sodium perborate |
| 0.6 % Savinase (a proteolytic enzyme) |
| balance sodium sulphate + minor ingredients |

| **Detergent composition B** |
|---|
| 9 % anionic detergent |
| 4 % nonionic detergent |
| 28% zeolite |
| 4.5% nitrilotriacetate |
| 5.5% sodium perborate |
| 3.5% tetraacetylethylenediamine |
| 0.5% Savinase |
| balance sodium sulphate + minor ingredients |

| **Detergent composition C** |
|---|
| 5 % anionic detergent |
| 4 % nonionic detergent |
| 1 % soap |
| 30 % zeolite |
| 3. % copolymer of acrylic acid with mateic anhydride |
| 7.5% sodium perborate |
| 3 % tetraacetylethylenediamine |
| balance sodium sulphate + minor ingredients |

| **Detergent composition D** |
|---|
| 8 % anionic synthetic detergent |
| 4 % nonionic synthetic detergent |
| 4 % soap |
| 35. % sodium carbonate |
| 20 % powdered calcite |
| 6 % sodium perborate |
| 2 % tetraacetylethylenediamine |
| 0.5% Savinase |
| balance sodium sulphate + minor ingredients |

| **Laundry detergent composition** | |
|---|---|
| Ingredients | Parts by weight |
| Sodium dodecyl benzene sulphonate | 8.5 |
| C12-C15 primary alcohol, condensed with 7 moles of ethylene oxide | 4 |
| Sodium-hardened rapeseed oil soap | 1.5 |
| Sodium triphosphate | 33 |
| Sodium carbonate | 5 |
| Sodium silicate | 6 |
| Sodium sulphate | 20 |
| Water | 9 |
| Fluorescers, soil-suspending agents, dyes, perfumes | minor amount |
| Sodium perborate | 12 |
| Tetraacetyl ethylene diamine (TAED) (granules) | 2 |
| Proteolytic enzyme (Savinase ex NOVO) | 0.4 |

| **Laundry detergent compositions** | | | | |
|---|---|---|---|---|
| | A | B | C | D |
| sodium dodecylbenzene sulphonate | 9 | 9 | 9 | 9 |
| C13-C15 linear primary alcohol, condensed with 7 moles of ethylene oxide (e.g. Synperonic A7) | 1 | 4 | 4 | 1 |
| C13-C15 linear primary alcohol, condensed with 3 moles of ethylene oxide (e.g. Synperonic A3) | 3 | 0 | 0 | 3 |
| sodium tripolyphosphate | 23 | 23 | 0 | 0 |
| zeolite type 4A | 0 | 0 | 24 | 24 |
| copolymer of acrylic acid with maleic anhydride | | | 4 | 4 |
| sodium polyacrylate | 2 | 2 | 0 | 0 |
| alkaline silicate | 5 | 5 | | |
| fluorescer | 0.25 | 0.25 | 0.16 | 0.16 |
| EDTA | 0.15 | 0.15 | 0.18 | 0.18 |
| SCMC | 0.5 | 0.5 | 0.55 | 0.55 |
| salt | 2 | 2 | | |
| sodium sulphate | 26.8 | 26.8 | 22.31 | 22.31 |
| sodium carbonate | 0 | 0 | 10.3 | 10.3 |
| moisture | 10 | 10 | 11 | 11 |
| TAED | 3 | 3 | 3.3 | 3.3 |
| sodium perborate monohydrate | 10 | 10 | 8 | 8 |
| calcium Dequest^{® 2047} | 0.7 | 0.7 | 0.3 | 0.3 |
| foam depressor | 3 | 3 | 2.5 | 2.5 |
| perfume | 0.2 | 0.2 | 0 | 0 |
| alkaline protease (Savinase (A) 6T) | 0.4 | 0.4 | 0.4 | 0.4 |

| Detergent composition | | | | |
|---|---|---|---|---|
| **Ingredients** | **Ex. 1** | **Ex. 2** | **Ex.3** | **Ex.4** |
| **Material** | **Level (parts as is)** | **Level (parts as is)** | **Level (parts as is)** | **Level (parts as is)** |
| Glycerol | 3.17 | 3.17 | 3.17 | 3.17 |
| MPG | 5.7 | 5.7 | 5.7 | 5.7 |
| NaOH | 2.13 | 2.13 | 2.13 | 2.13 |
| TEA | 2.05 | 2.05 | 2.05 | 2.05 |
| Neodol 25-7 | 12.74 | 12.74 | 12.74 | 12.74 |
| F-Dye | 0.18 | 0.18 | 0.18 | 0.18 |
| Citric Acid | 1.71 | 1.71 | 1.71 | 1.71 |
| LAS (as LAS Acid) | 8.49 | 8.49 | 8.49 | 8.49 |
| Fatty acid | 3.03 | 3.03 | 3.03 | 3.03 |
| Empigen BB | 1.5 | 1.5 | 1.5 | 1.5 |
| SLES | 4.24 | 4.24 | 4.24 | 4.24 |
| Dequest 2066 | 0.875 | 0.875 | 0.875 | 0.875 |
| Patent Blue | 0.00036 | 0.00036 | 0.00036 | 0.00036 |
| Acid Yellow | 0.00005 | 0.00005 | 0.00005 | 0.00005 |
| Opacifier | 0.0512 | 0.0512 | 0.0512 | 0.0512 |
| Perfume | 0.734 | 0.734 | 0.734 | 0.734 |
| Borax | 10 | 10 | 10 | 10 |
| Savinase | 2.362 | 2.362 | 2.362 | 2.362 |
| Stainzyme | 0.945 | 0.945 | 0.945 | 0.945 |
| Soap | 3.03 | 3.03 | 3.03 | 3.03 |
| EPEI 20E0 (ex Nippon Shokubai) polyethyleneimine having a weight average molecular weight of about 600, and wherein the polyethyleneimine has been modified by alkoxylation with an average 20 ethylene oxide moieties | 5.5 | 5.5 | 5.5 | 9 |
| Lipex^{®} (ex Novozymes) | 3 | 3 | 3 | 3 |
| Texcare SRN170 (ex Clariant) soil release polymer | 0 | 7.5 | 0 | 0 |
| Sokolan CP5 (ex BASF) Soil-release polymer | 0 | 0 | 20 | 0 |

As indicated above, the cleaning compositions of the present invention are formulated into any suitable form and prepared by any process chosen by the formulator, non-limiting examples of which are described in U.S. Pat. Nos. 5,879,584, 5,691,297, 5,574,005, 5,569,645, 5,516,448, 5,489,392, and 5,486,303. In some embodiments not covered by the invention in which a low pH cleaning composition is desired, the pH of such composition is adjusted via the addition of an acidic material such as HCl.

The cleaning compositions disclosed herein of find use in cleaning a *situs* (*e.g*., a surface, item, dishware, or fabric). Typically, at least a portion of the situs is contacted with an embodiment of the present cleaning composition, in neat form or diluted in a wash liquor, and then the situs is optionally washed and/or rinsed. For purposes of the present invention, "washing" includes but is not limited to, scrubbing, and mechanical agitation. In some embodiments not covered by the invention, the cleaning compositions are typically employed at concentrations of from about 500 ppm to about 15,000 ppm in solution. When the wash solvent is water, the water temperature typically ranges from about 5°C to about 90°C and, when the situs comprises a fabric, the water to fabric mass ratio is typically from about 1:1 to about 30:1.

### Processes of Making and Using Cleaning Compositions

The cleaning compositions of the present invention are formulated into any suitable form and prepared by any suitable process chosen by the formulator, (*See e.g*., US Patent Nos. 5,879,584, 5,691,297, 5,574,005, 5,569,645, 5,565,422, 5,516,448, 5,489,392, 5,486,303, 4,515,705, 4,537,706, 4,515,707, 4,550,862, 4,561,998, 4,597,898, 4,968,451, 5,565,145, 5,929,022, 6,294,514 and 6,376,445).

.

### Methods of Use

In some embodiments, the cleaning compositions of the present invention find use in cleaning surfaces (*e.g*., dishware), laundry, hard surfaces, contact lenses, etc. In some embodiments, at least a portion of the surface is contacted with at least one embodiment of the cleaning compositions of the present invention, in neat form or diluted in a wash liquor, and then the surface is optionally washed and/or rinsed. For purposes of the present invention, "washing" includes, but is not limited to, scrubbing, and mechanical washing.

The present invention provides methods for cleaning or washing an item or surface (*e.g*., hard surface) in need of cleaning, including, but not limited to methods for cleaning or washing a dishware item, a tableware item, a fabric item, a laundry item, personal care item, etc., or the like, and methods for cleaning or washing a hard or soft surface (*e.g*., a hard surface of an item).

In some embodiments, the present invention provides a method for cleaning an item, object, or surface in need of cleaning, the method comprising contacting the item or surface (or a portion of the item or surface desired to be cleaned) with at least one variant thermolysin protease of the present invention or a composition of the present invention for a sufficient time and/or under conditions suitable and/or effective to clean the item, object, or surface to a desired degree. Some such methods further comprise rinsing the item, object, or surface with water. For some such methods, the cleaning composition is a dishwashing detergent composition and the item or object to be cleaned is a dishware item or tableware item. As used herein, a "dishware item" is an item generally used in serving or eating food. A dishware item can be, but is not limited to for example, a dish, plate, cup, bowl, etc., and the like. As used herein, "tableware" is a broader term that includes, but is not limited to for example, dishes, cutlery, knives, forks, spoons, chopsticks, glassware, pitchers, sauce boats, drinking vessels, serving items, etc. It is intended that "tableware item" includes any of these or similar items for serving or eating food. For some such methods, the cleaning composition is an automatic dishwashing detergent composition or a hand dishwashing detergent composition and the item or object to be cleaned is a dishware or tableware item. For some such methods, the cleaning composition is a laundry detergent composition (*e.g*., a power laundry detergent composition or a liquid laundry detergent composition), and the item to be cleaned is a fabric item.

In some embodiments not covered by the invention, the present invention provides methods for cleaning or washing a fabric item optionally in need of cleaning or washing, respectively. In some embodiments, the methods comprise providing a composition comprising the variant protease, including but not limited to fabric or laundry cleaning composition, and a fabric item or laundry item in need of cleaning, and contacting the fabric item or laundry item (or a portion of the item desired to be cleaned) with the composition under conditions sufficient or effective to clean or wash the fabric or laundry item to a desired degree.

In some embodiments, the present invention provides a method for cleaning or washing an item or surface (*e.g*., hard surface) optionally in need of cleaning, the method comprising providing an item or surface to be cleaned or washed and contacting the item or surface (or a portion of the item or surface desired to be cleaned or washed) with at least one thermolysin variant of the invention or a composition of the invention comprising at least one such thermolysin variant for a sufficient time and/or under conditions sufficient or effective to clean or wash the item or surface to a desired degree. Such compositions include, but are not limited to for example, a cleaning composition or detergent composition of the invention (*e.g*., a hand dishwashing detergent composition, hand dishwashing cleaning composition, laundry detergent or fabric detergent or laundry or fabric cleaning composition, liquid laundry detergent, liquid laundry cleaning composition, powder laundry detergent composition, powder laundry cleaning composition, automatic dishwashing detergent composition, laundry booster cleaning or detergent composition, laundry cleaning additive, and laundry pre-spotter composition, etc.). In some embodiments, the method is repeated one or more times, particularly if additional cleaning or washing is desired. For example, in some instance, the method optionally further comprises allowing the item or surface to remain in contact with the at least one variant protease or composition for a period of time sufficient or effective to clean or wash the item or surface to the desired degree. In some embodiments , the methods further comprise rinsing the item or surface with water and/or another liquid. In some embodiments, the methods further comprise contacting the item or surface with at least one variant protease of the invention or a composition of the invention again and allowing the item or surface to remain in contact with the at least one variant protease or composition for a period of time sufficient to clean or wash the item or surface to the desired degree. In some embodiments, the cleaning composition is a dishwashing detergent composition and the item to be cleaned is a dishware or tableware item. In some embodiments not covered by the invention, the cleaning composition is an automatic dishwashing detergent composition or a hand dishwashing detergent composition and the item to be cleaned is a dishware or tableware item. In some embodiments of the methods, the cleaning composition is a laundry detergent composition and the item to be cleaned is a fabric item.

The present invention also provides methods for cleaning a surface, item or object optionally in need of cleaning, the method comprises contacting the item or surface (or a portion of the item or surface desired to be cleaned) with at least one variant thermolysin of the present invention or a cleaning composition of the invention in neat form or diluted in a wash liquor for a sufficient time and/or under conditions sufficient or effective to clean or wash the item or surface to a desired degree. The surface, item, or object may then be (optionally) washed and/or rinsed if desired. For purposes of the present invention, "washing" includes, but is not limited to for example, scrubbing and mechanical agitation. In some embodiments not covered by the invention, the cleaning compositions are employed at concentrations of from about 500 ppm to about 15,000 ppm in solution (e.g., aqueous solution). When the wash solvent is water, the water temperature typically ranges from about 5°C to about 90°C and when the surface, item or object comprises a fabric, the water to fabric mass ratio is typically from about 1:1 to about 30:1.

.

### EXPERIMENTAL

*Some of the examples are not covered by the invention but provide a useful understanding of the invention.*

### EXAMPLE 1

### Assays

The following assays are standard assays used in the examples described below. Occasionally specific protocols call for deviations from these standard assays. In those cases, deviations from these standard assay protocols below are identified in the examples.

### A. Performance Index

The performance index (PI) compares the performance of the variant (measured value) and the standard enzyme (theoretical value) at the same protein concentration. In addition, the theoretical values can be calculated, using the parameters of the Langmuir equation of the standard enzyme.

A performance index (PI) that is greater than 1 (PI>1) indicates improved performance by a variant as compared to the standard (e.g., Thermolysin), while a PI of 1 (PI=1) identifies a variant that performs the same as the standard, and a PI that is less than 1 (PI<1) identifies a variant that performs worse than the standard.

### B. Abz-AGLA-Nba Protease Assay in 96-well Microtiter Plates

In order to determine the protease activity of the Thermolysin metalloprotease and thermolysin metalloprotease variants, the hydrolysis of 2-Aminobenzoyl-L-alanylglycyl-L-leucyl-L-alanino-4-nitrobenzylamide (Abz-AGLA-Nba) is measured.

The reagent solutions used are:
i) MES buffer (52.6 mM MES, adjusted to pH 6.5 with NaOH, and containing 2.6 mM CaCl₂ and 0.00526% (v/v) TWEEN^{®}-80);
ii) Abz-AGLA-Nba stock solution (48 mM Abz-AGLA-Nba in DMF), kept at room temperature shielded from light;
iii) Enzyme dilution buffer with propylene glycol (10 mM NaCl, 0.1 mM CaCl₂ and 0.005% (v/v) TWEEN^{®}-80, 10% propylene glycol).

To prepare a 2.4 mM Abz-AGLA-Nba working solution, 1 mL of Abz-AGLA-Nba stock solution is added to 19 mL of MES buffer and mixed thoroughly for at least 10 seconds. The solution is kept at room temperature shielded from light.

To prepare the protease solutions, filtered culture supernatants of Thermolysin variants are diluted 50-fold in the enzyme dilution buffer.

The assay is performed in disposable black polystyrene flat-bottom 96-well micro plates suitable for fluorescence reading (e.g., Greiner 655076). First, 195 µL of 2.4 mM Abz-AGLA-Nba working solution is added to each well of the 96-well micro assay plates, followed by the addition of 5 µL of diluted protease samples. The solutions are mixed for 5 seconds and the fluorescence change is measured in kinetic mode (9 readings in 180 seconds, excitation wavelength 350 nm, emission wavelength 415 nm, no cut-off filter) at 25°C using a micro plate spectrofluorometer (SpectraMAX Gemini EM, Molecular Devices). The rate of fluorescence change in RFU/sec (RFU=relative fluorescence units) provides a measure of protease activity.

### C. Stability Assays

The thermostability of the Thermolysin variants relative to the wild-type Thermolysin enzyme having the amino acid sequence of SEQ ID NO: 3 is determined by incubating the protease samples under defined conditions in either HEPES buffer, or a detergent solution. The temperature of the incubation is chosen such that the remaining activity of wild-type Thermolysin after the incubation is equal to approximately 30% of the initial activity. The initial and residual Thermolysin activities are determined using the Abz-AGLA-Nba assay described above in section B.

The reagent solutions used for this set of assays are:
1. 2.4 mM Abz-AGLA-Nba working solution (see section B, above)
2. Dilution buffer: 10 mM NaCl, 0.1 mM CaCl₂, 0.005% (v/v) TWEEN^{®-}80
3. HEPES buffer: 10mM HEPES, 0.1mM CaCl₂, 0.005% (v/v) TWEEN^{®}-80, pH 7.15
4. AT formula pH 8 detergent (2,5g/L in 21°GH water)
5. Sun All-in-1 Turbo Gel pH 6.3 (3g/L in 21°GH water)
6. Filtered culture supernatants of Thermolysin variants

The equipment used for this set of assays includes a Biomek FX Robot (Beckman Coulter), a SpectraMAX Gemini EM micro plate spectrofluorometer (Molecular Devices) and Tetrad2 Peltier Thermal cycler (Bio-Rad).

### Thermostability assay in buffer:

Culture supernatants of Thermolysin variants are diluted to ~1µg/ml in HEPES buffer, and 50 µl/well of diluted enzyme sample is transferred to a 96-well PCR plate. The initial activity of the enzyme samples is measured using the Abz-AGLA-Nba assay as described in section B above, by transferring 5 µL of enzyme sample to a black 96-well assay micro plate (e.g., Greiner 655076) containing 195 µL of 2.4 mM Abz-AGLA-Nba substrate solution. The PCR plate containing the remaining 45 µl/well of the enzyme samples is sealed with an adhesive foil seal (Bio-Rad B-seal), placed in the Tetrad2 thermal cycler and incubated for 15 min at 83°C. After incubation, the samples in the PCR plate are cooled to room temperature and residual activity of the enzyme samples is measured using Abz-AGLA-Nba assay as described in section B above, by transferring 5 µL of enzyme sample to a black 96-well assay micro plate (e.g., Greiner 655076) containing 195 µL of 2.4 mM Abz-AGLA-Nba substrate solution. The thermostability activity ratio is calculated based on enzyme activity after the heat incubation divided by enzyme activity before the heat incubation, and is expressed as percentage remaining activity. The performance index for thermostability is determined by comparing the activity ratio of the variant enzyme with that of the similarly treated wild-type Thermolysin enzyme having the amino acid sequence of SEQ ID NO: 3.

### Detergent stability assays:

The detergent stability of the Thermolysin variants is monitored by incubating the variants under stress conditions in a 0.3% (w/v) solution of the liquid automatic dish detergent known commercially as Sun All-in-1 Turbo Gel (Unilever, The Netherlands) and in a 0.25% (w/v) solution of the AT formula pH 8 detergent (described in section E) at elevated temperature. Heat inactivation of enzyme present in the commercially available Sun All-in-1 Turbo Gel detergent is performed by incubating a 10% detergent solution at 80°C for 2 hours. At the end of the incubation, the measured pH value is 6.3.

Culture supernatants of Thermolysin variants are diluted to ~1µg/ml in the detergent solution, and 50 µl/well of diluted enzyme sample is transferred to a 96-well PCR plate. The PCR plate is sealed with an adhesive foil seal (Bio-Rad-B seal), placed in the Tetrad2 thermal cycler and incubated for 15 min. The temperature of the incubation is chosen such that the remaining activity of wild-type Thermolysin after the incubation is equal to approximately 30% of the initial activity. The samples in the heat-inactivated Sun All-in-1 Turbo Gel are incubated at 81°C for 15 min, the samples in the AT formula pH 8 detergent are incubated at 69°C for 15 min. After incubation, the samples in the PCR plate are cooled to room temperature and residual activity of the enzyme samples is measured using Abz-AGLA-Nba assay as described in section B above, by transferring 5 µL of enzyme sample to a black 96-well assay micro plate (e.g., Greiner 655076) containing 195 µL of 2.4 mM Abz-AGLA-Nba substrate solution.

The detergent activity ratio is calculated based on enzyme activity in the detergent solution after the heat incubation divided by enzyme activity in HEPES buffer before the heat incubation, and is expressed as percentage remaining activity.

The performance index for detergent stability is determined by comparing the activity ratio of the variant enzyme, with that of the similarly treated wild-type Thermolysin enzyme having the amino acid sequence of SEQ ID NO: 3.

### D. PAS-38 microswatch assay:

The cleaning performance of the Thermolysin variants is tested using a microswatch assay on polyacryl swatches pre-stained with egg yolk and pigment (Center for Testmaterials, CFT, The Netherlands), in a 96-well micro plate format. The principle of this protease wash-performance assay is based on the liberation of egg yolk particles and a carbon black dye due to the hydrolysis of egg yolk incorporated on a microswatch. The absorbance at 405 nm of the wash liquid is measured, providing a measure of protease activity in the sample analysed (at the desired conditions: pH, temperature, detergent).

### Reagent and solutions used:

1. PAS-38 microswatches (egg yolk on polyacryl fabric, aged and colored with carbon black dye; CFT-Vlaardingen, The Netherlands)
2. Citrate based detergent, pH8, with and without PAP (AT formulation, see section E)
3. The heat-inactivated commercially available liquid detergent Sun All-in-1 Turbo Gel
4. 100 mM CAPS buffer pH 10.2 (Rinse buffer)
5. Dilution buffer with propylene glycol: 10mM NaCl, 0.1mM CaCl₂, 0.005% TWEEN^{®}80 solution, 10% propylene glycol

### Detergents and Conditions:

The protease samples (filtered supernatants of bacterial cultures grown in MTP plates) are tested at appropriate concentrations under several conditions.
- AT formula pH 6 detergent, 50°C; final protease concentration in assay ~ 0.3 µg/ml
- AT formula pH 8 detergent, 50°C ; final protease concentration in assay ~ 0.2 µg/ml
- AT formula pH 8 detergent + PAP, 50°C ; final protease concentration in assay ~ 0.15 µg/ml
- Sun All-in-1 Turbo Gel, pH 6.3 detergent, 50°C; final protease concentration in assay ~0.5 µg/ml

### Method

PAS-38 swatches are cut into 5 mm diameter pieces and placed in each well of a 96 well microplate. Culture supernatant samples are diluted in dilution buffer to approximately 10 µg/ml. Using a Biomek FX pipetting robot, detergent solution and diluted enzyme samples are added to a 96-well microplate containing PAS-38 microswatches to a final volume of 180 µl/well. The MTP is sealed with an adhesive seal, placed in the iEMS incubator/shaker (Thermo Scientific) and incubated for 30 minutes at 50°C with shaking at 1150 RPM. After the incubation, 100 µL of wash liquid from each well is transferred to a new MTP, and the absorbance at 405 nm is measured using a SpectraMAX microplate spectrophotometer (Molecular Devices). This value is referred to as the "initial performance liquid". The remaining wash liquid from the microswatch plate is discarded and the microswatches are subsequently rinsed once with 200 µL of water. Finally, 180 µL of 0.1 M CAPS buffer is added to each well and the MTP is incubated for an additional period of 10 minutes in the iEMS incubator/shaker at 50°C with shaking at 1150 RPM. Following this incubation step, again 100 µL of liquid is transferred to a new MTP and the absorbance at 405 nm is measured using a SpectraMAX microplate spectrophotometer (Molecular Devices). This value is referred to as "rinse liquid". The two measurements (the "initial performance liquid" and the "rinse liquid") are added together and represent the "total performance". Control wells containing a microswatch, detergent but no enzyme are included for background subtraction.

### Calculation of the wash performance

The obtained absorbance value is corrected for the blank value (obtained after incubation of microswatches in the absence of enzyme), and the resulting absorbance is a measure of hydrolytic activity. A performance index (PI) is calculated for each sample. For the PI calculation for the wash performance indices, a Langmuir curve fit based on wild type Thermolysin is used. Using the protein concentration of the variants, the expected performance based on the curve fit is calculated. The observed performance is divided by the calculated performance and this value is then divided by the performance of the wild type Thermolysin enzyme having the amino acid sequence of SEQ ID NO: 3.

### E. Detergents

Two detergents are used:
1. Sun ALL-in-1 Turbo Gel (Unilever, The Netherlands) purchased commercially in 2010.
2. AT formula, ingredients listed below in Table 1.1

**Table 1.1. Composition of detergent AT formula**

| **Ingredient** | **concentration mg/ml** |
|---|---|
| MGDA (methylglycinediacetic acid) | 0.143 |
| Sodium citrate | 1.86 |
| Citric acid^{∗∗} | varies |
| PAP^{∗} (peracid *N,N-*phthaloylaminoperoxycaproic acid) | 0.057 |
| Plurafac^{®} LF 301 (a non-ionic surfactant) | 0.029 |
| Bismuthcitrate | 0.006 |
| Bayhibit^{®} S (Phosphonobutantricarboxylic acid sodium **salt)** | 0.006 |
| Acusol^{™} 587 (a calcium polyphosphate inhibitor) | 0.029 |
| PEG 6000 | 0.043 |
| PEG 1500 | 0.1 |
| | |

| | |
|---|---|
| ^{∗}PAP, was only added to the AT formula to prepare the AT formula pH 8 detergent + PAP. ^{∗∗}The pH of the AT formula detergent is adjusted to the desired value with citric acid. A 0.25% solution in 21°GH water is used for both the stability and the wash performance assays. | |

### F. Protein Determination

Protein determination of Thermolysin variants from culture supernatants is performed using an Agilent 1200 HPLC system. A calibration curve (18ppm-400ppm) using purified wild-type Thermolysin protein (concentration determined using A222 absorbance) is prepared in dilution buffer (10mM NaCl, 0.1mM CaCl₂, 0.005% TWEEN^{®}-80 solution, 10% propylene glycol) All samples are transferred to 96-well microplates, pretreated with hydrochloric acid (0.3 M final concentration) and incubated at 4 °C for 5 minutes. Prior to loading the samples using an auto-sampler to a size-exclusion column BioSuite 250 4 µm UHR, 4.6×300 mm (Waters Corporation, Milford, Massachusetts), the samples are treated with sodium dodecyl sulphate (SDS) to a final concentration of 2% (w/v). The samples are eluted from the column using 25 mM sodium phosphate, pH 6 containing 2% (w/v) SDS. The flow rate is 0.4 mL/min with a 14 min run. The absorption of the samples is measured at 222 nm using an UV-detector and the protein concentration determined based on the calibration curve.

The performance index is determined by comparing the expression of the variant enzyme with that of the *Bacillus thermoproteolyticus* Thermolysin enzyme having the amino acid sequence of SEQ ID NO: 3.

### EXAMPLE 2

### Generation of Bacillus thermoproteolyticus thermolysin Site Evaluation Libraries ("SELs")

Thermolysin-like proteases (TLPs) are members of the peptidase family M4 of which thermolysin (TLN; EC 3.4.24.27) is the prototype. The amino acid sequence of thermolysin, (EC 3.4.24.27) the neutral metallo endo-peptidase secreted from *Bacillus thermoproteolyticus* was first reported by Titani et al (Titani et al, (1972), Amino-acid sequence of thermolysin. Nature New Biol. 238:35-37). Subsequently, the gene for this enzyme was cloned by O'Donohue et al (O'Donohue,M.J (1994) Cloning and expression in Bacillus subtilis of the npr gene from Bacillus thermoproteolyticus Rokko coding for the thermostable metalloprotease thermolysin. Biochem. J. 300:599-603) and the sequence set forth as UniProtKB/Swiss-Prot Accession No. P00800 (SEQ ID NO:4). The only differences between the protein sequences reported by Titani et al and O'Donohue et al are the confirmation of Asn at position 37 (instead of Asp) and Gln at position 119 (instead of Glu).

The full-length thermolysin protein of *Bacillus thermoproteolyticus* (O'Donohue,M.J (1994) Biochem. J. 300:599-603*)* (shown here in SEQ ID NO:4) is greater than 99% identical to: the thermolysin of *Geobacillus caldoproteolyticus* (Chen et al (2004). Extremophiles 8:489-498, and described in WO2009058303) to the product of the nprS gene of *Bacillus stearothermophilus* (Nishiya,Y. and Imanaka,T. (1990) J. Bacteriol. 172:4861-4869), and to the *Bacillus stearothermophilus* nprM (M. Kubo and T. Imanaka, J. Gen. Microbiol. 134:1883-1892, 1988). As such the terms "thermolysin," "stearolysin", "bacillolysin," "proteinase-T", "PrT", "Thermolysin-like protease", and "TLPs", are used interchangeably herein to refer to the neutral metalloprotease enzyme of *Bacillus thermoproteolyticus.* The only sequence difference between full-length thermolysin protein of *Bacillus thermoproteolyticus* (SEQ ID NO:4) and thermolysin of *Geobacillus caldoproteolyticus* (SEQ ID NO:5), is the presence of Ala at position 115 (within the pro-region) instead of Ser, the result of a change of one nucleotide in the codon for that position.(TCG to GCG).

The pHPLT-ProteinaseT plasmid was provided to BaseClear (Leiden, The Netherlands) for the generation of Site Evaluation Libraries (SELs).This plasmid encodes the *Geobacillus caldoproteolyticus* thermolysin protein coding sequence. The full-length protein sequence (SEQ ID NO:2) differs in one amino acid within the pro-region of the molecule originally cloned (SEQ ID NO:5) but both produce identical 316 amino acid mature proteins. The amino acid sequence of the mature Thermolysin protein is shown in SEQ ID NO: 3. BaseClear generated positional libraries at each of the sites in the Thermolysin mature protein.

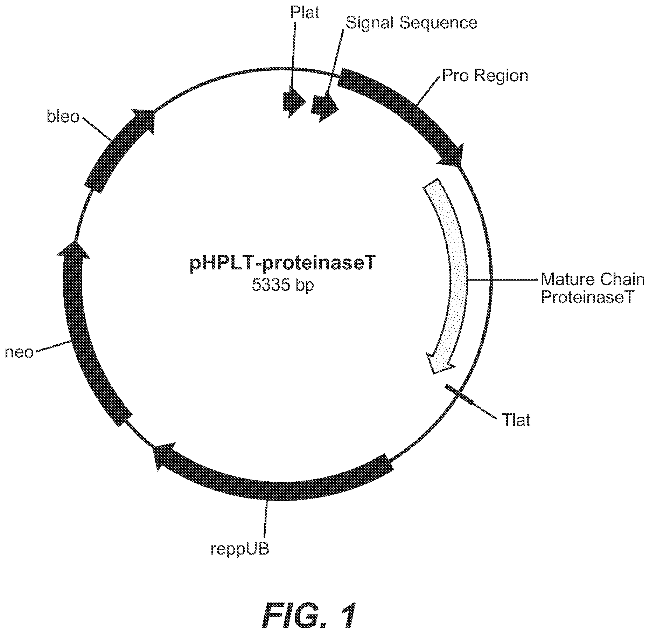

This *B. subtilis* expression plasmid, pHPLT-ProteinaseT, contains the Thermolysin expression cassette shown below, the *B. licheniformis* LAT promoter (Plat), and additional elements from pUB110 (McKenzie et al., Plasmid, 15:93-103, 1986) including a replicase gene (reppUB), a neomycin/kanamycin resistance gene (neo) and a bleomycin resistance marker (bleo) (Figure 4 in US Patent No. 6,566,112). The pHPLT-ProteinaseT plasmid map is provided in Figure 1. The Thermolysin expression cassette sequence is provided below in SEQ ID NO:1.

SEQ ID NO:1 sets forth the nucleotide sequence of Thermolysin gene from expression plasmid pHPLT-ProteinaseT (the native signal sequence is shown in lower case letters, native propeptide in lower case, underlined text, and mature sequence in uppercase letters):

atgaaaatgaaaatgaaattagcatcgtttggtcttgcagcaggactagcggcccaagtattttaccttacaatgcgctggcttcaacggaacacgttacat
ggaaccaacaatttcaaacccctcaattcatctccggtgatctgctgaaagtgaatggcacatccccagaagaactcgtctatcaatatgttgaaaaaaac
gaaaacaagtttaaatttcatgaaaacgctaaggatactctacaattgaaagaaaagaaaaatgataaccttggttttacgtttatgcacttccaacaaacgt
ataaagggattcctgtgtttggagcagtagtaactgcgcacgtgaaagatggcacgctgacggcgctatcagggacactgattccgaatttggacacga
aaggatccttaaaaagcgggaagaaattgagtgagaaacaagcgcgtgacattgctgaaaaagatttagtggcaaatgtaacaaaggaagtaccggaa
tatgaacagggaaaagacaccgagtttgttgtttatgtcaatggggacgaggcttctttagcgtacgttgtcaatttaaactttttaactcctgaaccaggaaa
ctggctgtatatcattgatgccgtagacggaaaaattttaaataaatttaaccaacttgacgccgcaaaaccaggtgacgtcaagtcgATAACAGG
AACATCAACTGTCGGAGTGGGAAGAGGAGTACTTGGTGATCAAAAAAATATTAATACAACC
TACTCTACGTACTACTATTTACAAGATAATACGCGTGGAAATGGGATTTTCACGTATGATGC
GAAATACCGTACGACATTGCCGGGAAGCTTATGGGCAGATGCAGATAACCAATTTTTTGCG
AGCTATGATGCTCCAGCGGTTGATGCTCATTATTACGCTGGTGTGACATATGACTACTATAA
AAATGTTCATAACCGTCTCAGTTACGACGGAAATAATGCAGCTATTAGATCATCCGTTCATT
ATAGCCAAGGCTATAATAACGCATTTTGGAACGGTTCGCAAATGGTGTATGGCGATGGTGA
TGGTCAAACATTTATTCCACTTTCTGGTGGTATTGATGTGGTCGCACATGAGTTAACGCATG
CGGTAACCGATTACACAGCCGGACTCATTTATCAAAACGAATCTGGTGCAATTAATGAGGC
AATATCTGATATTTTTGGAACGTTAGTCGAATTTTACGCTAACAAAAATCCAGATTGGGAAA
TTGGAGAGGATGTGTATACACCTGGTATTTCAGGGGATTCGCTCCGTTCGATGTCCGATCCG
GCAAAGTATGGTGATCCAGATCACTATTCAAAGCGCTATACAGGCACGCAAGATAATGGCG
GGGTTCATATCAATAGCGGAATTATCAACAAAGCCGCTTATTTGATTAGCCAAGGCGGTACG
CATTACGGTGTGAGTGTTGTCGGAATCGGACGCGATAAATTGGGGAAAATTTTCTATCGTGC
ATTAACGCAATATTTAACACCAACGTCCAACTTTAGCCAACTTCGTGCTGCCGCTGTTCAAT
CAGCCACTGACTTGTACGGTTCGACAAGCCAGGAAGTCGCTTCTGTGAAGCAGGCCTTTGAT
GCGGTAGGGGTGAAA

SEQ ID NO:2 sets forth the amino acid sequence of Thermolysin from expression plasmid pHPLT-ProteinaseT (the native signal sequence is shown in lower case letters, native propeptide in lower case, underlined text, and mature sequence in uppercase letters).

mkmkmklasfglaaglaaqvflpynalastehvtwnqqfqtpqfisgdllkvngtspeelvyqyveknenkfkfhenakdtlqlkekkndnlgft
fmhfqqtykgipvfgavvtahvkdgtltalsgtlipnldtkgslksgkklsekqardiaekdlvanvtkevpeyeqgkdtefvvyvngdeaslayvv
nlnfltpepgnwlyiidavdgkilnkfnqldaakpgdvksITGTSTVGVGRGVLGDQKNINTTYSTYYYLQDNTRGN
GIFTYDAKYRTTLPGSLWADADNQFFASYDAPAVDAHYYAGVTYDYYKNVHNRLSYDGNNA
AIRSSVHYSQGYNNAFWNGSQMVYGDGDGQTFIPLSGGIDVVAHELTHAVTDYTAGLIYQNES

GAINEAISDIFGTLVEFYANKNPDWEIGEDVYTPGISGDSLRSMSDPAKYGDPDHYSKRYTGTQD
NGGVHINSGIINKAAYLISQGGTHYGVSVVGIGRDKLGKIFYRALTQYLTPTSNFSQLRAAAVQS
ATDLYGSTSQEVASVKQAFDAVGVK

SEQ ID NO: 3 sets forth the amino acid sequence of the Thermolysin mature protein produced from pHPLT-ProteinaseT plasmid (316 residues):

ITGTSTVGVGRGVLGDQKNINTTYSTYYYLQDNTRGNGIFTYDAKYRTTL
PGSLWADADNQFFASYDAPAVDAHYYAGVTYDYYKNVHNRLSYDGNNAAI
RSSVHYSQGYNNAFWNGSQMVYGDGDGQTFIPLSGGIDVVAHELTHAVTD
YTAGLIYQNESGAINEAISDIFGTLVEFYANKNPDWEIGEDVYTPGISGD
SLRSMSDPAKYGDPDHYSKRYTGTQDNGGVHINSGIINKAAYLISQGGTH
YGVSVVGIGRDKLGKIFYRALTQYLTPTSNFSQLRAAAVQSATDLYGSTS
QEVASVKQAFDAVGVK

SEQ ID NO:4 sets forth the full-length amino acid sequence of the thermolysin from *Bacillus thermoproteolyticus* UniProtKB/Swiss-Prot Accession No. P00800

mkmkmklasfglaaglaaqvflpynalastehvtwnqqfqtpqfisgdllkvngtspeelvyqyveknenkfkfhenakdtlqlkekkndnlgft
fmrfqqtykgipvfgavvtshvkdgtltalsgtlipnldtkgslksgkklsekqardiaekdlvanvtkevpeyeqgkdtefvvyvngdeaslayvv
nlnfltpepgnwlyiidavdgkilnkfnqldaakpgdvksITGTSTVGVGRGVLGDQKNINTTYSTYYYLQDNTRGN
GIFTYDAKYRTTLPGSLWADADNQFFASYDAPAVDAHYYAGVTYDYYKNVHNRLSYDGNNA
AIRSSVHYSQGYNNAFWNGSQMVYGDGDGQTFIPLSGGIDVVAHELTHAVTDYTAGLIYQNES
GAINEAISDIFGTLVEFYANKNPDWEIGEDVYTPGISGDSLRSMSDPAKYGDPDHYSKRYTGTQD
NGGVHINSGIINKAAYLISQGGTHYGVSVVGIGRDKLGKIFYRALTQYLTPTSNFSQLRAAAVQS
ATDLYGSTSQEVASVKQAFDAVGVK

SEQ ID NO:5 sets forth the full-length amino acid sequence of the thermolysin from *Geobacillus caldoproteolyticus* (Chen et al (2004). Extremophiles 8:489-498, and described in WO2009058303).

mkmkmklasfglaaglaaqvflpynalastehvtwnqqfqtpqfisgdllkvngtspeelvyqyveknenkfkfhenakdtlqlkekkndnlgft
fmrfqqtykgipvfgavvtahvkdgtltalsgtlipnldtkgslksgkklsekqardiaekdlvanvtkevpeyeqgkdtefvvyvngdeaslayvv
nlnfltpepgnwlyiidavdgkilnkfnqldaakpgdvksITGTSTVGVGRGVLGDQKNINTTYSTYYYLQDNTRGN
GIFTYDAKYRTTLPGSLWADADNQFFASYDAPAVDAHYYAGVTYDYYKNVHNRLSYDGNNA
AIRSSVHYSQGYNNAFWNGSQMVYGDGDGQTFIPLSGGIDVVAHELTHAVTDYTAGLIYQNES
GAINEAISDIFGTLVEFYANKNPDWEIGEDVYTPGISGDSLRSMSDPAKYGDPDHYSKRYTGTQD
NGGVHINSGIINKAAYLISQGGTHYGVSVVGIGRDKLGKIFYRALTQYLTPTSNFSQLRAAAVQS
ATDLYGSTSQEVASVKQAFDAVGVK

### Production of Thermolysin Variants

The positional libraries for each of the 316 residues were constructed by BaseClear BV (Leiden, The Netherlands). The libraries consisted of transformed *B. subtilis* cells containing expression plasmids encoding Thermolysin variant sequences at the 316 positions of the mature protein. Each variant was confirmed by DNA sequencing analysis prior to protein activity evaluation. Individual clones were cultured as described below to obtain the different Thermolysin variants for functional characterization.

### Protein Expression

The *B. subtilis* transformants containing Thermolysin variants were cultured in 96 well plates for 16 hours in Tryptic Soy Broth (TSB) with 10 µg/ml neomycin, and 10 µl of this pre-culture was added to Corning 3599 MTP's filled with 190 µl of cultivation media (described below) supplemented with 10 µg/ml Neomycin. The plates were incubated for 22-26 hours at 37°C at 80% humidity with constant rotational mixing at 300 rpm. Cells were harvested by centrifugation at 2500 rpm for 10 minutes and filtered through Millipore Multiscreen filter plate using a Millipore vacuum system. After harvesting, propylene glycol was added to the culture supernatants to a final concentration of 10%, and these samples were used for assays. The cultivation media was an enriched semi-defined media based on phosphate buffer, glucose and maltodextrin as the main carbon sources, and supplemented with 0.2% soytone and 0.14% yeast extract for robust cell growth.

### EXAMPLE 3

### Identification of Combinable Mutations

Productive positions are described as those positions within a molecule that are most useful for making combinatorial variants exhibiting an improved characteristic, where the position itself allows for at least one combinable mutation. Combinable mutations can be described as those substitutions in a molecule that can be used to make combinatorial variants. Combinable mutations are ones that improve at least one desired property of the molecule, while not significantly decreasing either: expression, activity, or stability.

Combinable mutations are ones that improve at least one desired property of the molecule, while not significantly decreasing either: expression, activity, or stability. Combinable mutations in Thermolysin were determined using performance index (PI) values resulting from the assays described in Example 1: Abz-AGLA-Nba protease assay (activity), PAS-38 microswatch assay (activity), detergent stability and thermostability assays, and protein determination (expression).

In addition to Combinable mutations, a second group of mutations for thermolysin is Activity Combinable mutations. Activity Combinable mutations are ones that improve at least one activity property of the molecule, with a performance index greater than or equal to 1.5, while not decreasing either expression or stability PI values below 0.5.

Combinable mutations have been grouped according to the following criteria (summarized on **Table 3.1):**
A variant where the minimum performance indices (PI) relative to Thermolysin parent for PAS-38 microswatch cleaning at pH6 or pH8, activity on Abz-AGLA-Nba, detergent stability and thermostability are greater than or equal to 0.9, and in addition have a PI for any one of these tests that is greater than or equal to 1.0 **(Group A)**

A variant where the minimum performance indices (PI) relative to Thermolysin parent for PAS-38 microswatch cleaning at pH6 or pH8, activity on Abz-AGLA-Nba, detergent stability and thermostability are greater than or equal to 0.8, and in addition have a PI for any one of these tests that is greater than or equal to 1.2 **(Group B)**

A variant where the minimum performance indices (PI) relative to Thermolysin parent for PAS-38 microswatch cleaning at pH6 or pH8, activity on Abz-AGLA-Nba, detergent stability and thermostability are greater than or equal to 0.5, and in addition have a PI for any one of these tests that is greater than or equal to 1.5 **(Group C)**

**Table 3.1 Summary of criteria used for grouping the combinable mutations of Thermolysin variants**

| Group | Expression PI | Cleaning (at pH 6 or pH 8) PI | Synthetic substrate activity PI | Stability (detergent or thermal) PI | PI of at least X in one or more tests |
|---|---|---|---|---|---|
| A | ≥ 0.9 | ≥ 0.9 | ≥ 0.9 | ≥ 0.9 | X ≥ 1.0 |
| B | ≥ 0.8 | ≥ 0.8 | ≥ 0.8 | ≥ 0.8 | X ≥ 1.2 |
| C | ≥ 0.5 | ≥ 0.5 | ≥ 0.5 | ≥ 0.5 | X ≥ 1.5 |

Groups A, B, and C further contain amino acid positions that have differing degrees of tolerance for multiple substitutions. To identify productive positions, we measure the degree of substitutions tolerated at each position, and assign a Productivity Score to each position. The Productivity Score was assigned according to the percentage of substitutions (calculated based on all the tested variants) within each position that fall within groups A, B, or C, using the criteria set forth below.

Productive positions are defined as the positions which have shown a certain degree of tolerance for multiple substitutions, while at the same time meeting a set of criteria for combinability as set forth below.

The criteria to determine the Productivity Score for productive positions are as follows:
Positions where less than 15% of the substitutions at a given position fall within groups A, B, or C are given a Productivity Score of "**1**".
Positions where less than 40%, but greater than, or equal to 15% of the substitutions at a given position fall within groups A, B, or C are given a Productivity Score of "**2**".
Positions where less than 75%, but greater than, or equal to 40% of the substitutions at a given position fall within groups A, B, or C are given a Productivity Score of "**3**".
Positions where 75% or more of the substitutions at a given position fall within groups A, B, or C are given a Productivity Score of "**4**".

These amino acid substitutions are further assigned a Suitability Score based on the group(s) (A, B, C) where the substitution appears, and where a higher suitability score represents a substitution more suitable for use in making combinatorial variants. Suitability scores are defined in Table 3.2.

Table 3.2 defines each Suitability Score as it relates to groups of combinable mutations and productive positions.

| **Substitutions Occur in Group(s):** | **Suitability Score** |
|---|---|
| A, B and C | +++++ |
| A and B | ++++ |
| A or (B and C) | +++ |
| B | ++ |
| C | + |

**Table 3.3** shows the shows the productive positions in Thermolysin that fall within the previously described Productivity Score of "4" and the substitutions within those positions that are combinable. Position numbering based on mature Thermolysin protein listed in SEQ ID NO: 3.

**Table 3.3**

| **POS** | **Substitutions, WT 1ST** | **Productivity Score** |
|---|---|---|
| 2 | T,F,L,P,S,V,W,Y,Q,A,C,I,K,M | 4 |
| 26 | T,K,L,R,V,Y,W,F,G,H,I,M,C,D | 4 |
| 47 | R,A,C,H,K,N,D,E,G,L,M,Q,T | 4 |
| 49 | T,A,D,F,H,I,S,W,L,N,Q,V,E,M,Y | 4 |
| 53 | S,F,H,I,M,Q,T,W,K,R,A,N,V,C,L | 4 |
| 65 | S,I,M,Q,V,L,T,W,A,D,E,P,Y | 4 |
| 87 | V,D,E,G,I,S,P,R,T,C,K,L,M,N,Q,W,Y | 4 |
| 91 | L,D,E,F,K,M,P,Q,S,A,N,R,W,Y | 4 |
| 96 | N,C,D,I,V,F,T,G,H,Q,R,S,W,K,L,Y | 4 |
| 108 | Q,C,E,F,H,A,D,I,K,N,L,M | 4 |
| 118 | S,C,G,E,A,D,M,Q,R,T,V | 4 |
| 128 | Q,C,D,E,R,S,V,I,K,A,L,Y | 4 |
| 154 | G,L,Q,S,T,D,I,W,C,N,A,H,K,M,Y | 4 |
| 179 | Y,A,D,H,M,N,Q,S,T,W,F | 4 |
| 196 | G,D,E,T,K,R,V,H,L,Y,A,W | 4 |
| 197 | I,D,K,L,T,V,W,Y,A,H,N,E,Q,R,F,C | 4 |
| 198 | S,C,E,F,G,H,I,P,Q,T,V,M,N,R,W,A,K | 4 |
| 199 | G,C,E,F,H,Q,S,T,W,L,A,Y | 4 |
| 209 | A,D,E,L,S,T,V,G,I,K,P,R,Y,C,M | 4 |
| 211 | Y,A,C,D,F,G,H,I,L,N,Q,S,T,E,R | 4 |
| 217 | Y,Q,S,T,V,W,G,A,F,M,N,C,L | 4 |
| 219 | K,D,F,G,H,I,M,N,Q,T,A,E,R,S | 4 |
| 225 | Q,D,G,H,I,P,V,W,A,M,R,C,E,K,L,S | 4 |
| 232 | I,C,E,F,K,M,N,Q,W,G,L,R,S,T,V,Y | 4 |
| 256 | V,L,T,K,A,D,F,G,H,R,S,N | 4 |
| 257 | G,C,D,E,L,N,P,Q,S,T,Y,K,R | 4 |
| 259 | G,A,C,E,F,H,L,M,W,K,R,N,S,T | 4 |
| 261 | D,A,N,P,V,W,G,H,I,S | 4 |
| 265 | K,A,C,D,M,P,Q,S,G,I,L,R,N | 4 |
| 267 | F,E,G,N,S,V,W,A,C,H,I,K,L,M,T,Y | 4 |
| 272 | T,E,L,V,W,P,Y,C,F,N,Q,A,K | 4 |
| 276 | T,C,F,I,P,Q,W,H,A,L,V,Y | 4 |
| 277 | P,Q,S,T,E,F,G,H,N,R, V,W,A,D,Y | 4 |
| 286 | A,D,E,F,G,H,I,S,P,C,Q,R,T,K,L,M,N,Y | 4 |
| 289 | V,C,E,F,G,I,N,S,W,R,T,L,M,Y,A | 4 |
| 290 | Q,C,D,F,G,L,W,Y,R,T,V,A,H,N | 4 |
| 293 | T,C,E,F,G,H,Q,S,N,V,W,A,I,K,L,,M,Y | 4 |
| 295 | L,C,I,N,T,V,F,G,A,K,M,W | 4 |
| 298 | S,C,T,W,Y,E,N,P,A,G,K,M,R | 4 |
| 299 | T,C,F,L,M,R,W,P,D,Q,N,A,K | 4 |
| 300 | S,C,K,M,R,Y,I,L,H,P,V,W,A,G,T,D,N | 4 |
| 301 | Q,E,H,P,R,L,C,F,G,W,M,S,T,V,K | 4 |
| 303 | V,C,H,G,K,L,R,W,A,P,Y | 4 |
| 305 | S,G,I,L,N,W,Y,Q,H,T,V,A,K,M | 4 |
| 308 | Q,C,D,F,G,I,M,R,V,W,Y,A,L | 4 |
| 311 | D,C,E,F,G,I,Q,S,T,A,K,L,M,V,W,Y | 4 |
| 316 | K,D,E,F,G,H,L,N,P,Q,R,S,V,W,Y,A,M | 4 |

**Table 3.4** shows the shows the productive positions in Thermolysin that fall within the previously described Productivity Score of "3" and the substitutions within those positions that are combinable. Position numbering based on mature Thermolysin protein listed in SEQ ID NO: 3.

**Table 3.4**

| **POS** | **Substitutions, WT 1ST** | **Productivity Score** |
|---|---|---|
| 1 | I,K,M,V,A,H,W,Y,C,L | 3 |
| 4 | T,E,A,N,R,V,K,L,M,Y | 3 |
| 17 | Q,I,W,Y,C,R,V,T,L | 3 |
| 25 | S,D,F,A,C,K,M,R | 3 |
| 40 | F,E,G,M,Q,S,Y,W,A,K,L | 3 |
| 45 | K,E,L,S,F,H,Q,Y,A,G,M | 3 |
| 56 | A,K,Q,V,W,H,I,Y,E,M | 3 |
| 58 | A,N,Y,C,V,E,L | 3 |
| 61 | Q,M,R,W,F,V,C,I,L | 3 |
| 74 | H,E,L,V,C,F,M,N,Q,W | 3 |
| 86 | N,L,S,Y,A,C,E,F,G,K,D | 3 |
| 97 | N,K,C,R,S,Y,E,M | 3 |
| 101 | R,T,C,L,S,H | 3 |
| 109 | G,A,L,S,E,M,R,W | 3 |
| 149 | T,M,V,A,L,D,S,N | 3 |
| 150 | D,A,F,K,N,Q,T,V,S | 3 |
| 158 | Q,A,K,M,N,L,R,Y,S | 3 |
| 159 | N,R,W,A,C,G,M,T,S,Y | 3 |
| 172 | F,G,L,M,Q,S,V,W,Y,D,H | 3 |
| 181 | N,L,A,G,K,M,T,S | 3 |
| 214 | P,C,G,K,S,N,A,R | 3 |
| 216 | H,C,E,S,T,R,A | 3 |
| 218 | S,K,L,Y,F,G,T,V | 3 |
| 221 | Y,K,N,Q,R,S,T,V,A,F,G, M | 3 |
| 222 | T,C,D,L,Y,I,V,A,M,K | 3 |
| 224 | T,K,M,F,L,P,Q,V,Y,E,H | 3 |
| 250 | H,A,C,K,M,N,P,Q,R,V,Y | 3 |
| 253 | V,N,T,I,R,Y,M,Q | 3 |
| 254 | S,A,M,R,Y,K,L,N,V,W | 3 |
| 258 | I,E,L,M,N,R,S,A,C,K,Q, V | 3 |
| 263 | L,C,I,Q,T,H,K,N,V,A,M | 3 |
| 264 | G,C,R,A,N,P,Q,S,T | 3 |
| 266 | I,A,F,L,S,C,M,T,V | 3 |
| 268 | Y,M,Q,V,A,S,K | 3 |
| 271 | L,A,D,F,I,N,Y,H | 3 |
| 273 | Q,A,H,Y,C,S,W,E,G,N | 3 |
| 275 | L,I,M,V,C,Q,S,T | 3 |
| 278 | T,G,K,R,Y,C,H,M,N,Q,S | 3 |
| 279 | S,A,D,I,L,M,N,Q,T,G | 3 |
| 280 | N,A,C,D,E,G,Q,H,T | 3 |
| 282 | S,K,N,R,A,H,L,M,T | 3 |
| 283 | Q,K,L,P,R,W,Y,S | 3 |
| 287 | A,I,L,N,V,Y,K,R,T,D,C | 3 |
| 288 | A,C,I,S,T,V,Y,N,L,M | 3 |
| 291 | S,E,I,L,M,N,V,A,T | 3 |
| 297 | G,A,M,R,Y,C,F,K,T,D,N | 3 |
| 302 | E,K,L,G,T,V,D,Q,A | 3 |
| 304 | A,C,D,L,N,R,S,T,W,E,K, Y | 3 |
| 307 | K,A,C,G,I,M,N,Q,R,W,Y, H | 3 |
| 312 | A,G,M,V,L,N,R,T,C | 3 |

**Table 3.5** shows the shows the productive positions in Thermolysin that fall within the previously described Productivity Score of "2" and the substitutions within those positions that are combinable. Position numbering based on mature Thermolysin protein listed in SEQ ID NO: 3.

**Table 3.5**

| **POS** | **Substitutions, WT 1ST** | **Productivity Score** |
|---|---|---|
| 5 | S,D,N,P,H,L | 2 |
| 9 | V,L,T,I | 2 |
| 11 | R,I,Y,K | 2 |
| 19 | N,L,Y,K,S | 2 |
| 27 | Y,W,A,M,V,C,L | 2 |
| 31 | Q,A,K,V,I,C,Y | 2 |
| 33 | N,S,T,K,A,C,L,M | 2 |
| 37 | N,D,Q,R,L,K | 2 |
| 46 | Y,L,H,N,C | 2 |
| 64 | A,H,Q,T,D,E | 2 |
| 73 | A,I,F,L,M,W | 2 |
| 76 | Y,H,L,M,Q,T | 2 |
| 79 | V,L,Q,T,A,N,S | 2 |
| 80 | T,I,D,A,L,N | 2 |
| 85 | K,E,A,L,N,R,S | 2 |
| 89 | N,L,M,H | 2 |
| 95 | G,A,D,H,M,N,S | 2 |
| 98 | A,C,E,H,R,Y,K,V | 2 |
| 99 | A,E,K,P,R,S | 2 |
| 107 | S,D,K,Y,A,G | 2 |
| 127 | G,C,D,E | 2 |
| 129 | T,I,R,E,Y,L,M | 2 |
| 131 | I,Y,W,L | 2 |
| 137 | I,P,A,E,T,V,L | 2 |
| 141 | A,S,C,G | 2 |
| 145 | T,A,C,E,G,M,N,Q | 2 |
| 148 | V,L,N,Y,M,A,Q | 2 |
| 151 | Y,K,G,H,S,W | 2 |
| 152 | T,S,L,M,G | 2 |
| 155 | L,C,I,M | 2 |
| 156 | I,M,T,L,Q | 2 |
| 160 | E,L,Y,Q | 2 |
| 161 | S,A,N,P,T | 2 |
| 164 | I,L,N,S,T,V,C,A | 2 |
| 168 | I,A,M,T,L | 2 |
| 171 | I,C,E,F,L,S,G | 2 |
| 176 | V,L,N,C | 2 |
| 180 | A,E,G,K,T,S | 2 |
| 182 | K,L,A,W | 2 |
| 187 | E,L,D | 2 |
| 188 | I,L,V | 2 |
| 205 | M,L,A,V,Q | 2 |
| 206 | S,A,C,K,L,M,R | 2 |
| 207 | D,A,H,N | 2 |
| 210 | K,I,L,V | 2 |
| 212 | G,Y,A,D,Q | 2 |
| 213 | D,N,S,L,A,G,W | 2 |
| 220 | R,K,V,A | 2 |
| 227 | N,D,L,Y,A | 2 |
| 234 | S,D,N,A,C | 2 |
| 235 | G,M,C,Q,S,A | 2 |
| 236 | I,M,A,C | 2 |
| 237 | I,N,F,M | 2 |
| 242 | Y,C,F,N,V | 2 |
| 244 | I,T,V,F,A,M,L | 2 |
| 246 | Q,E,N,T,L,C,D | 2 |
| 248 | G,A,E,S | 2 |
| 249 | T,K,M,N,L,Y,P | 2 |
| 252 | G,K,Y,A,S,T,W | 2 |
| 255 | V,L,P,A,Y,M,N | 2 |
| 270 | A, C,F,I,L,S,G | 2 |
| 274 | Y,F,H,A,C,Q,T,M | 2 |
| 284 | L,V,W,A,M,Y | 2 |
| 294 | D,A,V,Q,N | 2 |
| 296 | Y,N,L,R,H,W,M | 2 |
| 306 | V,A,S,F,I,L,T | 2 |
| 309 | A,G,S,T,V,C | 2 |
| 310 | F,A,C,W,M | 2 |
| 313 | V,T,A,G,L,I,C | 2 |
| 314 | G,A,E,H,M,S,W,Q | 2 |
| 315 | V,A,C,I,M,L,T | 2 |

**Table 3.6** shows the shows the productive positions in Thermolysin that fall within the previously described Productivity Score of "1" and the substitutions within those positions that are combinable. Position numbering based on mature Thermolysin protein listed in SEQ ID NO: 3.

**Table 3.6**

| **POS** | **Substitutions, WT 1ST** | **Productivity Score** |
|---|---|---|
| 3 | G,Y | 1 |
| 6 | T,C,V | 1 |
| 7 | V,L,I | 1 |
| 20 | I,L,V | 1 |
| 23 | T,F,W | 1 |
| 24 | Y,W | 1 |
| 44 | A,C | 1 |
| 48 | T,E,D | 1 |
| 50 | L,P | 1 |
| 57 | D,K | 1 |
| 63 | F,Y,C | 1 |
| 72 | D,F,W | 1 |
| 75 | Y,A | 1 |
| 81 | Y,F | 1 |
| 92 | S,L | 1 |
| 93 | Y,T,C | 1 |
| 94 | D,T | 1 |
| 100 | I,L,V | 1 |
| 102 | S,G,N | 1 |
| 103 | S,T | 1 |
| 104 | V,A | 1 |
| 110 | Y,L | 1 |
| 117 | G,H | 1 |
| 120 | M,L | 1 |
| 134 | S,A,P | 1 |
| 135 | G,A | 1 |
| 136 | G,A,S | 1 |
| 140 | V,D | 1 |
| 144 | L,T | 1 |
| 153 | A,T | 1 |
| 173 | G,A,C | 1 |
| 174 | T,C,A | 1 |
| 175 | L,H,S | 1 |
| 178 | F,H,Y | 1 |
| 183 | N,S | 1 |
| 185 | D,E | 1 |
| 189 | G,A | 1 |
| 193 | Y,F | 1 |
| 201 | S,C,A | 1 |
| 223 | G,D,K | 1 |
| 230 | V,A | 1 |
| 238 | N,L,M | 1 |
| 239 | K,A | 1 |
| 241 | A,L,S | 1 |
| 247 | G,A,S | 1 |
| 251 | Y,M | 1 |
| 260 | R,A,N | 1 |
| 262 | K,A | 1 |
| 269 | R,V,K | 1 |
| 285 | R,K,Y | 1 |

### EXAMPLE 4

### Combinable Mutations and Suitability Scores

As shown in Example 3, combinable mutations in thermolysin were determined using performance index (PI) values resulting from the assays described in Example 1.

Combinable mutations were assigned to groups A, B or C according to criteria set forth in Example 3. These substitutions are further assigned a Suitability Score based on the group(s) (A, B, C) where the substitution appears, and where a higher suitability score represents a substitution more suitable for use in making combinatorial variants. Suitability scores are defined in Table 4.1. Suitability scores for individual substitutions of thermolysin that fit the above criteria are reported below.

Table 4.1 defines each Suitability Score as it relates to groups of combinable mutations and productive positions.

| **Substitutions Occur in Group(s):** | **Suitability Score** |
|---|---|
| A, B and C | +++++ |
| A and B | ++++ |
| A or (B and C) | +++ |
| B | ++ |
| C | + |

Variants with suitability score +++++:
I001L, T002A, T002C, T002I, T002K, T002M, T004K, T004L, T004M, T004Y, Q017L, N037K, F040K, F040L, K045A, K045G, K045M, T049E, T049M, T049Y, L050P, S053C, S053L, A056M, A058E, A058L, Q061L, F063C, A064D, A064E, S065A, S065D, S065E, S065P, S065Y, V087C, V087K, V087L, V087M, V087N, V087Q, V087W, V087Y, N096K, N096L, N096Y, R101H, Q108L, Q108M, G109E, G109M, G109R, G109W, S118A, S118D, S118M, S118Q, S118R, S118T, S118V, Q128A, Q128L, Q128Y, I131L, I137L, T149N, G154A, G154H, G154K, G154M, G154Y, L155M, I164A, N181S, G196A, G196W, I197C, S198A, S198K, G199A, G199Y, A209C, A209M, H216A, Y217C, Y217L, T222K, N227A, I244L, Q246D, V256N, L263A, L263M, T272K, Q273N, Y274M, P277A, P277D, P277Y, L284A, L284M, L284Y, A286K, A286L, A286M, A286N, A286Y, A287C, A288L, A288M, V289A, S291A, S291T, T293A, T293I, T293K, T293L, T293M, T293Y, L295A, L295K, L295M, L295W, Y296M, G297N, S298A, S298G, S298K, S298M, S298R, T299A, T299K, S300D, S300N, Q301K, E302A, V303A, V303P, V303Y, A304E, A304K, A304Y, S305A, S305K, S305M, V306L, V306T, A309C, F310M, D311A, D311K, D311L, D311M, D311V, D311W, D311Y, and A312C

Variants with suitability score ++++:
T002Q, T004V, V007I, V009I, R011K, I020L, I020V, S025A, S025C, S025K, S025M, S025R, T026C, T026D, Y027C, Y027L, N037L, F040A, A044C, K045F, K045H, K045Q, K045Y, Y046C, R047D, R047E, R047G, R047L, R047M, R047Q, R047T, T049L, T049N, T049Q, T049V, S053A, S053N, S053V, A056E, Q061C, Q061I, A064T, S065L, S065T, S065W, A073F, A073L, A073M, A073W, H074C, H074F, H074M, H074N, H074Q, H074W, T080L, T080N, K085S, N086D, V087R, V087T, L091A, L091N, L091R, L091W, L091Y, S092L, Y093C, N096G, N096H, N096Q, N096R, N096S, N096W, N097E, N097M, A099R, A099S, R101C, R101L, R101S, S102N, S107G, Q108I, Q108K, Q108N, G109S, S118E, M120L, Q128I, Q128K, T129L, T129M, I131W, S134P, G136S, I137E, I137T, I137V, V140D, V148A, V148Q, T149D, T149S, T152G, G154C, G154N, L155I, N159S, N159Y, I164C, I168L, I171G, Y179F, A180S, G189A, Y193F, G196H, G196L, G196Y, I197F, S198M, S198N, S198R, S198W, S201A, A209G, A209I, A209K, A209P, A209R, A209Y, Y211E, Y211R, P214A, P214R, Y217A, Y217F, Y217M, Y217N, K219A, K219E, K219R, K219S, R220A, Y221A, Y221F, Y221G, Y221M, T222A, T222M, Q225C, Q225E, Q225K, Q225L, Q225S, I232L, I232R, I232S, I232T, I232V, I232Y, S234A, S234C, G235A, I236C, I244A, I244M, Q246C, V256S, G257K, G257R, I258A, I258C, I258K, I258Q, I258V, G259N, G259S, G259T, L263H, L263K, L263N, L263V, G264A, G264N, G264P, G264Q, G264S, G264T, K265N, I266C, I266M, I266T, I266V, F267A, F267C, F267H, F267I, F267K, F267L, F267M, F267T, F267Y, R269K, A270G, L271H, T272A, Q273E, Q273G, L275C, L275Q, L275S, L275T, T276A, T276L, T276V, T276Y, P277E, P277F, P277G, P277H, P277N, P277R, P277V, P277W, S279G, R285Y, A286C, A286Q, A286R, A286T, A288N, V289L, V289M, V289Y, Q290A, Q290H, Q290N, S291V, T293N, T293V, T293W, D294N, L295F, L295G, Y296W, G297D, S298E, S298N, S298P, T299N, S300A, S300G, S300T, Q301M, Q301S, Q301T, Q301V, E302D, E302Q, V303G, V303K, V303L, V303R, V303W, A304R, A304S, A304T, A304W, S305H, S305T, S305V, V306I, Q308A, Q308L, F310C, F310W, D311F, D311G, D311I, D311Q, D311S, D311T, V313C, G314Q, V315L, V315T, K316A, and K316M

Variants with suitability score +++:
I001K, I001M, I001V, T002F, T002L, T002P, T002S, T002V, T002W, T002Y, T004E, S005D, S005N, S005P, T006C, R011I, Q017I, Q017W, Q017Y, S025D, S025F, T026K, T026L, T026R, T026V, T026Y, Y027W, Q031A, Q031K, Q031V, N033S, N033T, N037D, N037Q, N037R, F040E, F040G, F040M, F040Q, F040S, F040Y, K045E, K045L, K045S, Y046L, R047A, R047C, R047H, R047K, R047N, T048E, T049A, T049D, T049F, T049H, T049I, T049S, S053F, S053H, S053I, S053M, S053Q, S053T, S053W, A056K, A056Q, A056V, A056W, Q061M, S065I, S065M, S065Q, S065V, D072F, H074E, H074L, Y076H, Y076L, Y076M, Y076Q, V079L, V079Q, V079T, T080I, Y081F, K085E, N086L, N086S, V087D, V087E, V087G, V087I, V087S, L091D, L091E, L091F, L091K, L091M, L091P, L091Q, L091S, Y093T, G095A, G095D, G095H, G095M, G095N, G095S, N096C, N096D, N096I, N096V, N097K, A098C, A098E, A098H, A098R, A099E, A099K, A099P, S107D, Q108C, Q108E, Q108F, Q108H, G127C, G127D, G127E, Q128C, Q128D, Q128E, Q128R, Q128S, T129I, T129R, S134A, I137P, A141S, T145A, T145C, T145E, T145G, T145M, T145N, T145Q, V148L, V148N, V148Y, T149M, T149V, Y151K, T152S, A153T, G154L, G154Q, G154S, G154T, L155C, Q158A, Q158K, Q158M, Q158N, N159R, N159W, S161A, S161N, S161P, S161T, I164L, I164N, I164S, I164T, I164V, I171C, I171E, I171F, I171L, I171S, F172G, F172L, F172M, F172Q, F172S, F172V, F172W, F172Y, G173A, G173C, T174C, V176L, V176N, N181L, G196D, G196E, G196T, I197D, I197K, I197L, I197T, I197V, I197W, I197Y, S198C, S198E, S198F, S198G, S198H, S198I, S198P, S198Q, S198T, S198V, G199C, G199E, G199F, G199H, G199Q, G199S, G199T, G199W, M205L, A209D, A209E, A209L, A209S, A209T, A209V, Y211A, Y211C, Y211D, Y211F, Y211G, Y211H, Y211I, Y211L, Y211N, Y211Q, Y211S, Y211T, D213N, D213S, P214C, P214G, P214K, P214S, H216C, H216E, H216S, H216T, Y217Q, Y217S, Y217T, Y217V, Y217W, S218K, S218L, S218Y, K219D, K219F, K219G, K219H, K219I, K219M, K219N, K219Q, K219T, R220K, R220V, Y221K, Y221N, Y221Q, Y221R, Y221S, Y221T, Y221V, T222C, T222D, T222L, T222Y, T224K, T224M, Q225D, Q225G, Q225H, Q225I, Q225P, Q225V, Q225W, I232C, I232E, I232F, I232K, I232M, I232N, I232Q, I232W, S234D, G235M, I236M, Y242C, Y242F, Y242N, Y242V, I244T, I244V, Q246E, Q246N, Q246T, G247A, G247S, T249K, T249M, T249N, H250A, H250C, G252K, G252Y, V253N, V253T, S254A, S254M, S254R, S254Y, V255L, V255P, V256L, V256T, G257C, G257D, G257E, G257L, G257N, G257P, G257Q, G257S, G257T, G257Y, I258E, I258L, I258M, I258N, G259A, G259C, G259E, G259F, G259H, G259L, G259M, G259W, D261A, D261N, L263C, L263I, L263Q, L263T, K265A, K265C, K265D, K265M, K265P, K265Q, K265S, I266A, I266F, I266L, I266S, F267E, F267G, F267N, F267S, F267V, F267W, Y268M, Y268Q, Y268V, A270C, A270F, A270I, A270L, A270S, L271A, L271D, L271F, L271I, T272E, T272L, T272V, T272W, Q273A, Q273H, Q273Y, Y274F, Y274H, L275I, L275M, L275V, T276C, T276F, T276I, T276P, T276Q, T276W, P277Q, P277S, P277T, T278G, S279A, S279D, S279I, S279L, S279M, S279N, S279Q, S279T, N280A, N280C, N280D, N280E, S282K, S282N, L284V, L284W, R285K, A286D, A286E, A286F, A286G, A286H, A286I, A286S, A287I, A287L, A287N, A287V, A287Y, A288C, A288I, A288S, A288T, A288V, V289C, V289E, V289F, V289G, V289I, V289N, V289S, V289W, Q290C, Q290D, Q290F, Q290G, Q290L, Q290W, S291E, T293C, T293E, T293F, T293G, T293H, T293Q, T293S, L295C, L295I, L295N, Y296N, G297A, G297M, G297R, G297Y, S298C, S298T, S298W, S298Y, T299C, T299F, T299L, T299M, T299R, T299W, S300C, S300K, S300M, S300R, S300Y, Q301E, Q301H, Q301P, Q301R, V303C, V303H, A304C, A304D, A304L, A304N, S305G, S305I, S305L, S305N, S305W, S305Y, V306A, V306S, K307A, K307C, K307G, K307I, K307M, K307N, K307Q, K307R, K307W, K307Y, Q308C, Q308D, Q308F, Q308G, Q308I, Q308M, A309G, A309S, D311C, D311E, A312G, A312M, A312V, V313T, G314A, G314E, G314H, G314M, G314S, G314W, V315A, V315C, V315I, V315M, K316D, K316E, K316F, K316G, K316H, K316L, K316N, K316P, K316Q, K316R, K316S, K316V, K316W, and K316Y

Variants with suitability score ++:
1001C, T004R, T006V, Q017T, N019K, N019S, T023F, T023W, Y024W, T026F, T026G, T026H, T026I, T026M, Y027M, Y027V, Q031C, Q031Y, N033A, N033C, N033L, N033M, Y046H, Y046N, T048D, T049W, A058C, A058V, Q061F, Q061V, A064H, A064Q, D072W, A073I, H074V, Y076T, V079S, T080A, K085A, K085L, K085N, K085R, N086A, N086C, N086E, N086F, N086G, N086K, N089H, N096F, N096T, N097C, N097R, N097S, N097Y, A098K, A098V, I100L, I100V, R101T, S102G, S103T, S107A, Q108D, G117H, S118G, Q128V, T129Y, G136A, A141G, L144T, V148M, D150S, Y151G, Y151H, Y151S, Y151W, G154D, G154I, G154W, I156L, I156Q, Q158S, N159A, N159C, N159G, N159M, N159T, E160Q, I168A, I168M, I168T, F172D, F172H, L175S, V176C, F178H, F178Y, Y179A, Y179D, Y179H, Y179M, Y179N, Y179Q, Y179S, Y179T, Y179W, A180E, A180G, A180K, A180T, N181G, N181K, N181M, N181T, K182A, K182W, N183S, D185E, E187D, I188V, G196K, G196R, G196V, I197E, I197Q, I197R, G199L, S201C, M205Q, S206M, S206R, D207H, D207N, K210I, K210L, K210V, G212A, G212D, G212Q, D213A, D213G, D213W, P214N, Y217G, S218G, S218T, S218V, T222I, T222V, G223D, G223K, T224E, T224H, Q225A, Q225M, Q225R, V230A, I232G, S234N, G235C, G235Q, G235S, I237F, I237M, I244F, G248A, G248E, G248S, T249P, Y251M, G252A, G252S, G252T, G252W, V253M, V253Q, S254N, S254V, S254W, V255M, V255N, V256A, V256D, V256F, V256G, V256H, V256R, I258R, I258S, G259K, G259R, R260A, R260N, D261G, D261H, D261I, D261S, G264C, G264R, K265G, K265I, K265L, K265R, Y268K, L271N, L271Y, T272C, T272F, T272N, T272Q, Y274C, Y274Q, Y274T, T276H, T278C, T278H, T278M, T278N, T278Q, T278S, N280H, N280T, S282A, S282H, S282L, S282M, S282T, Q283S, A286P, A287D, A288Y, V289R, V289T, Q290R, Q290T, Q290V, D294Q, L295T, L295V, Y296H, G297C, G297F, G297K, G297T, T299D, T299Q, S300H, S300P, S300V, S300W, Q301C, Q301F, Q301G, Q301W, E302G, E302T, E302V, S305Q, V306F, K307H, Q308R, Q308V, Q308W, Q308Y, A309T, A309V, A312T, and V3131

Variants with suitability +:
I001A, I001H, I001W, I001Y, G003Y, T004A, T004N, S005H, S005L, V007L, V009L, V009T, R011Y, Q017C, Q017R, Q017V, N019L, N019Y, T026W, Y027A, Q031I, N033K, F040W, S053K, S053R, A056H, A056I, A056Y, D057K, A058N, A058Y, Q061R, Q061W, F063Y, Y075A, V079A, V079N, T080D, N086Y, V087P, N089L, N089M, D094T, A098Y, V104A, S107K, S107Y, Q108A, G109A, G109L, Y110L, S118C, T129E, I131Y, G135A, I137A, A141C, T149A, T149L, D150A, D150F, D150K, D150N, D150Q, D150T, D150V, T152L, T152M, I156M, I156T, Q158L, Q158R, Q158Y, E160L, E160Y, T174A, L175H, N181A, K182L, E187L, I188L, I197A, I197H, I197N, M205A, M205V, S206A, S206C, S206K, S206L, D207A, G212Y, D213L, H216R, S218F, T224F, T224L, T224P, T224Q, T224V, T224Y, N227D, N227L, N227Y, I236A, I237N, N238L, N238M, K239A, A241L, A241S, Q246L, T249L, T249Y, H250K, H250M, H250N, H250P, H250Q, H250R, H250V, H250Y, V253I, V253R, V253Y, S254K, S254L, V255A, V255Y, V256K, D261P, D261V, D261W, K262A, Y268A, Y268S, R269V, T272P, T272Y, Q273C, Q273S, Q273W, Y274A, T278K, T278R, T278Y, N280G, N280Q, S282R, Q283K, Q283L, Q283P, Q283R, Q283W, Q283Y, A287K, A287R, A287T, Q290Y, S291I, S291L, S291M, S291N, D294A, D294V, Y296L, Y296R, T299P, S300I, S300L, Q301L, E302K, E302L, F310A, A312L, A312N, A312R, V313A, V313G, and V313L

**Table 4.2** shows the Activity combinable variants in Thermolysin. Position numbering is based on mature Thermolysin protein listed in SEQ ID NO: 3.

| **POS** | **Variants** |
|---|---|
| 17 | E,F,P |
| 19 | A,D,H,I,R,T,V |
| 24 | F,H |
| 25 | H |
| 31 | L |
| 33 | Q |
| 40 | C |
| 48 | A,R |
| 73 | Y |
| 79 | C |
| 80 | C,R |
| 81 | H |
| 85 | C,M,Y |
| 86 | V |
| 89 | K,R,T,V |
| 94 | E |
| 109 | D |
| 117 | A,K,R,T |
| 140 | S |
| 141 | T |
| 150 | E,M,W |
| 151 | A,C,E,I |
| 152 | D |
| 153 | V |
| 156 | H,R |
| 158 | F,G,I,V |
| 159 | F,I,K |
| 160 | S |
| 161 | Y |
| 168 | N |
| 171 | D |
| 174 | S,V |
| 175 | C,E,F,G,I |
| 176 | E,Q |
| 178 | C,M |
| 180 | L,W |
| 181 | Y |
| 182 | F,R |
| 183 | H,I,L,M,Q,R,T |
| 189 | C |
| 205 | C,F |
| 206 | F,H,I,T,V,Y |
| 207 | T |
| 210 | A,E,F,G,H,T |
| 212 | F,H,K,M,N,R,S,T |
| 213 | I,K,R,V,Y |
| 214 | Q |
| 218 | R |
| 223 | Y |
| 224 | I,R |
| 227 | C,E,G,K,Q,R,S,T,V |
| 235 | D,L,T |
| 236 | P |
| 237 | A,Q |
| 238 | A,C,D,E,R,S |
| 239 | C,G,H,L,Q,R,S,V,Y |
| 241 | E,F,G,I,T,V |
| 244 | Q |
| 246 | K,R |
| 248 | C,H |
| 249 | G,V |
| 250 | F,S |
| 251 | H |
| 252 | F,I,L |
| 253 | A,D,E,P |
| 254 | C,F,G,H,I,P |
| 255 | F,Q |
| 258 | F |
| 259 | I |
| 260 | C,D,I |
| 261 | K,R,T |
| 262 | C,F,H,L,P,R |
| 266 | W |
| 268 | F,R |
| 269 | P,T,W,Y |
| 270 | M,N,P,V |
| 271 | V |
| 272 | R |
| 273 | R |
| 274 | D,E |
| 276 | G,S |
| 278 | V |
| 279 | E |
| 280 | P,R,V |
| 282 | P |
| 283 | A,C,E,G,H,T,V |
| 294 | T |
| 295 | R |
| 296 | E,I |
| 297 | I,V |
| 300 | Q |
| 302 | W |
| 306 | Y |
| 310 | I,N |
| 312 | Q |

Further listed are selected productive position variants of Thermolysin. Position numbering is based on mature Thermolysin protein listed in SEQ ID NO: 3. T002I, T002M, T048E, A058L, F063C, V087L, N096H, Q128Y, Y151R, A180E, S198A, I244T, Q273N, P277R, T278R, Q283E, T293L, T293N, L295F, S298A, Q301I, N019D, S025A, T026R, T049K, T049Q, F063L, S065A, S065T, L091M, N096Q, N096R, N096Y, N097K, R101M, G109A, S118A, I131L, V140D, Q158A, N159E, N159K, L175V, A180R, G196H, G196T, G196Y, K219S, Q225E, I232R, I244L, Q246D, D261N, P277G, T293Y, S300G, Q301F, Q301M, V303R, S305A, D311A.

### EXAMPLE 5

### Identification of thermolysin homologs

### A. Identification of related molecules in MEROPS database of proteases

Thermolysin of *Bacillus* thermoproteolyticus is classified under Family M4 (M for metalloprotease) in the MEROPS protease database (http://merops.sanger.ac.uk). Thermolysin is the prototype for the M4 family (thermolysin family) of metalloproteases and the type-example of clan MA. It is further classified into subbclan MA(E) also known as Glu-Zincins, because the third zinc ligand is a glutamate. Thermolysin of *Bacillus thermoproteolyticus* was assigned Merops accession number MER001026.

The *MEROPS* database uses a hierarchical, structure-based classification of the peptidases (proteases). In this, each peptidase is assigned to a Family on the basis of statistically significant similarities in amino acid sequence, and families that are thought to be homologous are grouped together in a Clan. The classification of peptidases by molecular structure and homology was developed in the 1990s because it depends on the availability of data for amino acid sequences and three-dimensional structures in quantities that were realized then. In 1993, Rawlings & Barrett described a system in which individual peptidases were assigned to families, and the families were grouped in clans (Rawlings, N.D. & Barrett, A.J. (1993) Evolutionary families of peptidases. Biochem J 290, 205-218).

All peptidases in the M4 family bind a single, catalytic zinc ion. As in many other families of metallopeptidases, there is an HEXXH motif, in which the histidines are zinc ligands and the glutamate is an active site residue. Most members of this family are endopeptidases active at neutral pH and are almost exclusively from bacteria, and thermostability has been attributed to binding of calcium ions. Proteins and peptides are degraded with a preference for cleavage of Xaa+Yaa, in which Xaa is a hydrophobic residue and Yaa is Leu, Phe, Ile, or Val. Thermolysin has a two-domain structure with the active site between the domains. The N-terminal domain includes a distinctive six-strand beta sheet with two helices, one of which carries the HEXXH zinc-binding motif. The C-terminal domain, which is unique for the family, is predominantly helical and carries the third zinc ligand.

A BLAST search for homologs of mature thermolysin protein (SEQ ID NO: 3) within MEROPS (version 9.5) yielded the results shown below (**Table 5.1**). Each enzyme is listed by MEROPS database unique accession number, gene origin and shows percent identity calculated by the program.

**Table 5.1. MEROPs database output for members of the M4 family of metalloproteases, which includes thermolysin.**

| **MEROPS ID #** | **Origin** | **% ID** |
|---|---|---|
| MER001026 | - thermolysin (Bacillus thermoproteolyticus) | 100.00% |
| MER001027 | - thermolysin (Geobacillus stearothermophilus) | 100.00% |
| MER212338 | - thermolysin (Geobacillus sp. C56-T3) | 87.13% |
| MER168133 | - thermolysin (Geobacillus sp. Y412MC61) | 87.13% |
| MER001353 | - thermolysin (Alicyclobacillus acidocaldarius) | 86.13 |
| MER001927 | - thermolysin (Bacillus sp.) | 87.13 |
| MER234417 | - thermolysin (Geobacillus sp. Y412MC52) | 87.13% |
| MER001034 | - thermolysin (Bacillus caldolyticus) | 86.80 |
| MER001025 | - stearolysin (Geobacillus stearothermophilus) | 86.14 |
| MER040474 | - thermolysin (Geobacillus kaustophilus) | 87.76% |
| MER109364 | - stearolysin (Bacillus sp. SG-1) | 74.75% |
| MER187808 | - thermolysin (Bacillus cereus) | 73.42% |
| MER176709 | - thermolysin (Bacillus pseudomycoides) | 73.75% |
| MER003181 | - thermolysin (Bacillus thuringiensis) | 73.75% |
| MER061817 | - thermolysin (Bacillus cereus) | 73.42% |
| MER001031 | - thermolysin (Bacillus megaterium) | 73.18% |
| MER001030 | - thermolysin (Bacillus cereus) | 73.75% |
| MER001354 | - thermolysin (Lactobacillus sp.) | 72.76% |
| MER187798 | - thermolysin (Bacillus mycoides) | 73.75% |
| MER187790 | - thermolysin (Bacillus pseudomycoides) | 72.76% |
| MER021824 | - thermolysin (Bacillus anthracis) | 72.76% |
| MER109427 | - thermolysin (Bacillus sp. SG-1) | 72.88% |
| MER109389 | - thermolysin (Bacillus weihenstephanensis) | 73.42% |
| MER187794 | - thermolysin (Bacillus mycoides) | 72.43% |
| MER091675 | - thermolysin (Exiguobacterium sibiricum) | 70.61% |
| MER124526 | - thermolysin (Exiguobacterium sp. AT1b) | 68.90% |
| MER001028 | - thermolysin (Brevibacillus brevis) | 67.55% |
| MER169677 | - thermolysin (Brevibacillus brevis) | 63.04% |
| MER187793 | - thermolysin (Bacillus pseudomycoides) | 61.24% |
| MER187797 | - thermolysin (Bacillus mycoides) | 60.91% |
| MER187765 | - family M4 unassigned peptidases (Paenibacillus larvae) | 59.67% |
| MER001033 | - family M4 unassigned peptidases (Paenibacillus polymyxa) | 56.62% |
| MER001029 | - neutral peptidase B (Bacillus subtilis) | 54.05% |
| MER187796 | - neutral peptidase B (Bacillus mycoides) | 55.26% |
| MER187792 | - neutral peptidase B (Bacillus pseudomycoides) | 55.26% |
| MER038281 | - family M4 unassigned peptidases (Bacillus vietnamensis) | 57.89% |
| MER091650 | - family M4 unassigned peptidases (Herpetosiphon aurantiacus) | 56.90% |
| MER084165 | - family M4 unassigned peptidases (Bacillus cereus) | 55.59% |
| MER187771 | - family M4 unassigned peptidases (Bacillus coahuilensis) | 57.05% |
| MER151875 | - neutral peptidase B (Bacillus cereus) | 54.93% |
| MER187800 | - neutral peptidase B (Bacillus mycoides) | 53.95% |
| MER028887 | - neutral peptidase B (Bacillus cereus) | 54.05% |
| MER084215 | - neutral peptidase B (Bacillus weihenstephanensis) | 53.62% |
| MER187810 | - neutral peptidase B (Bacillus cereus) | 53.95% |
| MER039810 | - neutral peptidase B (Bacillus thuringiensis) | 54.28% |
| MER062589 | M4 unassigned peptidases (Bacillus sp. NRRL B-14911) | 56.62% |
| MER021804 | - neutral peptidase B (Bacillus anthracis) | 54.61% |
| MER109478 | M4 unassigned peptidases (Bacillus sp. SG-1) | 55.45% |
| MER187779 | - neutral peptidase B (Bacillus thuringiensis) | 52.98% |
| MER187806 | - neutral peptidase B (Bacillus cereus) | 52.63% |
| MER168882 | - thermolysin (Paenibacillus larvae) | 53.33% |
| MER062591 | - family M4 unassigned peptidases (Bacillus cereus) | 52.72% |
| MER187770 | - family M4 unassigned peptidases (Bacillus cereus) | 52.40% |
| MER080987 | - family M4 unassigned peptidases (Bacillus thuringiensis) | 52.40% |
| MER187805 | - family M4 unassigned peptidases (Bacillus cereus) | 52.55% |
| MER050323 | - family M4 unassigned peptidases (Bacillus cereus) | 53.21% |
| MER187780 | - family M4 unassigned peptidases (Bacillus thuringiensis) | 53.21% |
| MER022038 | - neutral peptidase B (Oceanobacillus iheyensis) | 49.50% |
| MER187809 | - family M4 unassigned peptidases (Bacillus cereus) | 49.05% |
| MER117663 | - family M4 unassigned peptidases (Shewanella halifaxensis) | 51.03% |
| MER014937 | - family M4 unassigned peptidases (Clostridium acetobutylicum) | 48.04% |
| MER002103 | - lambda toxin (Clostridium perfringens) | 46.86% |
| MER048471 | - bacillolysin (Brevibacillus laterosporus) | 49.51 |
| MER001035 | - bacillolysin (Bacillus amyloliquefaciens) | 49.51% |
| MER001038 | - bacillolysin (Bacillus sp.) | 49.51% |
| MER054676 | - bacillolysin (Bacillus sp. B16) | 49.18% |
| MER057051 | - aureolysin (Staphylococcus saprophyticus) | 47.02% |
| MER080743 | - bacillolysin (Bacillus sp. RH219) | 49.66% |
| MER187789 | - family M4 unassigned peptidases (Bacillus thuringiensis) | 48.72% |
| MER003790 | - family M4 unassigned peptidases (Clostridium histolyticum) | 47.71% |
| MER080014 | - bacillolysin (Bacillus subtilis) | 49.32% |
| MER001032 | - bacillolysin (Bacillus subtilis) | 47.37% |
| MER091634 | - bacillolysin (Bacillus pumilus) | 47.37% |
| MERO 14941 | - lambda toxin (Clostridium acetobutylicum) | 45.48% |
| MER091620 | - family M4 unassigned peptidases (Flavobacterium columnare) | 45.40% |
| MER155135 | - aureolysin (Macrococcus caseolyticus) | 48.65% |
| MER203088 | - family M4 unassigned peptidases (Shewanella violacea) | 49.82% |
| MER086404 | - family M4 unassigned peptidases (Stigmatella aurantiaca) | 45.11% |
| MER068045 | - family M4 unassigned peptidases (Myxococcus xanthus) | 45.39% |
| MER187787 | - family M4 unassigned peptidases (Bacillus thuringiensis) | 58.88% |
| MER251173 | - family M4 unassigned peptidases (Myxococcus fulvus) | 45.39% |
| MER091640 | - family M4 unassigned peptidases (Stigmatella aurantiaca) | 48.43% |
| MER086488 | - family M4 unassigned peptidases (Stigmatella aurantiaca) | 44.44% |
| MER025442 | - family M4 unassigned peptidases (Vibrio vulnificus) | 46.49% |
| MER001869 | - aureolysin (Staphylococcus epidermidis) | 46.13% |
| MER178903 | - aureolysin (Staphylococcus capitis) | 47.47% |
| MER017697 | - family M4 unassigned peptidases (Methanosarcina acetivorans) | 44.14% |
| MER062832 | - family M4 unassigned peptidases (Flavobacterium johnsoniae) | 45.71% |
| MER187814 | - aureolysin (Staphylococcus warneri) | 45.21% |
| MER004711 | - aureolysin (Staphylococcus aureus) | 46.28% |
| MER229315 | - family M4 unassigned peptidases (Vibrio mimicus) | 44.15% |
| MER011075 | - aureolysin (Staphylococcus chromogenes) | 43.00% |
| MER179736 | - aureolysin (Staphylococcus pseudintermedius) | 45.83% |
| MER187776 | - family M4 unassigned peptidases (Chryseobacterium gleum)] | 42.81% |
| MER068475 | - family M4 unassigned peptidases (Myxococcus xanthus) | 43.84% |
| MER063156 | - family M4 unassigned peptidases (Pseudoalteromonas tunicata) | 46.56% |
| MER252532 | - family M4 unassigned peptidases (Myxococcus fulvus) | 46.32% |
| MER091643 | - family M4 unassigned peptidases (Stigmatella aurantiaca)] | 45.64% |

Further analysis of members of the various families in the MEROPS database can be performed, such as the generation of phylogenetic trees. The architecture for the 424 members of the Family M4

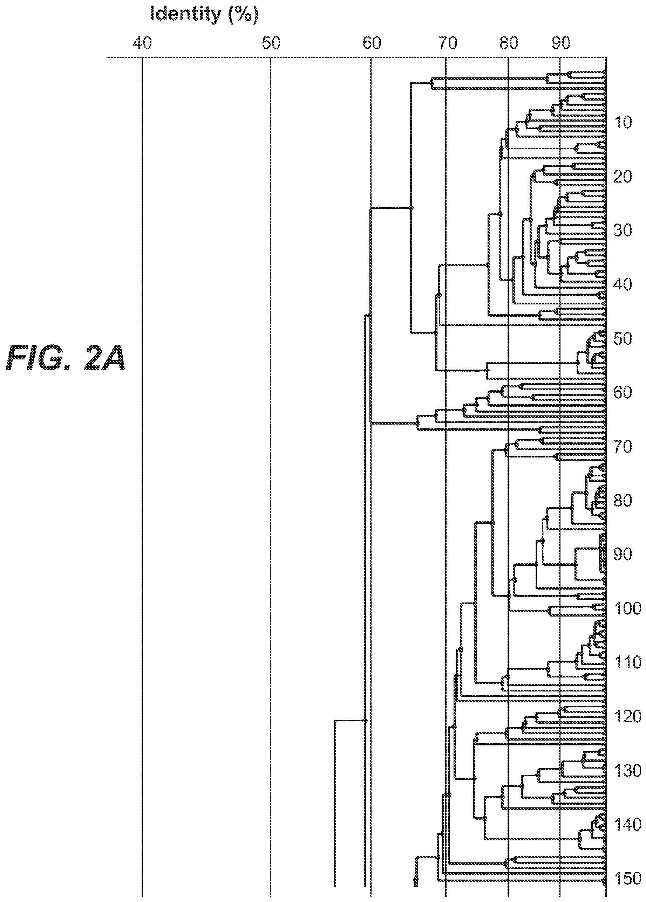

phylogenetic tree (http://merops.sanger.ac.uk/cgi-bin/famwrap/famcards/trees/m4_tree.htm) is provided below (Figures 2A- 2C).

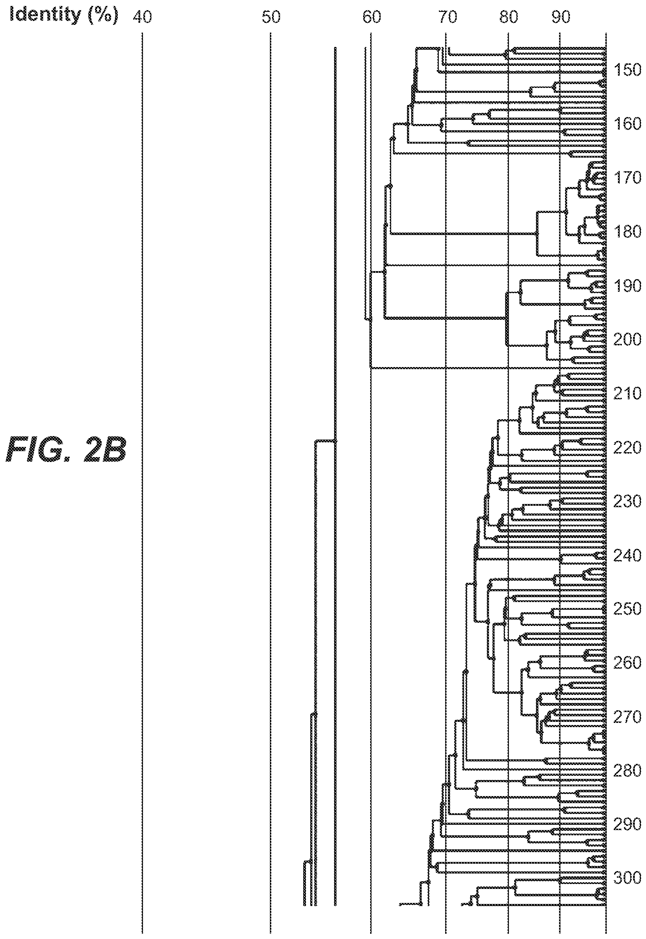

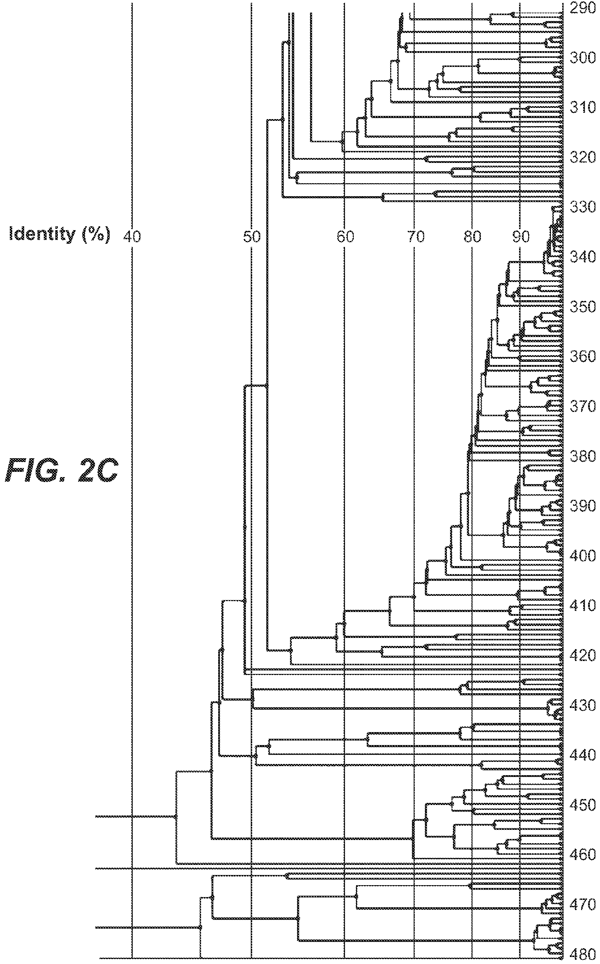

**Key to sequences and architecture of phylogenetic tree Family M4 shown above in** **Figure 2****.**

### PepSY~Peptidase_M4-Peptidase_M4_C

1 *Stigmatella aurantiaca)* family M4 unassigned peptidases (MER086404)

### P_proprotein~Peptidase_M4~Peptidase_M4_C

*2 (Stigmatella aurantiaca)* family M4 unassigned peptidases (MER086488)
*3 (Myxococcus xanthus)* family M4 unassigned peptidases (MER068045)

### Peptidase_M4~Peptidase_M4_C~PPC~PPC

*4 (Pseudoalteromonas tunicata)* family M4 unassigned peptidases (MER063156)

### Peptidase_M4~Peptidase_M4_ C

5 M04.017 *(Streptomyces avermitilis)* griselysin (MER028561)
6 M04.017 *(Streptomyces sviceus)* griselysin (MER144000)
7 M04.017 *(Streptomyces viridochromogenes)* griselysin (MER229668)
8 M04.017 *(Streptomyces coelicolor)* griselysin (MER012275)
9 M04.017 *(Streptomyces scabiei)* griselysin (MER200776)
10 M04.017 *(Kribbella flavida)* griselysin (MER076577)
11 M04.017 *(Janibacter sp. HTCC2649)* griselysin (MER119370)
12 M04.017 *(Nocardioides sp. JS614)* griselysin (MER075575)

### PepSY~Peptidase_M4-Peptidase M4_C

13 M04.017 *(Stigmatella aurantiaca)* griselysin (MER086497)
14 M04.017 *(Xanthomonas campestris)* griselysin (MER070193)
15 M04.017 *(Xanthomonas axonopodis)* griselysin (XAC0465 protein) (MER019560)
16 M04.017 *(Xanthomonas oryzae)* griselysin (MER113870)
17 M04.017 *(Micromonospora sp. L5)* griselysin (MER230635)
18 M04.017 *(Streptomyces avermitilis)* griselysin (SAV1037 protein) (MER028563)
19 M04.017 *(Streptomyces sviceus)* griselysin (MER187827)

### Peptidase_M4~Peptidase_M4_C

20 M04.017 *(Streptomyces pristinaespiralis)* griselysin (MER137080)
21 M04.017 *(Streptomyces sp. SPB74)* griselysin (MER163965)
22 M04.017 *(Streptomyces albus)* griselysin (MER187823)
23 M04.017 *(Streptomyces avermitilis)* griselysin (SAV2795 protein) (MER028566)
24 M04.017 *(Streptomyces sviceus)* griselysin (MER137175)
25 M04.017 *(Streptomyces ghanaensis)* griselysin (MER187817)
26 M04.017 *(Streptomyces coelicolor)* griselysin (MER019351)

### PepSY~Peptidase_M4-Peptidase M4_C

27 M04.017 *(Streptomyces scabiei)* griselysin (MER200969)

### Peptidase-M4~Peptidase_M4_C~P_proprotein

28 M04.017 *(Streptomyces sp. SPB74)* griselysin (MER137964)
29 M04.017 *(Streptomyces sviceus)* griselysin (MER187826)

### Peptidase_M4~Peptidase_M4_C~He_PIG

30 M04.017 *(Streptomyces avermitilis)* griselysin (MER028567)

### Peptidase_M4~Peptidase_M4_ C

31 M04.017 *(Streptomyces septatus)* griselysin (MER108931)
32 M04.017 *(Streptomyces scabiei)* griselysin (MER200878)
33 M04.017 *(Streptomyces sp. Mg1)* griselysin (MER180683)
34 M04.017 *(Streptomyces sviceus)* griselysin (MER187825)

### Peptidase_M4~Peptidase_M4_C~P_proprotein

35 M04.017 *(Streptomyces coelicolor)* griselysin (MER011085)
36 M04.017 *(Streptomyces scabiei)* griselysin (MER200968)
37 M04.017 *(Streptomyces ghanaensis)* griselysin (MER187816)
38 M04.017 *(Streptomyces griseus)* griselysin (MER004744)
39 M04.017 *(Streptomyces filamentosus)* griselysin (MER187821)
40 M04.017 *(Streptomyces avermitilis)* griselysin (MER028565)
41 M04.017 *(Streptomyces sp. Mg1)* griselysin (MER163416)

### Peptidase_M4~Peptidase_M4_ C

42 M04.017 *(Streptomyces griseus)* griselysin (MER121393)
43 M04.017 *(Streptomyces filamentosus)* griselysin (MER187820)
44 M04.017 *(Streptomyces sp. TH-3)* griselysin (MER169964)

### PepSY~Peptidase_M4-Peptidase M4_C

45 M04.017 *(Janibacter sp. HTCC2649)* griselysin (MER109443)
46 M04.017 *(Janibacter sp. HTCC2649)* griselysin (MER109417)
47 M04.017 *(Kribbella flavida)* griselysin (MER096497)
48 M04.017 *(Streptomyces avermitilis)* griselysin (MER028564)
49 M04.022 *(Burkholderia pseudomallei)* ZmpA peptidase (MER029961)
50 M04.022 *(Burkholderia mallei)* ZmpA peptidase (MER040142)
51 M04.022 *(Burkholderia thailandensis)* ZmpA peptidase (MER058477)
52 M04.022 *(Burkholderia oklahomensis)* ZmpA peptidase (MER1877661
53 M04.022 *(Burkholderia cenocepacia)* ZmpA peptidase (MER050804)
54 M04.022 *(Burkholderia cepacia)* ZmpA peptidase (MER028622)
55 M04.022 *(Burkholderia ambifaria)* ZmpA peptidase (MER055697)
56 M04.022 *(Burkholderia sp. 383)* ZmpA peptidase (MER056816)
57 M04.022 *(Burkholderia ubonensis)* ZmpA peptidase. (MER166266)
58 *(Dehalococcoides sp.* VS) M4 unassigned peptidases (MER109883)
59 *(unidentified eubacterium SCB49)* M4 unassigned peptidases (MER137229)
60 *(Croceibacter atlanticus)* M4 unassigned peptidases (MER118340)

### PepSY~Peptidase_M4~Peptidase_M4_C~fn3

*61* (*Flavobacterium johnsoniae*) M4 unassigned peptidases (MER062832)
*62* (*Flavobacterium columnare*) M4 unassigned peptidases (MER091620)

### Peptidase_M4~Peptidase_M4_ C

63 (*Croceibacter atlanticus*) M4 unassigned peptidases (MER109847)
*64* (*Chrvseobacterium gleum*) M4 unassigned peptidases (MER187776)
65 (*Kordia algicida*) M4 unassigned peptidases (MER166403)

### PepSY~Peptidase_M4~Peptidase_M4_C~MAH

*66* (*Microscilla marina*) M4 unassigned peptidases (MER091624)
*67* (*Croceibacter atlanticus*) M4 unassigned peptidases (MER117388)
68 (*Croceibacter atlanticus*) M4 unassigned peptidases (MER138802)
69 (*Paenibacillus larvae*) M4 unassigned peptidases (MER187765)
70 M04.001 (*Paenibacillus larvae*) thermolysin (MER168882)

### Peptidase_M4~Peptidase_M4_ C

*71* (*Paenibacillus polymyxa*) M4 unassigned peptidases (MER001033)

### PepSY~Peptidase_M4-Peptidase M4_C

72 M04.001 *(Brevibacillus brevis)* thermolysin (MER001028)
73 M04.001 *(Brevibacillus brevis)* thermolysin (npr protein) (MER169677)
74 M04.001 *(Bacillus pseudomvcoides)* thermolysin (MER187790)
75 M04.001 *(Bacillus mvcoides)* thermolysin (MER187794)
76 M04.001 *(Bacillus cereus)* thermolysin (MER061817)
77 M04.001 *(Bacillus cereus)* thermolysin (MER187808)
78 M04.001 *(Bacillus weihenstephanensis)* thermolysin (MER109389)
79 M04.001 *(Bacillus mvcoides)* thermolysin (MER187798)
80 M04.001 *(Bacillus cereus)* thermolysin (MER001030)
81 M04.001 *(Bacillus thuringiensis)* thermolysin (MER003181)
82 M04.001 *(Bacillus pseudomvcoides)* thermolysin (MER176709)
83 M04.001 *(Lactobacillus sp.)* thermolysin (MER001354)
84 M04.001 *(Bacillus anthracis)* thermolysin (MER021824)
85 M04.001 *(Bacillus megaterium)* thermolysin (MER001031)
86 M04.001 *(Bacillus sp. SG-1* thermolysin (MER109427)
87 M04.001 *(Bacillus caldolvticus)* thermolysin (MER001034)
88 M04.018 *(Geobacillus stearothermophilus)* stearolysin (MER001025)
89 M04.001 *(Geobacillus sp. Y412MC52)* thermolysin (MER234417)
90 M04.001 *(Alicvclobacillus acidocaldarius)* thermolysin (MER001353)
91 M04.001 *(Bacillus sp.)* thermolysin (MER001927)
92 M04.001 *(Geobacillus sp. Y412MC61)* thermolysin (MER168133)
93 M04.001 *(Geobacillus sp. C56-T3)* thermolysin (MER212338)
94 M04.001 *(Geobacillus kaustophilus)* thermolysin (MER040474)
**95 M04.001 *(Bacillus thermoproteolyticus)* thermolysin (MER001026)**
96 M04.001 *(Geobacillus stearothermophilus)* thermolysin (MER001027)
97 M04.018 *(Bacillus sp. SG-1)* stearolysin (MER109364)
98 M04.001 *(Exignobacterium sibiricum)* thermolysin (MER091675)
99 M04.001 *(Exiguobacterium sp. AT1b)* thermolysin (MER124526)
100 M04.001 *(Bacillus mycoides)* thermolysin (MER187797)
101 M04.001 *(Bacillus pseudomycoides)* thermolysin (MER187793)
102 *(Bacillus thuringiensis)* M4 unassigned peptidases (MER187787)
103 M04.012 *(Bacillus thuringiensis)* neutral peptidase B (MER039810)
104 M04.012 *(Bacillus cereus)* neutral peptidase B (MER028887)
105 M04.012 *(Bacillus weihenstephanensis)* neutral peptidase B (MER084215)
106 M04.012 *(Bacillus mvcoides)* neutral peptidase B (MER187800)
107 M04.012 *(Bacillus cereus)* neutral peptidase B (MER151875)
108 M04.012 *(Bacillus anthracis)* neutral peptidase B (MER021804)
109 M04.012 *(Bacillus pseudomycoides)* neutral peptidase B (MER187792)
110 M04.012 *(Bacillus mvcoides)* neutral peptidase B (MER187796)
111 *(Bacillus cereus)* M4 unassigned peptidases (MER084165)
112 M04.012 *(Bacillus cereus)* neutral peptidase B (MER187810)
113 M04.012 *(Bacillus cereus)* neutral peptidase B (MER187806)
114 M04.012 *(Bacillus thuringiensis)* neutral peptidase B (MER1877791
115 M04.012 *(Oceanobacillus ihevensis)* neutral peptidase B (MER022038)
116 M04.012 *(Bacillus subtilis)* neutral peptidase B (MER001029)

### Peptidase_M4~Peptidase_M4_C

117 *(Herpetosiphon aurantiacus)* M4 unassigned peptidases (MER091650)
118 *(Bacillus cereus)* M4 unassigned peptidases (MER187809)

### PepSY~Peptidase_M4-Peptidase M4_C

119 M04.009 *(Staphylococcus epidermidis)* aureolysin (MER001869)
120 M04.009 *(Staphylococcus capitis)* aureolysin (MER178903)
121 M04.009 *(Staphylococcus aureus)* aureolysin (MER004711)
122 M04.009 *(Macrococcus caseolyticus)* aureolysin (MER155135)
123 M04.009 *(Staphylococcus pseudintermedius)* aureolysin (MER179736)
124 M04.009 *(Staphylococcus warneri)* aureolysin (MER187814)
125 M04.009 *(Staphylococcus chromogenes)* aureolysin (MER011075)
126 M04.009 *(Staphylococcus saprophvticus)* aureolysin (MER057051)
*127 (Bacillus cereus)* M4 unassigned peptidases (MER050323)
128 *(Bacillus thuringiensis* M4 unassigned peptidases (MER187780)
129 *(Bacillus cereus)* unassigned peptidases (MER062591)
130 *(Bacillus cereus)* M4 unassigned peptidases (MER187770)
131 *(Bacillus thuringiensis)* M4 unassigned peptidases (MER080987)
132 *(Bacillus cereus* M4 unassigned peptidases (MER187805)
133 *(Bacillus thuringiensis)* M4 unassigned peptidases (MER187789)
134 *(Bacillus vietnamensis)* M4 unassigned peptidases (MER038281)
135 *(Bacillus sp. NRRL B-14911)* M4 unassigned peptidases (MER062589)
136 *(Bacillus sp. SG-1* M4 unassigned peptidases (MER109478)
137 *(Bacillus coahuilensis)* M4 unassigned peptidases (MER187771)

### PepSY~Peptidase_M4-Peptidase M4_C~PPC

138 M04.021 *(Thermoactinomyces sp. 27a)* neutral peptidase (MER029719)

### PepSY~Peptidase_M4-Peptidase M4_C

139 M04.014 *(Bacillus subtilis)* bacillolysin (MER080014)
140 M04.014 *(Bacillus sp. RH219)* bacillolysin (MER080743)
141 M04.014 *(Bacillus sp. B16)* bacillolysin (MER054676)

### Peptidase_M4~Peptidase_M4_C

142 M04.014 *(Brevibacillus laterosporus)* bacillolysin (MER048471)

### PepSY~Peptidase_M4-Peptidase M4_C

143 M04.014 *(Bacillus amyloliquefaciens)* bacillolysin (MER001035)
144 M04.014 *(Bacillus sp.)* bacillolysin (MER001038)

### Peptidase_M4~Peptidase_M4_ C

145 M04.014 *(Bacillus pumilus)* bacillolysin (MER091634)

### PepSY~Peptidase_M4-Peptidase M4_C

146 M04.014 *(Bacillus subtilis)* bacillolysin (MER001032)
*147 (Clostridium acetobutylicum)* family M4 unassigned peptidases (MER014937)
148 M04.011 *(Clostridium perfrinsens)* lambda toxin (MER002103)
149 M04.011 *(Clostridium acetobutylicum)* lambda toxin (MER014941)
150 *(Clostridium histolyticum)* M4 unassigned peptidases (MER003790)

### Peptidase_ M4~Peptidase_ M4_C~PPC

151 *(Chloroflexus aurantiacus)* family M4 unassigned peptidases (MER001453)
152 *(Chloroflexus sp. Y-400-fl)* family M4 unassigned peptidases (MER155497)
153 M04.008 *(Listeria innocua)* Mpl peptidase *(Listeria* sp.) (MER229925)

### PepSY~Peptidase_M4-Peptidase M4_C

154 M04.008 *(Listeria monocytogenes)* Mpl peptidase (MER001047)
155 M04.008 *(Listeria ivanovii)* Mpl peptidase (MER045739)
156 M04.008 *(Listeria seeliseri)* Mpl peptidase (MER045740)
157 *(Plesiocystis pacifica)* M4 unassigned peptidases (MER160603)

### Peptidase_M4~Peptidase_M4_C~PPC~PPC

158 *(Stigmatella aurantiaca)* M4 unassigned peptidases (MER091643)
159 *(Stigmatella aurantiaca)* M4 unassigned peptidases (MER091640)

### Peptidase_M4~Peptidase_M4_ C

160 *(Myxococcus xanthus)* M4 unassigned peptidases (MER068475)

### Peptidase_M4~Peptidase_M4_C~PPC~PPC

161 *Myxococcus xanthus)* M4 unassigned peptidases (MER017624)

### Peptidase_M4~Peptidase_M4_C

162 *(Shewanella halifaxensis)* M4 unassigned peptidases (MER117663)
163 *(Shewanella violacea)* M4 unassigned peptidases (MER203088)
164 *(Haliscomenobacter hydrossis)* M4 unassigned peptidases (MER248570)
165 *(Cytophasa hutchinsonii)* M4 unassigned peptidases (MER023927)
166 *(Vibrio mimicus)* M4 unassigned peptidases (MER229315)

### Peptidase_M4~Peptidase_M4_C

167 *(Vibrio vulnificus)* M4 unassigned peptidases (MER025442)
168 *(Bacillus cereus)* M4 unassigned peptidases (MER187802)
169 *(Bacillus cereus)* M4 unassigned peptidases (MER091678)
170 *(Bacillus anthracis)* M4 unassigned peptidases (MER019065)
171 *(Bacillus cereus)* M4 unassigned peptidases (MER054507)
*172 (Bacillus thuringiensis)* M4 unassigned peptidases (MER039813)
173 *(Bacillus cereus)* M4 unassigned peptidases (MER187804)
*174 (Bacillus thuringiensis)* M4 unassigned peptidases (MER091674)

### PepSY~Peptidase_M4~Peptidase_M4_C~Big_3~Gram_pos_anchor

175 *(Bacillus cereus)* M4 unassigned peptidases (MER028889)
176 *(Bacillus thuringiensis)* M4 unassigned peptidases (MER039811)
*177 (Bacillus cereus)* M4 unassigned peptidases (MER028890)
178 *(Bacillus anthracis)* M4 unassigned peptidases (MER020840)
179 *(Bacillus mvcoides)* M4 unassigned peptidases (MER187799)
180 *(Bacillus weihenstephanensis)* M4 unassigned peptidases (MER109684)
181 *(Bacillus pseudomvcoides)* M4 unassigned peptidases (MER187791)
182 *(Bacillus mvcoides)* M4 unassigned peptidases (MER187795)
183 *(Bacillus mvcoides)* M4 unassigned peptidases (MER187801)
184 *(Bacillus thuringiensis)* M4 unassigned peptidases (MER039814)

### Peptidase_M4~Peptidase_M4_C

185 *(Bacillus cereus)* M4 unassigned peptidases (MER028888)
186 *(Bacillus anthracis)* M4 unassigned peptidases (MER020835)
187 *(Hahella cheiuensis* M4 unassigned peptidases (MER058667)

### PepSY~Peptidase_M4-Peptidase M4_C

188 *(Clostridium botulinum)* M4 unassigned peptidases (npr protein) (MER088299)

### PepSY~Peptidase_M4-Peptidase M4_C

189 *(Clostridium botulinum)* M4 unassigned peptidases (npr-1 protein) (MER079342)
190 *(Clostridium sporogenes)* M4 unassigned peptidases (MER137542)
191 *(Clostridium botulinum)* family M4 unassigned peptidases (npr protein) (MER187767)
192 *(Clostridium botulinum)* M4 unassigned peptidases (npr_1protein) (MER187754)
193 *(Clostridium botulinum)* M4 unassigned peptidases (MER187753)
194 *(Clostridium botulinum)* M4 unassigned peptidases (MER079338)
195 *(Clostridium sporogenes)* M4 unassigned peptidases (MER144884)
196 *(Clostridium botulinum)* M4 unassigned peptidases (npr protein) (MER079341)
197 *(Clostridium sporogenes)* M4 unassigned peptidases (MER187769)
198 *(Clostridium botulinum)* M4 unassigned peptidases (npr_2protein) (MER187755)
199 *(Clostridium botulinum)* M4 unassigned peptidases (MER079340)
200 *(Clostridium botulinum)* M4 unassigned peptidases (npr-4 protein) (MER095317)
201 *(Clostridium botulinum)* M4 unassigned peptidases (npr-4 protein) (MER094802)
*202 (Clostridium botulinum)* M4 unassigned peptidases (npr protein) (MER094801)
203 *(Clostridium sporogenes)* M4 unassigned peptidases (MER136684)
*204 (Clostridium botulinum)* M4 unassigned peptidases (npr protein) (MER079339)
205 *(Clostridium sporogenes)* M4 unassigned peptidases (MER178275)

### Peptidase_M4~Peptidase_M4_C~P_proprotein~P_proprotein

206 *(Methanosarcina acetivorans)* M4 unassigned peptidases (MER017697)
*207 (Streptomyces ghanaensis)* M4 unassigned peptidases (MER187818)

### Peptidase_M4~Peptidase_M4_ C

208 *(Streptomyces coelicolor)* family M4 unassigned peptidases (MER011082)
209 *(Streptomyces scabiei)* family M4 unassigned peptidases (MER200705)
210 *(Streptomyces avermitilis)* family M4 unassigned peptidases (MER028562)
211 *(Streptomyces sviceus)* family M4 unassigned peptidases (MER137373)
*212 (Streptomyces sp. Mg1)* family M4 unassigned peptidases (MER137463)
*213 (Streptomyces griseus)* family M4 unassigned peptidases (MER121447)
*214 (Streptomyces filamentosus)* family M4 unassigned peptidases (MER187819)
215 *(Streptomyces pristinaespiralis)* family M4 unassigned peptidases (MER140364)
216 *(Streptomyces albus)* family M4 unassigned peptidases (MER187822)
*217 (Streptomyces sp. SPB74)* family M4 unassigned peptidases (MER163861)
218http://merops.sanger.ac.uk/cgi-bin/pepsum?id=M04.UPW *(Streptomyces clavuliserus)* family M4 unassigned peptidases (MER187824)
219 *(Arthrobacter chlorophenolicus)* family M4 unassigned peptidases (MER126758)
*220 (Arthrobacter phenanthrenivorans)* family M4 unassigned peptidases (MER240183)
*221 (Arthrobacter sp. FB24)* family M4 unassigned peptidases (MER050759)
*222 (Arthrobacter aurescens)* family M4 unassigned peptidases (MER075195)
*223 (marine actinobacterium PHSC20C1)* family M4 unassigned peptidases
*224 (Brachybacterium faecium)* family M4 unassigned peptidases (MER127552)
*225 (Clavibacter michiganensis)* family M4 unassigned peptidases (MER121216)
*226 (Clavibacter michiganensis)* family M4 unassigned peptidases (MER115299)
*227 (Microbacterium testaceum)* family M4 unassigned peptidases (MER247399)
*228 (Intrasporamium calvum)* family M4 unassigned peptidases (MER231738)
*229 (Janibacter sp. HTCC2649)* family M4 unassigned peptidases (MER115301)
230 *(Frankia alni)* family M4 unassigned peptidases (MER091651)
231 *(Frankia sp. CcI3)* family M4 unassigned peptidases (MER051510)
*232 (Frankia sp. EAN1pec)* family M4 unassigned peptidases (MER051747)
233 *(Meiothermus silvanus)* family M4 unassigned peptidases (MER038269)
*234 (Pseudomonas fluorescens)* family M4 unassigned peptidases (MER187756)
235 *(Myxococcus xanthus)* family M4 unassigned peptidases (MER068095)
236 *(Burkholderia sp. CCGE1002)* family M4 unassigned peptidases (MER203878)
*237 (Ricinus communis)* family M4 unassigned peptidases (MER162821)
238 *(Catenulispora acidiphila)* family M4 unassigned peptidases (MER132795)
239 *(Brevibacterium linens)* family M4 unassigned peptidases (MER115300)
*240 (Anabaena variabilis)* family M4 unassigned peptidases (MER054976)
*241 (Nostoc sp. PCC 7120)* family M4 unassigned peptidases (MER016719)
*242 (Nostoc punctiforme)* family M4 unassigned peptidases (MER024259)
243 *(Xanthomonas axonopodis)* family M4 unassigned peptidases (MER019561)
*244 (Xanthomonas campestris)* family M4 unassigned peptidases (MER070175)
*245 (Xanthomonas orvzae)* family M4 unassigned peptidases (MER027496)
*246 (Xanthomonas campestris)* family M4 unassigned peptidases (MER019416)
*247 (Thermomonospora curvata)* family M4 unassigned peptidases (MER129229)
*248 (Halomonas elongata)* family M4 unassigned peptidases (MER223548)
*249 (Chromohalobacter salexigens)* family M4 unassigned peptidases (MER050897)
250 *(Bordetella parapertussis)* family M4 unassigned peptidases (MER030706)
251 *(Bordetella bronchiseptica)* family M4 unassigned peptidases (MER030781)
*252 (Bordetella petrii)* family M4 unassigned peptidases (MER114690)
253 *(Variovorax paradoxus)* family M4 unassigned peptidases (MER187757)
*254 (Variovorax paradoxus)* family M4 unassigned peptidases (MER235281)
255 *(Pseudomonas brassicacearum)* family M4 unassigned peptidases (MER244770)
256 *(Pseudomonas fulva)* family M4 unassigned peptidases (MER249215)
*257 (Pseudomonas stutzeri)* family M4 unassigned peptidases (MER094699)
258 *(Dickeya dadantii)* family M4 unassigned peptidases (MER223843)
259 *(Dickeya dadantii)* family M4 unassigned peptidases (MER193415)
260 *(Dickeya zeae)* family M4 unassigned peptidases (MER187758)
261 *(Pectobacterium carotovorum)* family M4 unassigned peptidases (MER001045)
*262 (Pectobacterium wasabiae)* family M4 unassigned peptidases (MER187830)
263 *(Dickeya dadantii)* family M4 unassigned peptidases (MER182707)
264 M04.023 *(Citrobacter rodentium)* zpx peptidase (MER196184)
265 M04.023 *(Enterobacter cancerogenus)* zpx peptidase (MER187772)
266 M04.023 *(Salmonella enterica)* zpx peptidase (MER108712)
267 M04.023 *(Enterobacter sakazakii)* zpx peptidase (zpx protein) (MER091601)
268 M04.023 *(Erwinia amylovora)* zpx peptidase (prt1 protein) (MER202074)
269 M04.025 *(Erwinia billingiae)* protealysin (mpr protein) (MER220902)
270 M04.025 *(Pantoea sp. At-9b)* protealysin (MER232022)
271 M04.025 *(Pantoea ananatis)* protealysin (MER202817)
272 M04.025 *(Rahnella sp. Y9602)* protealysin (MER237139)
273 M04.025 *(Serratia grimesii)* protealysin (MER115298)
274 M04.025 *(Serratia sp. A2)* protealysin (MER119664)
275 M04.025 *(Serratia proteamaculans)* protealysin (MER059439)
276 M04.025 *(Serratia sp. AS9)* protealysin (MER249825)
277 M04.025 *(Serratia sp. AS12)* protealysin (MER249807)
*278 (Geodermatophilus obscurus)* family M4 unassigned peptidases (MER132589)
*279 (Gemmata obscuriglobus)* family M4 unassigned peptidases (MER187768)
280 *(Nocardioides sp. JS614)* family M4 unassigned peptidases (MER049523)
281 M04.024 *(Xenorhabdus bovienii)* PrtS peptidase *(Photorhabdus luminescens)* (MER200616)
282 M04.024 *(Xenorhabdus nematophila)* PrtS peptidase (MER219816)
283 M04.024 *(Xenorhabdus nematophila)* PrtS peptidase (MER219815)
284 M04.024 *(Photorhabdus asymbiotica)* PrtS peptidase (MER1877591
285 M04.024 *(Photorhabdus luminescens)* PrtS peptidase (MER033481)
286 M04.024 *(Photorhabdus sp. Az29)* PrtS peptidase (MER115297)
*287 (Aspergillus terreus)* family M4 unassigned peptidases (MER091644)
288 *(Neosartorya fischeri)* family M4 unassigned peptidases (MER091615)
289 *(Pyrenophora tritici-repentis)* family M4 unassigned peptidases (MER138903)
290 *(Saccharopolyspora erythraea)* family M4 unassigned peptidases (MER088688)
291 *(Nectria haematococca)* family M4 unassigned peptidases (MER243771)
*292 (Gibberella zeae)* family M4 unassigned peptidases (MER064838)
293 *(Metarhizium anisopliae)* family M4 unassigned peptidases (MER243770)
*294 (Metarhizium acridum)* family M4 unassigned peptidases (MER243769)
295 *(Waddlia chondrophila)* family M4 unassigned peptidases (MER211844)
296 *(Pseudomonas savastanoi)* family M4 unassigned peptidases (MER232822)
*297 (Pseudomonas syringae)* family M4 unassigned peptidases (MER052672)
298 *(Pseudomonas coronafaciens)* family M4 unassigned peptidases (MER187813)
299 *(Cyanothece sp. ATCC 51142)* family M4 unassigned peptidases (MER103362)
300 *(Bacillus thuringiensis)* family M4 unassigned peptidases (MER187783)
301 *(Bacillus cereus)* family M4 unassigned peptidases (MER178978)
302 *(Bacillus cereus)* family M4 unassigned peptidases (MER187811)
303 *(Bacillus thuringiensis)* family M4 unassigned peptidases (MER187778)
304 *(Bacillus cereus)* family M4 unassigned peptidases (MER187807)
305 *(Methanosarcina acetivorans)* family M4 unassigned peptidases (MER0176981
306 *(Bacillus thuringiensis)* family M4 unassigned peptidases (MER187784)
307 *(Bacillus thuringiensis)* family M4 unassigned peptidases (MER187788)
308 *(Cellulophaga algicola)* family M4 unassigned peptidases (MER235562)
309 *(Aspergillus niger)* family M4 unassigned peptidases (MER091631)
310 *(Providencia rustigianii)* family M4 unassigned peptidases (MER187773)
311 *(Providencia alcalifaciens)* family M4 unassigned peptidases (MER187774)
312 *(Providencia rettseri)* family M4 unassigned peptidases (MER187775)
313 *(Providencia stuartii)* family M4 unassigned peptidases (MER122839)
314 *(Mycobacterium abscessus)* family M4 unassigned peptidases (MER117364)
315 (*Mycobacterium abscessus*) family M4 unassigned peptidases (MER117363)
316 (*Bradvrhizobium japonicum*) family M4 unassigned peptidases (MER026988)
317 (*Agrobacterium vitis*) family M4 unassigned peptidases (MER162454)
318 (*Mucilaginibacter paludis*) family M4 unassigned peptidases (MER229316)

### Peptidase_M4_C

319 (*Serratia marcescens*) family M4 unassigned peptidases (MER001046)
320 (*Streptomvces ghanaensis*) family M4 unassigned peptidases (MER187815)

### Peptidase_M4~Peptidase_M4_C

321 (*Sorangium cellulosum*) family M4 unassigned peptidases (MER114292)

### Peptidase_M4~Peptidase_M4_C

322 (*Streptomvces filamentosus*) family M4 unassigned peptidases (MER091679)

### PepSY~Peptidase_M4-Peptidase_M4_C

323 (*Streptomyces avermitilis*) family M4 unassigned peptidases (MER028519)
324 (*Streptosporansium roseum*) family M4 unassigned peptidases (MER187812)

### Peptidase_M4~Peptidase_M4_C

325 M04.007 *(Enterococcus faecium*) coccolysin (MER187749)
326 M04.007 *(Enterococcus faecalis*) coccolysin (MER002810)
327 (*Renibacterium salmoninarum*) family M4 unassigned peptidases (MER002083)
328 (*Kribbella flavida*) family M4 unassigned peptidases (MER079366)
329 (*Herpetosiphon aurantiacus*) family M4 unassigned peptidases (MER085851)

### Peptidase_M4~Peptidase_M4_C

330 M04.016 *(Aeromonas jandaei*) PA peptidase *(Aeromonas-type)* (MER079815)
331 M04.016 *(Aeromonas eucrenophila*) PA peptidase (*Aeromonas*-type) (MER079803)

### PepSY~Peptidase_M4-Peptidase_M4_C~PPC

332 M04.016 *(Aeromonas punctata*) PA peptidase *(Aeromonas-type)* (MER029943)

### Peptidase_M4~Peptidase_M4_C

333 M04.016 (*Aeromonas encheleia*) PA peptidase (MER079817)
334 M04.016 *(Aeromonas bestiarum*) PA peptidase (MER079816)
335 M04.016 *(Aeromonas media*) PA peptidase (MER079802)
336 M04.016 *(Aeromonas salmonicida*) PA peptidase (MER079819)

### PepSY~Peptidase_M4-Peptidase_M4_C~PPC

337 M04.016 *(Aeromonas punctata*) PA peptidase (MER030073)

### Peptidase_M4~Peptidase_M4_C

338 M04.016 (*Aeromonas encheleia*) PA peptidase (MER079804)
339 M04.016 *(Aeromonas popoffii*) PA peptidase (MER079805)
340 M04.016 *(Aeromonas hydrophila*) PA peptidase (MER011853)
341 M04.016 (*Aeromonas sp. CDC 2478-85*) PA peptidase (MER079818)
342 M04.016 *(Aeromonas schubertii*) PA peptidase (*Aeromonas-*type) (MER079806)

### PepSY~Peptidase_M4-Peptidase_M4_C~PPC

343 (*Aeromonas veronii*) M4 unassigned peptidases (MER055154)

### Peptidase_M4~Peptidase_M4_C

*344 (Aeromonas sobria*) M4 unassigned peptidases (MER079820)

### FTP~PepSY~Peptidase_M4∼Peptidase_M4_C~PPC~PPC

345 (*Reinekea sp. MED297*) family M4 unassigned peptidases (MER083727)
346 (*Shewanella denitrificans*) family M4 unassigned peptidases (MER050231)

### PepSY~Peptidase_M4~Peptidase_M4_C~PPC~PKD

*347 (Shewanella baltica*) family M4 unassigned peptidases (MER048895)
348 *(Shewanella amazonensis*) family M4 unassigned peptidases (MER048811)
349 *(Shewanella woodyi*) family M4 unassigned peptidases (MER087265)

### PepSY~Peptidase_M4-Peptidase_M4_C

350 *(Chromobacterium violaceum*) family M4 unassigned peptidases (MER027350)

### PepSY~Peptidase_M4~Peptidase_M4_C~PPC~PPC

351 (*Pseudoalteromonas sp. SB-B1*) family M4 unassigned peptidases (MER140592)
352 (*Pseudoalteromonas sp. SM9913*) family M4 unassigned peptidases (MER091617)
353 (*Pseudoalteromonas sp. A28*) family M4 unassigned peptidases (MER019098)
354 (*Antarctic bacterium str. 643*) family M4 unassigned peptidases (MER012255)
355 (*Pseudoalteromonas sp. SM495)* family M4 unassigned peptidases (MER187748)
356 (*Pseudoalteromonas piscicida*) family M4 unassigned peptidases (MER019099)
357 (*Pseudoalteromonas ruthenica*) family M4 unassigned peptidases (MER187751)
358 (*Pseudoalteromonas tunicata*) family M4 unassigned peptidases (MER108855)
359 (*Moritella viscosa*) family M4 unassigned peptidases (MER139442)
360 (*Haliansium ochraceum*) family M4 unassigned peptidases (MER114761)
361 (*Haliansium ochraceum*) family M4 unassigned peptidases (MER124450)
362 (*Kangiella koreensis*) family M4 unassigned peptidases (MER065613)

### PepSY~Peptidase_M4-Peptidase_M4_C~PPC

363 M04.003 *(Marinomonas sp. MED121*) vibriolysin (MER139826)
364 (*Vibrio splendidus*) family M4 unassigned peptidases (MER122486)

### PepSY~Peptidase_M4~Peptidase_M4_C~PPC~PKD

365 (*Vibrionales bacterium SWAT-3*) family M4 unassigned peptidases (MER139254)

### PepSY~Peptidase_M4~Peptidase_M4_C~PKD

366 *(Vibrio tubiashii*) family M4 unassigned peptidases (MER187750)
367 *(Vibrio harveyi*) family M4 unassigned peptidases (MER091688)
368 *(Vibrio campbellii*) family M4 unassigned peptidases (MER139568)

### PepSY~Peptidase_M4-Peptidase_M4_C

369 *(Shewanella sp. MR-7*) family M4 unassigned peptidases (MER072768)
370 *(Shewanella sp. MR-4*) family M4 unassigned peptidases (MER073030)
371 *(Shewanella sp. ANA-3*) family M4 unassigned peptidases (MER073381)
*372 (Shewanella amazonensis*) family M4 unassigned peptidases (MER049928)
373 *(Shewanella woodyi*) family M4 unassigned peptidases (MER087209)
*374 (Vibrio parahaemolyticus*) family M4 unassigned peptidases (MER027936)

### PepSY~Peptidase_M4-Peptidase_M4_C~PPC

375 *(Vibrio sp. Ex25*) family M4 unassigned peptidases (MER139749)
376 *(Vibrio harveyi*) family M4 unassigned peptidases (MER109271)
*377* (*Hahella cheiuensis*) family M4 unassigned peptidases (MER080002)
378 M04.003 (*Moritella sp. PE36*) vibriolysin (MER113768)

### PepSY~Peptidase_M4~Peptidase_M4_C~PPC~PPC~P_proprotein

379 *(uncultured bacterium pTW3*) family M4 unassigned peptidases (MER164961)
380 *(uncultured bacterium pTW2*) family M4 unassigned peptidases (MER164951)

### PepSY~Peptidase_M4-Peptidase_M4_C

381 M04.005 (*Pseudomonas aeruginosa*) pseudolysin (MER001024)

### PepSY~Peptidase_M4-Peptidase_M4_C~PPC

382 M04.003 (*Vibrio tubiashii*) vibriolysin (MER139044)
383 M04.003 (*Vibrio proteolyticus*) vibriolysin (MER001043)
384 M04.003 (*Listonella amuillarum*) vibriolysin (MER120583)
385 M04.003 (*Vibrio anguillarum*) vibriolysin (MER001044)
386 M04.003 (*Listonella amuillarum*) vibriolysin (MER120671)
387 M04.003 (*Vibrio aestuarianus*) vibriolysin (MER113809)
388 M04.003 (*Vibrio vulnificus*) vibriolysin (MER003353)
389 M04.010 (*Vibrio splendidus*) vimelysin (MER091636)
390 M04.010 (*Vibrio sp. MED222*) vimelysin (MER113711)
391 M04.010 *(Vibrio sp. T-1800*) vimelysin (MER029796)
392 M04.010 (*Vibrionales bacterium SWAT-3*) vimelysin (MER109237)
393 M04.003 *(Vibrio cholerae*) vibriolysin (MER001041)
394 M04.003 *(Vibrio mimicus*) vibriolysin (MER122299)
395 M04.003 *(Vibrio fluvialis*) vibriolysin (MER019097)
396 M04.003 (*Salinivibrio sp. AF-2004*) vibriolysin (MER091639)
397 M04.010 *(Photobacterium sp. SKA34*) vimelysin (MER110034)
398 M04.010 *(Vibrio angustum*) vimelysin (MER109056)
399 M04.010 *(Photobacterium angustum*) vimelysin (MER187763)
400 M04.010 *(Vibrio angustum*) vimelysin (MER109302)

### Peptidase_M4~Peptidase_M4_C

401 (*Moritella sp. PE36*) family M4 unassigned peptidases (MER109180)

### Peptidase_M4~Peptidase_M4_C

*402 (Alteromonadales bacterium TW-7*) family M4 unassigned peptidases (MER091610)
403 (*Shewanella violacea*) family M4 unassigned peptidases (MER203253)
*404 (Vibrio campbellii*) family M4 unassigned peptidases (MER168125)

### PepSY~Peptidase_M4-Peptidase_M4_C

405 *(Legionella longbeachae*) family M4 unassigned peptidases (MER198565)
406 M04.020 (*Vibrio sp. AND4)* pap6 peptidase (MER187764)

### PepSY~Peptidase_M4-Peptidase_M4_C

407 M04.020 *(Vibrio campbellii)* pap6 peptidase (MER118605)
408 M04.020 *(Vibrio harveyi*) pap6 peptidase (MER020240)

### PepSY~Peptidase_M4-Peptidase_M4_C

409 *(Vibrio splendidus*) family M4 unassigned peptidases (MER139945)

### Peptidase_M4~Peptidase_M4_C

410 M04.006 *(Legionella pneumophila*) Msp peptidase (*Legionella*-type) (MER001039)
411 M04.006 *(Legionella drancourtii*) Msp peptidase (*Legionella*-type) (MER187828)

### PepSY~Peptidase_M4-Peptidase_M4_C

412 M04.006 *(Legionella longbeachae*) Msp peptidase (*Legionella*-type) (MER002394)
413 (*Legionella pneumophila*) family M4 unassigned peptidases (MER040780)
*414 (Legionella drancourtii*) family M4 unassigned peptidases (MER187829)
415 *(Legionella longbeachae*) family M4 unassigned peptidases (MER198471)
416 (*Legionella pneumophila*) family M4 unassigned peptidases (MER040782)
*417 (Legionella pneumophila*) family M4 unassigned peptidases (MER040781)

### Peptidase_M4~Peptidase_M4_C~PKD~PKD~PKD

418 M04.019 (*Pseudoalteromonas piscicida*) MprIII (MER024591)
419 (*Teredinibacter turnerae*) M4 unassigned peptidases (MER187760)

### Peptidase_M4~Peptidase_M4_C~PKD~PKD~PKD~PKD

420 (*Reinekea sp. MED297*) family M4 unassigned peptidases (MER083722)
421 (*Reinekea blandensis*) family M4 unassigned peptidases (MER187762)

### Peptidase_M4~Peptidase_M4_C

422 (*Reinekea sp. MED297*) family M4 unassigned peptidases (MER117392)
*423* (*Alteromonas sp. SN2)* family M4 unassigned peptidases (MER247991)

### PepSY~Peptidase_M4_C

*424* (*Hydrosenivirsa sv. 128-5-R1-1*) family M4 unassigned peptidases (MER142070)

### B. Identification of related molecules using Genome Quest search algorithm

A protein BLAST analysis (Altschul SF, Madden TL, Schäffer AA, Zhang J, Zhang Z, Miller W, Lipman DJ.(1997) Nucleic Acids Res. 25:3389-402) run within Genome Quest (www.genomequest.com) against patent and public domain databases, using the sequence of Thermolysin (SEQ ID NO: 3) as query yielded the results shown below **(Table 5.2).** BLAST is GenomeQuest's implementation of the NCBI BLAST2 algorithm and finds the most relevant sequences in terms of biological similarity. The sequence search has the following default BLAST parameters for protein searches: Word size: 3, E-value cutoff: 10, Scoring Matrix: BLOSUM62, Gap Opening: 11, Gap extension: 2

**Table 5.2. Homologs of thermolysin protein (SEQ ID NO: 3) identified by BLAST analysis. Terms used: %ID = percent sequence identity, Identifier = patent number-SEQ ID NO or public domain accession number.**

| **% ID** | **Identifier** | **organism of origin** | **protein name** | **REFERENCE** |
|---|---|---|---|---|
| 100 | US20090263882-0183 | Bacillus stearothermophilus | | |
| 100 | 8TLN | Bacillus thermoproteolyticus | thermolysin | Holland DR, Tronrud DE, Pley HW, Flaherty KM, Stark W, Jansonius JN, Mckay DB, Matthews BW Biochemistry 31, 11310-11316 (1992) |
| 100 | AAA22625 | Geobacillus stearothermophilus | neutral protease (nprS) precursor | Nishiya,Y. and Imanaka,T. J. Bacteriol. 172 (9), 4861-4869 (1990) |
| 99.68 | US20090263882-0182 | Bacillus thermoproteolyticus | | |
| 99.37 | JP1994014788-0002 | Unidentified | | |
| 99.37 | CAA54291 | Bacillus thermoproteolyticus | thermolysin | O'Donohue,M.J., Biochem. J. 300 (PT 2), 599-603 (1994) |
| 99.05 | 1LNA | Bacillus Thermoproteolyticus | Chain E, | Titani,K., Nature New Biol. 238 (80), 35-37 (1972) |
| 87.66 | AAB18652 | Bacillus caldolyticus | neutral proteinase | Saul,D.J., Biochim. Biophys. Acta 1308 (1), 74-80 (1996) |
| 87.66 | WO2004011619-0003 | Not specified | | |
| 87.34 | US20090263882-0184 | Bacillus sp. | | |
| 87.34 | AAA22623 | Bacillus caldolyticus | neutral protease | van den Burg,B., J. Bacteriol. 173 (13), 4107-4115 (1991) |
| 86.39 | EP0867512-0001 | Unidentified | | |
| 86.39 | AAA22621 | Geobacillus stearothermophilus | thermostable neutral protease (nprT) | Takagi,M., J. Bacteriol. 163 (3), 824-831 (1985) |
| 79.43 | BAD77123 | Geobacillus kaustophilus HTA426 | hypothetical protein | Takami,H., Nucleic Acids Res. 32 (21), 6292-6303 (2004) |
| 73.42 | 1NPC | Bacillus Cereus, Strain Dsm 3101 | Neutral Protease (E.C.3.4.24.27) | Sidler,W., Biol. Chem. Hoppe-Seyler 367 (7), 643-657 (1986) |
| 73.42 | US20090263882-0195 | Bacillus cereus | | |
| 73.42 | AAB62279 | Bacillus thuringiensis serovar kurstaki | neutral protease A | Donovan,W.P.,Appl. Environ. Microbiol. 63 (6), 2311-2317 (1997) |
| 73.42 | US5759538-0004 | Bacillus thuringiensis | | |
| 73.1 | AAK69076 | Bacillus thuringiensis | neutral protease | Choi,S.-K., Submitted (13-JUN-2000) Korea Research Institute of Bioscience and Biotechnology, Taejon, Korea |
| 73.1 | US20090263882-0178 | Bacillus thuringiensis | | |
| 72.78 | AAU19730 | Bacillus cereus E33L | bacillolysin (thermolysin-like metalloprotease, peptidase M4) | Brettin,T.S., Submitted (14-JUL-2004) Joint Genome Institute, Department of Energy, CA 94598, USA |
| 72.47 | BAA06144 | Lactobacillus sp. | hydrolase | Maeda,T., J. Ferment. Bioeng. (1994) |
| 72.47 | JP1995184649-0001 | Lactobacillus sp. | | |
| 68.99 | ACB62386 | Exiguobacterium sibiricum 255-15 | Thermolysin | Rodrigues Extremophiles 10 (4), 285-294 (2006) |
| 67.72 | ACQ69059 | Exiguobacterium sp. AT1b | peptidase M4 thermolysin | Vishnivetskaya, T.A., J. Bacteriol. 193 (11), 2880-2881 (2011) |
| 67.41 | US7642079-0142 | Unknown | | |
| 66.14 | CAA43589 | Brevibacillus brevis | microbial metalloproteinases | Avakov,A.S Dokl. Biochem. 24, 1363-1372 (1990) |
| 65.82 | AEI46285 | Paenibacillus mucilaginosus KNP414 | Npr | Wang,J., Submitted (08-JUN-2011) Zhejiang Sci-Tech University, Hangzhou, Zhejiang 310018, China |
| 62.34 | BAH42306 | Brevibacillus brevis NBRC 100599 | bacillolysin precursor | Hosoyama,A., Submitted (31-MAR-2005) Contact: Director- NITE Genome Analysis Center (NGAC), Tokyo 151-0066, Japan |
| 57.91 | AEG80144 | Bacillus thuringiensis | metalloprotease | Chudasama,C.J., Submitted (18-APR-2011) V P Science College, Sardar PatelUniversity, Gujarat 388120, India |
| 57.59 | BAD13318 | Bacillus vietnamensis | protease | Kim,M Biosci. Biotechnol. Biochem. 68 (7), 1533-1540 (2004) |
| 56.65 | ADM71641 | Paenibacillus polymyxa E681 | Bacillolysin precursor (Neutral protease) | Kim,J.F., J. Bacteriol. 192 (22), 6103-6104 (2010) |
| 56.33 | ADR72651 | Bacillus sp. NprB gene MB | neutral protease B | Mustapha,S., Submitted (14-JUL-2010) University Malaysia Sabah, Biotechnology Research Institute, Kota Kinabalu, Sabah 88999, Malaysia |
| 56.01 | ADO58270 | Paenibacillus polymyxa SC2 | Bacillolysin | Ma,M., J. Bacteriol. 193 (1), 311-312 (2011) |
| 56.01 | AAP35685 | Thermoactinomyces sp. 27a | neutral protease precursor | Zabolotskaya,M.V., Protein J. 23 (7), 483-492 (2004) |
| 55.7 | US20090263882-0187 | Bacillus polymyxa | | Thermostable Neutral Metalloproteases |
| 55.7 | BAA00734 | Paenibacillus polymyxa | extracellular neutral protease | Takekawa,S., J. Bacteriol. 173 (21), 6820-6825 (1991) |
| 55.06 | ABK00710 | Bacillus cereus | putative metallopeptidase | Rasko,D.A., J. Bacteriol. 189 (1), 52-64 (2007) |
| 54.75 | AAU15507 | Bacillus cereus E33L | neutral protease B | Brettin,T.S., Submitted (14-JUL-2004) Joint Genome Institute, Department of Energy, CA 94598, USA |
| 54.43 | ABY46015 | Bacillus weihenstephanensis KBAB4 | peptidase M4 thermolysin | Lapidus,A, Chem. Biol. Interact. 171 (2), 236-249 (2008) |
| 54.43 | US20090263882-0180 | Bacillus subtilis | | Thermostable Neutral Metalloproteases |
| 54.11 | ABS21909 | Bacillus cytotoxicus NVH 391-98 | peptidase M4 thermolysin | Lapidus,A Chem. Biol. Interact. 171 (2), 236-249 (2008) |
| 53.8 | ADM71642 | [Paenibacillus polymyxa E681] | Bacillolysin precursor (Neutral protease) | Kim,J.F., J. Bacteriol. 192 (22), 6103-6104 (2010) |
| 53.48 | ACO28045 | Bacillus cereus 03BB102] | neutral protease | Dodson,R.J., Submitted (03-FEB-2009) Los Alamos National Laboratory, Los Alamos, NM, USA |
| 52.85 | ACQ49186 | Bacillus anthracis str. A0248 | neutral protease B | Dodson,R.J., Submitted (09-APR-2009) Los Alamos National Laboratory, Los Alamos, NM, USA |
| 49.68 | ABU53636 | Bacillus subtilis | neutral protease precursor | Zhao,C Submitted (11-JUL-2007) College of Biotechnology, Tianjin Univ. of Science and Technology, 13 Street, Tianjing, Tianjin 300457, China |
| 49.05 | WO2009058661-0019 | Synthetic construct | | Use And Production Of Citrate-Stable Neutral Metalloproteases |
| 48.73 | ABS73818 | Bacillus amyloliquefaciens FZB42 | NprE | Chen,X.H Nat. Biotechnol. 25 (9), 1007-1014 (2007) |
| 48.42 | AAW59490 | Brevibacillus laterosporus | extracellular neutral protease precursor | Tian,B.Y., Submitted (28-DEC-2004) Key Laboratory of Conservation and Utilization for Bioresources, Yunnan Univ, No. 2 North Road of Cuihu, Kunming, Yunnan 650091, China |
| 48.1 | ADZ21343 | Clostridium acetobutylicum EA 2018] | Extracellular neutral metalloprotease, NPRE, fused to ChW-repeats | Hu,S BMC Genomics 12, 93 (2011) |
| 47.78 | BAJ41480 | Bacillus subtilis | neutral protease | Takenaka,S., Biosci. Biotechnol. Biochem. 75 (1), 148-151 (2011) |
| 47.47 | AEJ66824 | Staphylococcus epidermis | Sequence 359 from patent US 7968297 | Meinke,A., Patent: US 7968297-B2 359 28-JUN-2011; |
| 47.15 | BAH18382 | Macrococcus caseolyticus JCSC5402 | zinc MMP aureolysin homolog | Baba,T J., Bacteriol. 191 (4), 1180-1190 (2009) |
| 46.84 | AAA22670 | Bacillus amyloliquefaciens | neutral protease | Shimada,H., J. Biotechnol. 2, 75-85 (1985) |
| 46.52 | ADX06849 | Bacillus subtilis | NprE | Liu,C., Wang,Z. and Yang,W. Submitted (29-DEC-2010) Anhui Agricultural University, College of Life Science, Changjiang West Road, Hefei, Anhui 230036, China |
| 46.2 | ADP31979 | Bacillus atrophaeus 1942 | extracellular neutral metalloprotease | Gibbons,H.S Submitted (13-SEP-2010) Genomics Integrated Product Team, US Army Edgewood Chemical Biological Center, 5183 Blackhawk Rd, Aberdeen Proving Ground, MD 21010-5424, USA |
| 45.57 | ABN71638 | Staphylococcus aureus | aureolysin | Sabat,A.J., BMC Microbiol. 8, 129 (2008) PUBMED 664262 |
| 45.25 | 1BQB | Staphylococcus Aureus | Aureolysin, Metalloproteinase | Medrano,F.J., Submitted (12-AUG-1998) |
| 44.94 | ABN71626 | Staphylococcus aureus | aureolysin | Sabat,A.J. BMC Microbiol. 8, 129 (2008) |
| 44.62 | ABN71636 | Staphylococcus aureus | aureolysin | Sabat,A.J., , BMC Microbiol. 8, 129 (2008) |
| 44.2 | WO2007044993-0013 | Bacillus amyloliquefaciens | | Use And Production Of Storage-Stable Neutral Metalloprotease |
| 44.2 | AAB05346 | Bacillus amyloliquefaciens | preproneutral protease (gtg start codon) | Vasantha,N., J. Bacteriol. 159 (3), 811-819 (1984) |
| 44.2 | AEB24126 | Bacillus amyloliquefaciens TA208 | extracellular neutral metalloprotease | Zhang,G., J. Bacteriol. 193 (12), 3142-3143 (2011) |
| 44.2 | CBI42672 | Bacillus amyloliquefaciens DSM 7 | extracellular neutral metalloprotease | Borriss,R., Int. J. Syst. Evol. Microbiol. 61 (PT 8), 1786-1801 (2011) |
| 44.2 | US20090263882-0003 WO2007044993-0003 | Bacillus amyloliquefaciens | | Thermostable Neutral Metalloproteases/Use And Production Of Storage-Stable Neutral Metalloprotease |
| 44.2 | WO2007044993-0018 | Bacillus amyloliquefaciens | (mature NprE sequence) | Use And Production Of Storage-Stable Neutral Metalloprotease |

### Example 6

### Using temperature factors to identify variants of Thermolysin with enhanced stability

Crystallographic temperature factors are a measure of the relative motion of individual atoms of a macromolecule. These temperature factors arise as a product of refinement of the model so that the calculated diffraction pattern given as individual intensities of crystal x-ray diffraction maxima best matches the observed pattern. The temperature factor is refined as an attenuation factor to reflect that atoms with higher motion will have a diminishing effect of the overall macromolecule aggregate diffraction as a function of the scattering angle (theta), using the form -exp(-Bsin²θ/λ.) where the B is the temperature factor (Blundell, T. L. and Johnson L. N., Protein Crystallography, Academic Press, 1976, pp121).

It is likely that regions with higher overall mobility might also represent points where the folded macromolecule is less stable and thus might be points where unfolding begins as the molecule is stressed by increasing temperature or denaturants. It would be further expected that these regions of higher overall mobility would be regions where the average temperature factors would be highest.

The crystallographic structure of *Bacillus thermoproteolyticus* Thermolysin protein has been determined by a number of independent laboratories. Three independent models of the protein were selected from the Protein Data Bank with entry identifications of 8TLN, 2TLX and 3DO1. We looked for regions of overall mobility by screening regions in the crystal structure where the temperature factors for the main chain are the highest and specifically where the average main chain temperature factor exceeds at least 1.5 times the observed variance from the mean. Tables 6a-6c list the residues for which the average main chain temperature factor has a z-score greater than 1.5 compared to the variance observed for the average main chain temperature factor for the overall molecule in a given crystallographic model. In these three structures, the same regions are found to exhibit temperature factors that are greater than 1.5 times the observed variance above the mean main chain temperature factor for all residues in Thermolysin. These regions represent consensus flexibility regions and include the following residues:

1-2 (N-terminal residues), 127-128, 180-181, 195-199, 211, 223-224,298-300, and 316 (C-terminal residue).

| Table 6a: PDB: 8TLN | | | |
|---|---|---|---|
| WT AA | POS | Average main chain B-factor mean value (17.79) and variance (5.43) for the entire molecule | |
| | | Mean | z-score |
| G | 196 | 49.31 | 5.81 |
| I | 1 | 42.17 | 4.49 |
| K | 182 | 38.18 | 3.76 |
| S | 198 | 37.91 | 3.71 |
| K | 316 | 37.53 | 3.64 |
| T | 2 | 36.91 | 3.52 |
| I | 197 | 35.04 | 3.18 |
| Y | 211 | 33.64 | 2.92 |
| P | 195 | 33.4 | 2.88 |
| G | 199 | 30.8 | 2.4 |
| S | 298 | 30.3 | 2.3 |
| Q | 128 | 29.22 | 2.11 |
| T | 299 | 29.11 | 2.09 |
| T | 224 | 28.82 | 2.03 |
| G | 127 | 28.5 | 1.97 |
| G | 223 | 27.98 | 1.88 |
| T | 293 | 27.57 | 1.8 |
| D | 124 | 8.3 | 1.75 |
| G | 252 | 27.28 | 1.75 |
| G | 109 | 27.16 | 1.73 |
| Q | 301 | 27.03 | 1.7 |
| K | 210 | 26.6 | 1.62 |
| A | 73 | 9.38 | 1.55 |
| D | 126 | 26.19 | 1.55 |

| Table 6b: PDB: 3DO1 | | | |
|---|---|---|---|
| WT AA | POS | Average main chain B-factor mean value (19.92) and variance (3.72) for the entire molecule | |
| | | Mean | z-score |
| S | 298 | 33.38 | 3.62 |
| T | 299 | 32.9 | 3.49 |
| G | 297 | 31.99 | 3.24 |
| S | 300 | 31.01 | 2.98 |
| Y | 296 | 30.95 | 2.97 |
| L | 295 | 29.54 | 2.59 |
| Q | 301 | 29.34 | 2.53 |
| N | 181 | 29.14 | 2.48 |
| S | 198 | 29.13 | 2.48 |
| D | 294 | 29.05 | 2.46 |
| K | 182 | 29.02 | 2.45 |
| G | 196 | 28.81 | 2.39 |
| I | 197 | 28.75 | 2.37 |
| T | 293 | 28.17 | 2.22 |
| G | 199 | 27.73 | 2.1 |
| E | 302 | 26.75 | 1.84 |
| A | 292 | 26.39 | 1.74 |
| Q | 128 | 26.13 | 1.67 |
| A | 180 | 25.86 | 1.6 |
| V | 303 | 25.81 | 1.58 |
| Y | 211 | 25.77 | 1.57 |
| K | 316 | 25.75 | 1.57 |
| P | 195 | 25.69 | 1.55 |
| G | 248 | 25.62 | 1.53 |
| H | 74 | 14.33 | 1.51 |

| Table 6c: PDB: 2TLX | | | |
|---|---|---|---|
| WT AA | POS | Average main chain B-factor mean value (15.07) and variance (4.68) for the entire molecule | |
| | | Mean | z-score |
| G | 196 | 34.46 | 4.15 |
| S | 198 | 32.73 | 3.78 |
| T | 299 | 31.52 | 3.52 |
| I | 197 | 31.25 | 3.46 |
| I | 1 | 30.27 | 3.25 |
| K | 316 | 29.83 | 3.16 |
| T | 2 | 29.61 | 3.11 |
| S | 298 | 29.53 | 3.09 |
| T | 224 | 28.08 | 2.78 |
| P | 195 | 27.95 | 2.76 |
| G | 223 | 27.95 | 2.76 |
| T | 222 | 27.77 | 2.72 |
| G | 199 | 27.62 | 2.68 |
| K | 182 | 27.34 | 2.63 |
| Y | 211 | 26.34 | 2.41 |
| N | 181 | 26.29 | 2.4 |
| G | 127 | 25.6 | 2.25 |
| G | 297 | 25.48 | 2.23 |
| Q | 128 | 25.42 | 2.21 |
| S | 300 | 23.98 | 1.91 |
| Q | 225 | 22.79 | 1.65 |
| G | 212 | 22.72 | 1.64 |
| G | 3 | 22.45 | 1.58 |
| Q | 301 | 22.28 | 1.54 |
| D | 294 | 22.2 | 1.52 |

All sites in Thermolysin were screened by making as many possible single substitutions of amino acids in the molecule. Several variants that confer either thermostability or improved laundry performance at elevated temperatures in different laundry detergent formulations were found occurring at a site corresponding to one of these consensus flexibility regions. Representative substitutions in the consensus flexibility regions that confer improved laundry performance in Sun All-in-1 Turbo Gel or AT formula pH 8 detergent or improved thermostability are listed in Table 6.2. The working hypothesis is that these flexible regions are the initial sites of protein unfolding. Based on this hypothesis, combinations of variants from different consensus flexibility regions might be predicted to provide more stabilization. Simultaneous stabilization of several flexible regions selected from those shown in Table 2 would result in a substantially more stable molecule.

| Table 6.2: Stability variants in consensus flexibility regions | | |
|---|---|---|
| Consensus flexibility region | Position | Stability variants (WT AA 1^{st}) |
| 1-2 | 1 | I,V |
| | 2 | T,C,I,M,P,Q,V |
| 127-128 | 127 | G,C |
| | 128 | Q,C,E,F,I,L,V,Y |
| 180-181 | 180 | A,E,N |
| | 181 | N,A,G,Q,S |
| 195-199 | 196 | G,L,Y |
| | 197 | I,F |
| | 198 | S,A,C,D,E,H,I,M,P,Q,T,V,Y |
| 211 | 211 | Y,A,C,E,F,H,I,Q,S,T,V,W |
| 223-224 | 224 | T,D,H,Y |
| 298-300 | 298 | S,A,C,E,F,G,K,M,N,P,Q,R,T,W,Y |
| | 299 | T,A,C,D,F,G,H,I,K,L,M,N,P,Q,R,S,W |
| 316 | 316 | K,A,D,E,H,M,N,P,Q,S, T,V,Y |

## Claims

1. A thermolysin enzyme variant of the thermolysin of SEQ ID NO: 3, wherein the variant differs from the amino acid sequence of SEQ ID NO: 3 only by the amino acid substitution P277R.

2. A cleaning composition comprising at least one thermolysin enzyme variant of claim 1.

3. The cleaning composition of claim 2, wherein said cleaning composition is a detergent composition.

4. The cleaning composition of claim 2 or claim 3, wherein said cleaning composition is a laundry detergent composition, a dish detergent composition, or a hard surface cleaning composition.

5. A method of cleaning, comprising contacting a surface or an item with a cleaning composition comprising at least one thermolysin enzyme variant of claim 1.

6. A method of cleaning comprising contacting a surface or an item with a cleaning composition set forth in any one of claims 2-4.

## Patentansprüche

1. Thermolysin-Enzymvariante des Thermolysins unter SEQ ID NO: 3, wobei sich die Variante von der Aminosäuresequenz unter SEQ ID NO: 3 nur durch die Aminosäuresubstitution P277R unterscheidet.

2. Reinigungszusammensetzung, umfassend wenigstens eine Thermolysin-Enzymvariante nach Anspruch 1.

3. Reinigungszusammensetzung nach Anspruch 2, wobei es sich bei der Reinigungszusammensetzung um eine Waschmittelzusammensetzung handelt.

4. Reinigungszusammensetzung nach Anspruch 2 oder Anspruch 3, wobei es sich bei der Reinigungszusammensetzung um eine Waschmittelzusammensetzung für Wäsche, eine Waschmittelzusammensetzung für Geschirr oder eine Reinigungszusammensetzung für harte Oberflächen handelt.

5. Reinigungsverfahren, umfassend In-Kontakt-Bringen einer Oberfläche oder eines Artikels mit einer Reinigungszusammensetzung, die wenigstens eine Thermolysin-Enzymvariante nach Anspruch 1 umfasst.

6. Reinigungsverfahren, umfassend In-Kontakt-Bringen einer Oberfläche oder eines Artikels mit einer Reinigungszusammensetzung gemäß einem der Ansprüche 2-4.

## Revendications

1. Variant d'enzyme thermolysine de la thermolysine de la SEQ ID NO: 3, le variant différant de la séquence d'acides aminés de la SEQ ID NO: 3 seulement par la substitution d'acide aminé P277R.

2. Composition de nettoyage comprenant au moins un variant d'enzyme thermolysine selon la revendication 1.

3. Composition de nettoyage selon la revendication 2, ladite composition de nettoyage étant une composition de détergent.

4. Composition de nettoyage selon la revendication 2 ou la revendication 3, ladite composition de nettoyage étant une composition de détergent de blanchisserie, une composition de détergent pour vaisselle, ou une composition de nettoyage de surface dure.

5. Procédé de nettoyage, comprenant la mise en contact d'une surface ou d'un objet avec une composition de nettoyage comprenant au moins un variant d'enzyme thermolysine selon la revendication 1.

6. Procédé de nettoyage comprenant la mise en contact d'une surface ou d'un objet avec une composition de nettoyage telle qu'exposée dans l'une quelconque des revendications 2 à 4.
